# EUROPEAN PATENT SPECIFICATION

(11) **EP 3 668 522 B1**
(45) Date of publication and mention of the grant of the patent: **11.09.2024**
(21) Application number: 18846700.5
(22) Date of filing: 17.08.2018
(51) Int. Cl.: A61K 31/7088, A61K 39/145, A61K 9/51, G01N 33/50, A61P 31/16, A61K 47/69

(54) **EFFICACIOUS MRNA VACCINES**
WIRKSAME MRNA-IMPFSTOFFE
VACCINS À BASE D'ARNM EFFICACES

(30) Priority: 18.08.2017 US 201762547665 P; 19.09.2017 US 201762560548 P
(43) Date of publication of application: 24.06.2020
(73) Proprietor: ModernaTX, Inc., Cambridge, MA 02142 (US)
(72) Inventor: HOGE, Stephen, Cambridge, MA 02139 (US); CIARAMELLA, Giuseppe, Sudbury, MA 01776 (US)
(74) Representative: Hoffmann Eitle
(86) International application number: PCT/US2018/046974
(87) International publication number: WO 2019/036670

(56) References cited:
- WO-A1-2015/164674
- WO-A1-2016/154553
- WO-A1-2016/176330
- WO-A1-2017/191258
- WO-A1-2018/078053
- WO-A1-2018/081638
- WO-A1-2018/170245
- WO-A2-2018/089851
- REGINA HEIDENREICH ET AL: "A novel RNA-based adjuvant combines strong immunostimulatory capacities with a favorable safety profile : RNAdjuvant promotes anti-tumor responses of protein and peptide vaccines", INTERNATIONAL JOURNAL OF CANCER, vol. 137, no. 2, 21 December 2014 (2014-12-21), US, pages 372 - 384, XP055462897, ISSN: 0020-7136, DOI: 10.1002/ijc.29402
- BENTEBIBEL S.-E. ET AL: "ICOS+PD-1+CXCR3+ T follicular helper cells contribute to the generation of high-avidity antibodies following influenza vaccination", SCIENTIFIC REPORTS, vol. 6, 27 May 2016 (2016-05-27), pages 26494, XP055611535, Retrieved from the Internet <URL:https://www.nature.com/articles/srep26494> DOI: 10.1038/srep26494
- LIANG F. ET AL: "Vaccine priming is restricted to draining lymph nodes and controlled by adjuvant-mediated antigen uptake", SCIENCE TRANSLATIONAL MEDICINE, vol. 9, no. 393, 7 June 2017 (2017-06-07), pages eaal2094, XP055611537, Retrieved from the Internet <URL:http://stm.sciencemag.org/content/9/393/eaa12094> DOI: 10.1126/scitranslmed.aal2094
- WANG C. ET AL: "Lymphatic-targeted cationic liposomes: A robust vaccine adjuvant forpromoting long-term immunological memory", VACCINE, vol. 32, no. 42, 22 September 2014 (2014-09-22), pages 5475 - 5483, XP055611539, DOI: 10.1016/j.vaccine.2014.07.081
- HEITHOFF D.M. ET AL: "Conditions that diminish Myeloid-Derived Suppressor Cell activities stimulate cross-protective immunity", INFECTION AND IMMUNITY, vol. 76, no. 11, 1 November 2008 (2008-11-01), pages 5191 - 5199, XP055611543, DOI: 10.1128/IAI.00759-08

## Description

### BACKGROUND

Nucleic acid vaccines based on plasmid DNA, viral vectors or messenger RNA (mRNA) have been evaluated for several clinical applications including cancer, allergy and gene replacement therapies, and have proven to be effective as vaccines against infectious diseases. There has been considerable focus on modified mRNA vaccines during the last decade, as they are safe, scalable and offer precision in antigen design. They circumvent the problem of pre-existing immunity associated with viral vectors and appear to be more potent than DNA vaccines. mRNA vaccines may be especially valuable for emerging infections such as pandemic influenza.

### SUMMARY

While a multitude of RNA vaccines, including mRNA vaccines and self-replicating RNA vaccines, have been produced, the therapeutic efficacy of such vaccines has been inconsistent. The inventors have discovered a novel metric, differential T cell activation potential (dTCAP), which may be used to determine the quality of a vaccine, including whether or not it generates a therapeutic response with minimal to no toxicity. The vaccines of the present disclosure do not require viral or other forms of artificial replication to elicit a strong immune response, and therefore, have minimal toxicity. Accordingly, methods for determining optimal formulations for delivering mRNA vaccines to achieve these properties, as indicated by beneficial dTCAP values, have been discovered.

The new measure of quality of the invention allows for the production of new vaccines having enhanced properties and minimal toxicity. mRNA vaccines formulated in a different carriers may be developed having beneficial dTCAP values in order to provide maximal therapeutic benefits.

Based on the disclosure that is contained herein, the present invention provides a method for evaluating whether an mRNA vaccine composition, comprising mRNA encoding an antigen, will produce immunity against the antigen, the method comprising the steps of: identifying in a mammalian subject that has been injected with the mRNA vaccine composition, T cell suppression values in local populations of inflammatory cells in a sample taken from the injection site of the mRNA vaccine composition and in a sample taken from the draining lymph node of the subject, and determining a quantitative value of the amount of differential T Cell Activation Potential (dTCAP) based on the T cell suppression values from the injection site (T-supplS) and draining lymph node (T-suppLN), wherein the quantitative value of the dTCAP is indicative of the nature of the immune response generated in response to the mRNA vaccine administration; wherein the dTCAP is calculated as a ratio of the T-supplS to the T-suppLN and wherein a ratio of at least 6:1 is indicative of a positive mRNA vaccine outcome; wherein the T-suppLN is measured as an amount of Myeloid-Derived Suppressor Cells (MDSCs) present in a draining lymph node at a time following mRNA vaccine administration; and wherein the T-supplS is measured as an amount of MDSCs present in the injection site at a time following mRNA vaccine administration.

In a further aspect, the present invention provides a method for evaluating whether an mRNA vaccine composition, comprising mRNA encoding an antigen, will produce immunity against the antigen, the method comprising the steps of: determining a quantitative value of the amount of differential T Cell Activation Potential (dTCAP) in a sample taken from the draining lymph node of a mammalian subject that has been injected with a the mRNA vaccine composition, wherein the quantitative value of the dTCAP is indicative of the nature of the immune response generated in response to the mRNA vaccine administration; wherein the dTCAP is calculated as the ratio of draining lymph node antigenicity to draining lymph node tolerability; wherein the draining lymph node antigenicity is the amount of T cell activators present at the draining lymph node, wherein the T cell activators are T follicular helper cells (Tfh); wherein the draining lymph node tolerability is the amount of MDSCs present at the draining lymph node; and wherein a ratio of greater than 1000:1 is indicative of a positive mRNA vaccine outcome.

The present invention and some preferred embodiments thereof are set out in the appended claims.

### DETAILED DESCRIPTION

The technical information set out below may in some respects go beyond the scope of the invention, which is defined exclusively by the appended claims. The additional technical information is provided to place the actual invention in a broader technical context and to illustrate possible related technical developments.

In addition, incidental references to methods for treatment of the human or animal body by surgery or therapy and diagnostic methods practised on the human or animal body are not to be construed as claiming protection for such methods as such, but are instead to be construed as referring to products, in particular substances or compositions, for use in any of these methods.

In some aspects the disclosure provides a vaccine composition of an mRNA encoding an antigen formulated in a carrier, wherein the vaccine composition exhibits a threshold dTCAP in a mammalian subject.

In some embodiments the mammalian subject is a human. In one embodiment, the antigen is derived from a human pathogen. In other embodiments the antigen is not an influenza or Zika antigen.

In some embodiments, e.g., when the dTCAP of a potential vaccine is being tested, the mammalian subject is a non-human mammal. In one embodiment, the sample from the injection site or the draining lymph node is a biopsy sample.

In some embodiments the carrier is a lipid nanoparticle (LNP), a polymeric nanoparticle, a lipid carrier such as a lipidoid, a liposome, a lipoplex, a peptide carrier, a nanoparticle mimic, a nanotube, or a conjugate.

In some embodiments the mRNA is chemically modified mRNA.

In some embodiments the mRNA comprises a microRNA (miR) binding site. The mRNA may be at least two different microRNA (miR) binding sites. The mRNA may comprise first and second microRNA binding sites of the same microRNA. In some embodiments the microRNA binding sites are of the 3p and 5p arms of the same microRNA. In other embodiments the microRNA binding site is for a microRNA selected from the group consisting of miR-126, miR-142, miR-144, miR-146, miR-150, miR-155, miR-16, miR-21, miR-223, miR-24, miR-27, miR-26a, or any combination thereof. In some embodiments the microRNA binding sites are located in the 5' UTR, 3' UTR, or both the 5' UTR and 3' UTR of the mRNA.

In some embodiments the mRNA has a 5' terminal cap that comprises a Cap0, Cap1, ARCA, inosine, N1-methyl-guanosine, 2'-fluoro-guanosine, 7-deaza-guanosine, 8-oxo-guanosine, 2-amino-guanosine, LNA-guanosine, 2-azidoguanosine, Cap2, Cap4, 5' methylG cap, or an analog thereof.

In some embodiments the mRNA comprises a poly-A region. The poly-A region may have about 10 to about 200, about 20 to about 180, about 50 to about 160, about 70 to about 140, or about 80 to about 120 nucleotides in length.

In some embodiments the mRNA comprises at least one chemically modified nucleobase, sugar, backbone, or any combination thereof. In embodiments the at least one chemically modified nucleobase is selected from the group consisting of pseudouracil (ψ), N1-methylpseudouracil (m1ψ), 1-ethylpseudouracil, 2-thiouracil (s2U), 4'-thiouracil, 5-methylcytosine, 5-methyluracil, 5-methoxyuracil, and any combination thereof.

In some embodiments at least about 25%, at least about 30%, at least about 40%, at least about 50%, at least about 60%, at least about 70%, at least about 80%, at least about 90%, at least about 95%, at least about 99%, or 100% of the guanines, adenines, uracils or thymines are chemically modified.

In some embodiments the mRNA is purified.

The claimed methods are set out in the appended claims.

In one aspect of the invention, the dTCAP is calculated as a ratio of the T-supp_{LN} to the T-supp_{IS} and wherein the ratio is at least 6:1 for producing a threshold dTCAP for a high quality vaccine. In this aspect, the T-supp_{LN} may be measured as an amount of Myeloid-Derived Suppressor Cells (MDSCs) present in a draining lymph node at a time following vaccine administration. The amount of MDSCs present in the draining lymph node may be calculated 1-7 days following vaccine administration. In some embodiments the amount of MDSCs present in the draining lymph node is 0-10 MDSCs/10⁵ live cells.

In this aspect, the T-supp_{IS} is measured as an amount of MDSCs present in the injection site at a time following vaccine administration.

In some embodiments the amount of MDSCs present in the injection site is calculated 1-7 days following vaccine administration. The amount of MDSCs present in the injection site may be 50-1,000 MDCSs/10⁵ live cells. In some embodiments the quantitative value of the dTCAP is indicative of an immunostimulatory activity of the vaccine. In some embodiments the activity is antigen specific T-cell activity.

Each of the limitations of the disclosure can encompass various embodiments of the disclosure. It is, therefore, anticipated that each of the limitations of the disclosure involving any one element or combinations of elements can be included in each aspect of the disclosure. This disclosure is not limited in its application to the details of construction and the arrangement of components set forth in the following description or illustrated in the drawings. The disclosure is capable of other embodiments and of being practiced or of being carried out in various ways.

### BRIEF DESCRIPTION OF DRAWINGS

The accompanying drawings are not intended to be drawn to scale. In the drawings, each identical or nearly identical component that is illustrated in various figures is represented by a like numeral. For purposes of clarity, not every component may be labeled in every drawing. In the drawings:

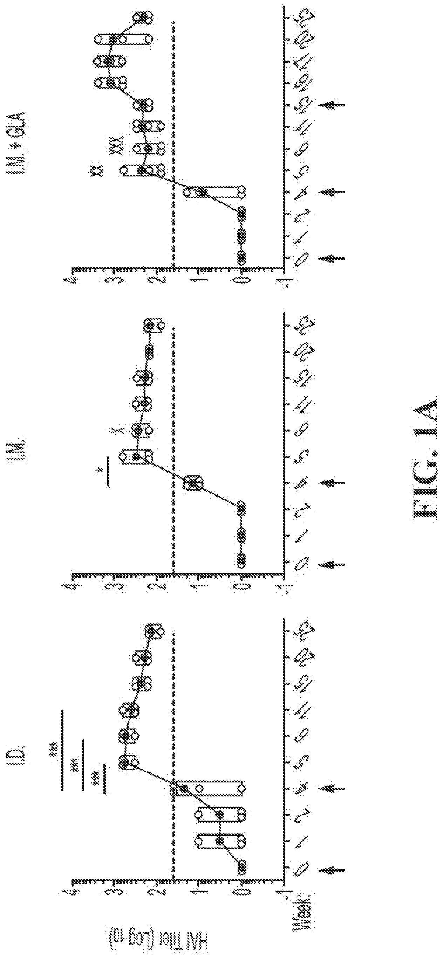

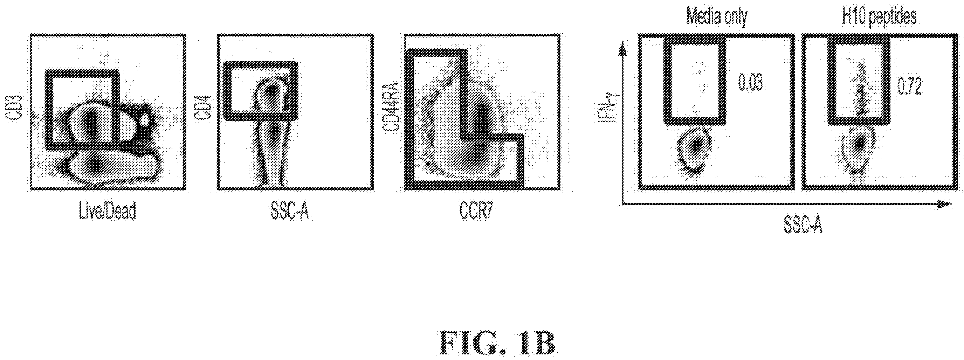

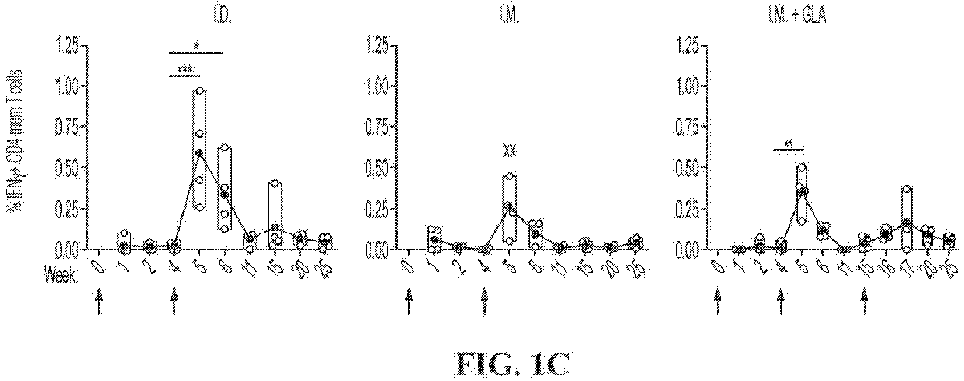

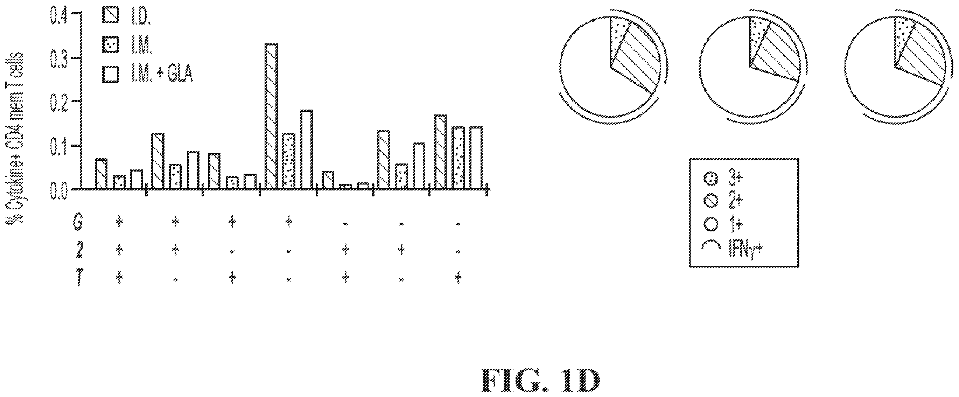

**FIGs. 1A-1D** show that the lipid nanoparticle-encapsulated mRNA platform elicits strong vaccine immunity by distinct vaccination routes. Rhesus macaques received intradermal (I.D.) or intramuscular (I.M.) immunizations with lipid nanoparticles (LNP) containing mRNA encoding H10 (hemagglutinin of H10N8 influenza A virus) or co-formulated with GLA adjuvant (I.M. +GLA), (n=4/group). **FIG. 1A** shows H10-specific hemagglutination inhibition (HAI) titers in the different groups, which received immunizations at week 0 and 4. The I.M.+GLA group received additional boost at week 15. Black circles show the mean and arrows indicate the immunizations. Threshold for protective titers are indicated by dashed horizontal lines. **FIG. 1B** shows the gating strategy of cytokine+total CD4 memory T cells. **FIG. 1C** shows % IFN-γ+ total CD4+ memory T cells after H10 peptide recall stimulation in the same animals. **FIG. 1D** shows the percent of cytokine-positive CD4 memory T cells, as represented by sole or simultaneous expression of IFN-γ (G), IL-2 (2) and/or TNF (T). The pie chart slivers in black, grey and light grey indicate triple, double or single cytokine+ cells, respectively, and IFN-γ+cells are indicated by the arcs. Cross(es) indicate significantly lower HAI titer or % IFN-y+ cells compared to the same time point in the I.D. group. Two-way Anova test. *p<0.05, **p<0.01, ***p<0.001.

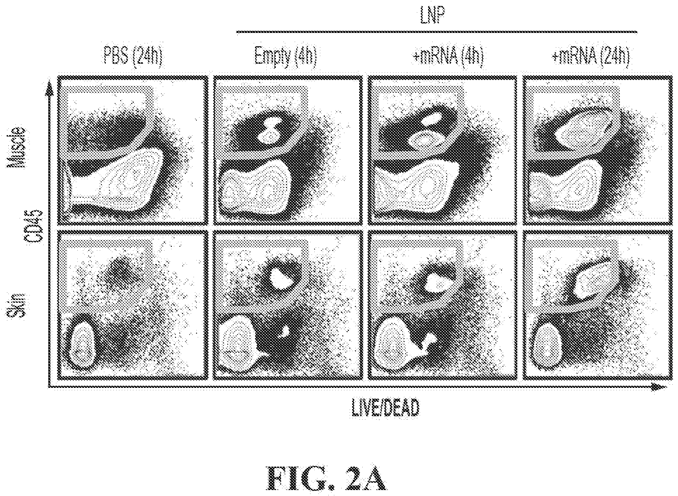

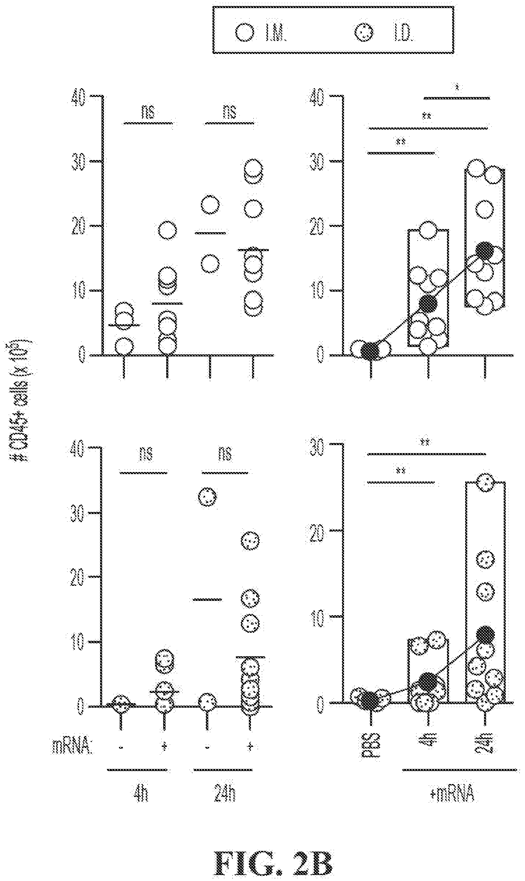

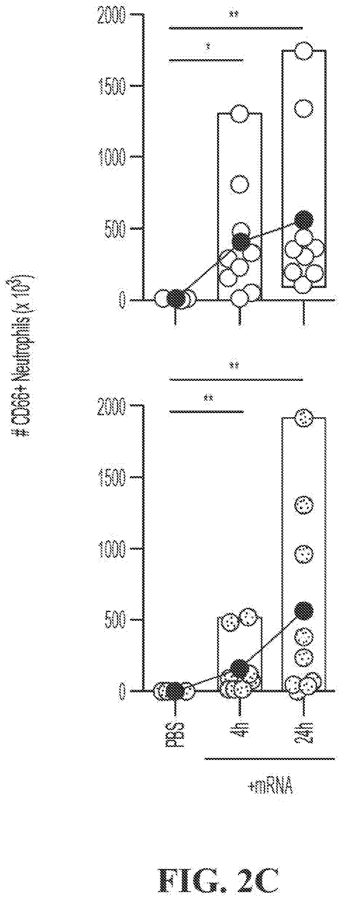

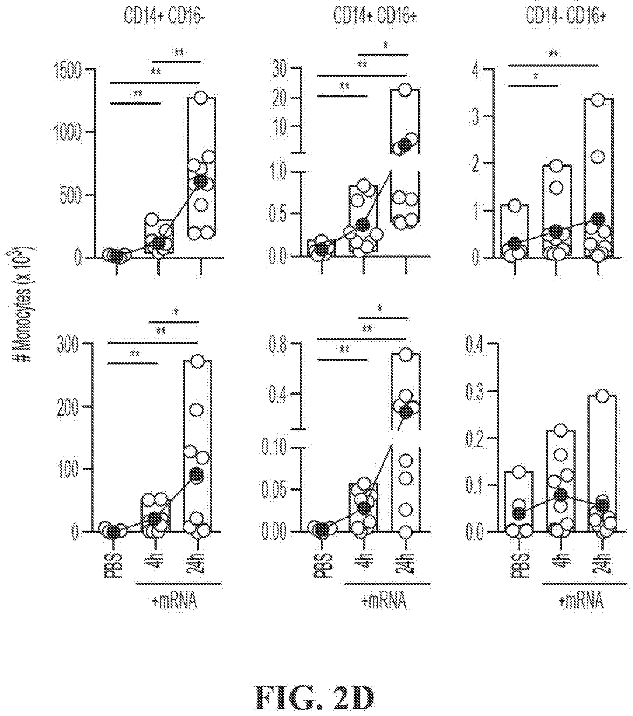

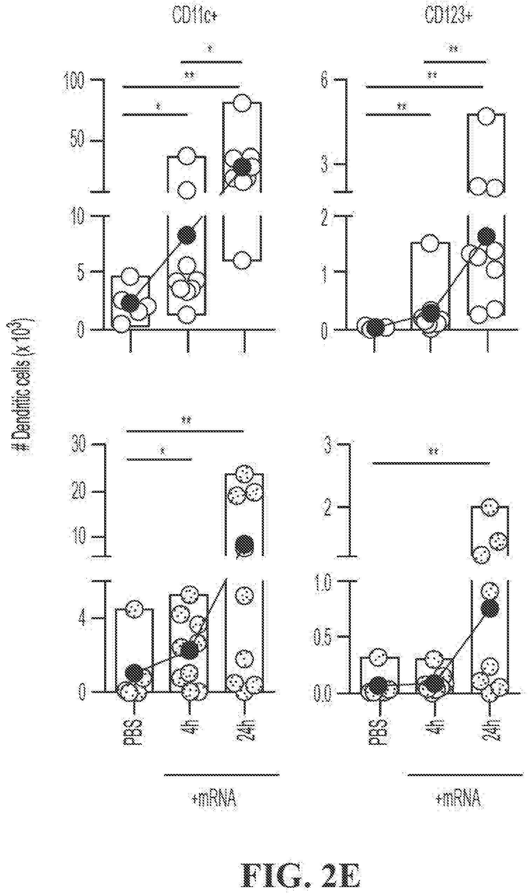

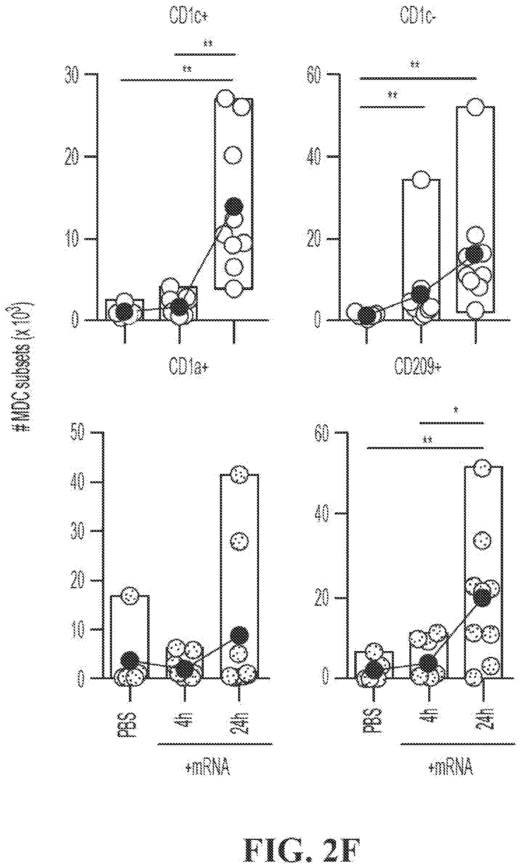

**FIGs. 2A-2F** show rapid immune cell infiltration in response to LNP/mRNA administration to distinct injection sites. **FIG. 2A** shows gating of infiltrating live CD45+ immune cells in cell suspensions of muscle and skin injected with PBS or LNP with or without encapsulated mRNA at 4h and 24h. **FIG. 2B** shows a comparison of CD45+ immune cell levels in muscle (I.M., white circles) vs. skin (I.D., grey circles) at 4h or 24h after injection of LNP without (-) or with (+) mRNA cargo. **FIGs. 2C-2F** show characterization of the CD45+ mobilized immune cell subsets: CD66abce+ neutrophils **(****FIG. 2C****),** CD14+CD16- classical monocytes, CD14+ CD16+ intermediate monocytes, CD14- CD16+non-classical monocytes **(****FIG. 2D****),** CD11c+myeloid dendritic cells (DCs) and CD123+plasmacytoid DCs **(****FIG. 2E****),** CD1c (BDCA-1)+or- DCs in muscle and CD1a+ or CD209 (DC-SIGN)+APCs in skin **(****FIG. 2F****).** (n=5/group). Black circles show the mean. *p<0.05, **p<0.01, ns (not significant). Wilcoxon signed rank test.

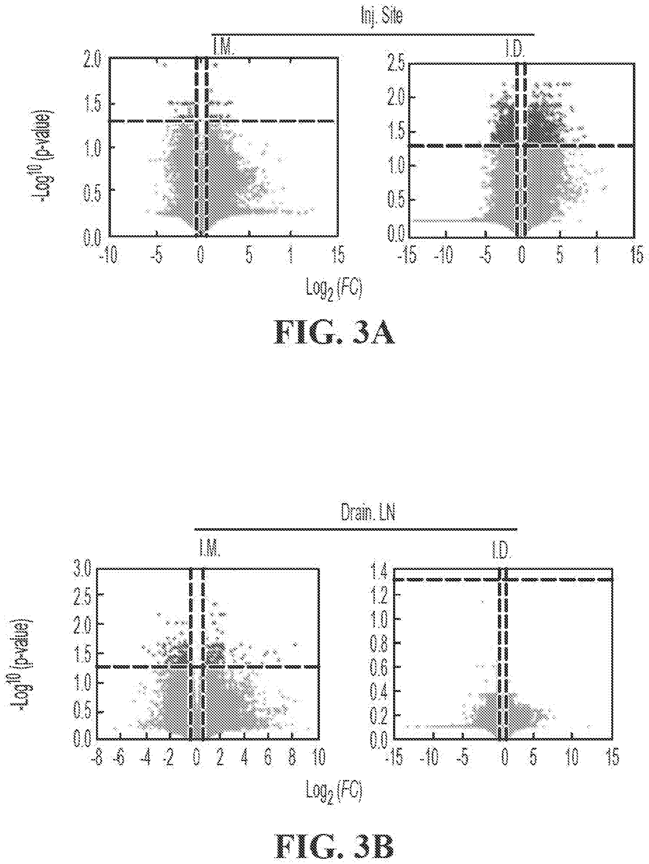

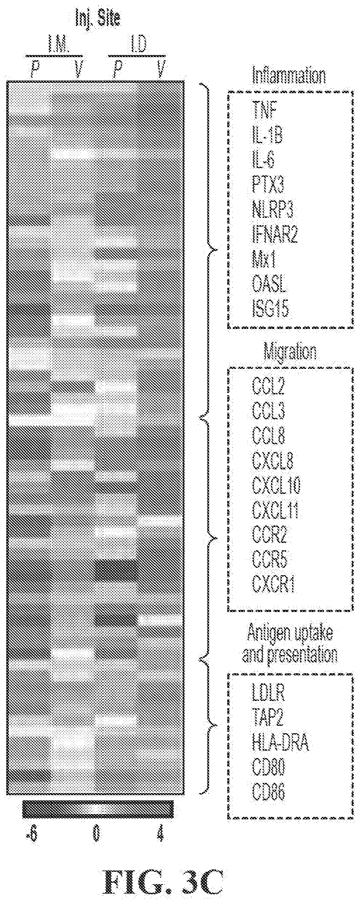

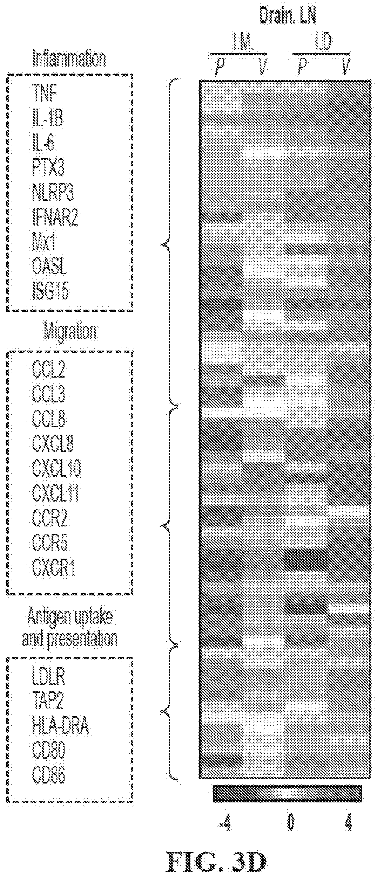

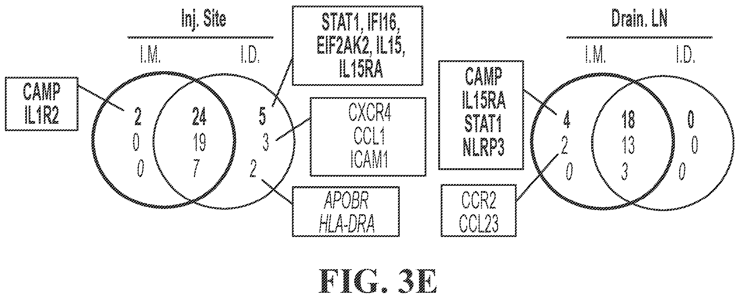

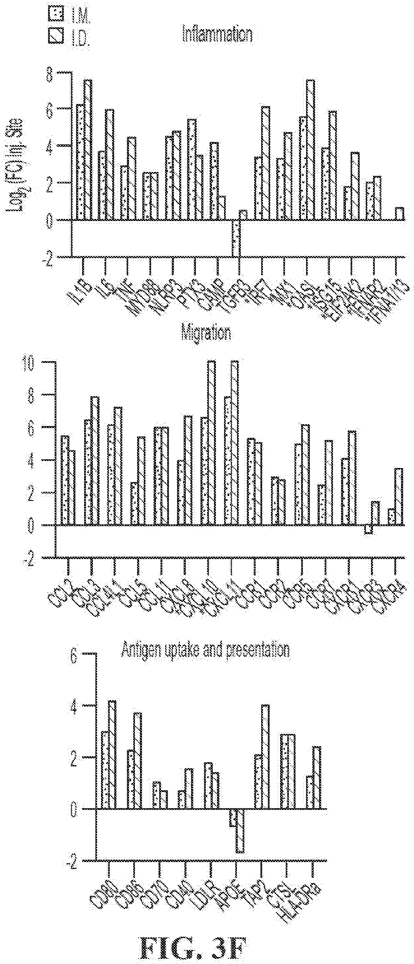

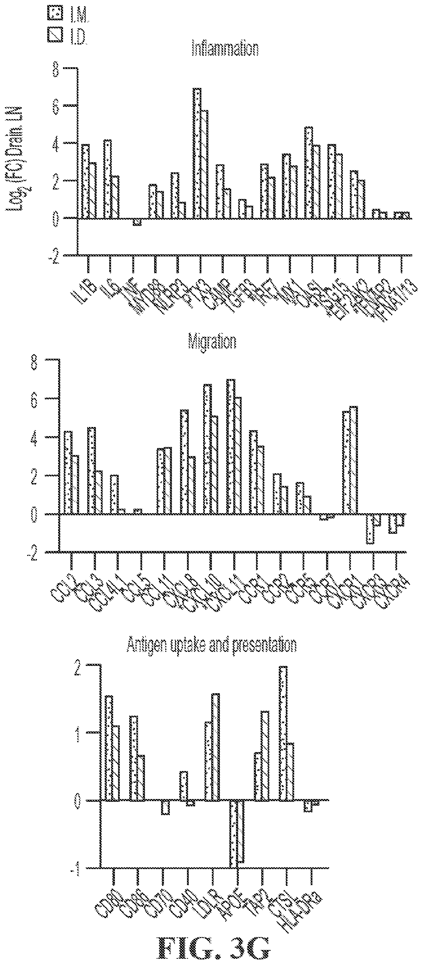

**FIGs. 3A-3G** show gene expression analysis of LNP/mRNA injection sites and draining lymph nodes (dLNs). **FIGS. 3A-3B** show volcano plots illustrating gene regulation in the LNP/mRNA injection sites (**FIG. 3A**) and draining LNs (**FIG. 3B**) relative to PBS controls. Dots represent gene expression in regards to fold change (FC) obtained by log₂ (ratio of the mean expression in vaccine and PBS sites, respectively) and -log₁₀ of FOR corrected p-values from unpaired Students' t-test. Up- and down-regulated genes in the vaccine relative to PBS are shown to the right vs. left side of the vertical dashes. Dark grey dots above the horizontal dashes for -log₁₀ p-value of 0.05, are significantly regulated genes. **FIGs. 3C-3D** show heat maps of selected genes in muscle or skin (**FIG. 3C****)** injected with PBS (P) or LNP/mRNA vaccine (V) and LNs draining these injection sites (**FIG. 3D****)**, which are primarily involved in inflammatory, migratory and antigen uptake and presentation. Level of expression was determined by log₂ (mean expression in vaccine or PBS site/ mean expression in vaccine and PBS site combined). Examples of the selected genes are shown in the dashed boxes. **FIG. 3E** shows the number of selected genes with log₂ (FC) ≥ 2 exclusively or mutually expressed in I.M. and I.D groups. Numbers in dark gray, medium gray, and light gray represent genes involved in inflammation, migration and antigen uptake and presentation, respectively. Boxes show genes with log₂ (FC) ≥ 2 upregulation only in I.M. or I.D group. **FIGs. 3F-3G** show log₂(FC) of specific genes of interest relative to PBS controls at the injection sites (**FIG. 3F**) and draining LNs (**FIG. 3G**) related to the indicated pathways. Asterisks with arrows indicate genes involved in IFN signaling and asterisks without arrows are IFN-inducible genes. (n=4/group).

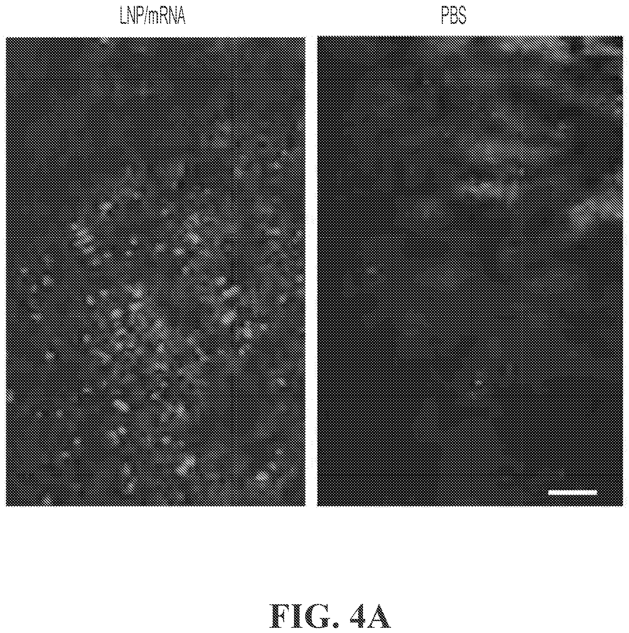

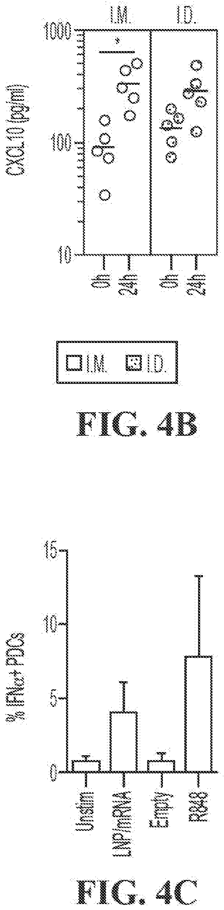

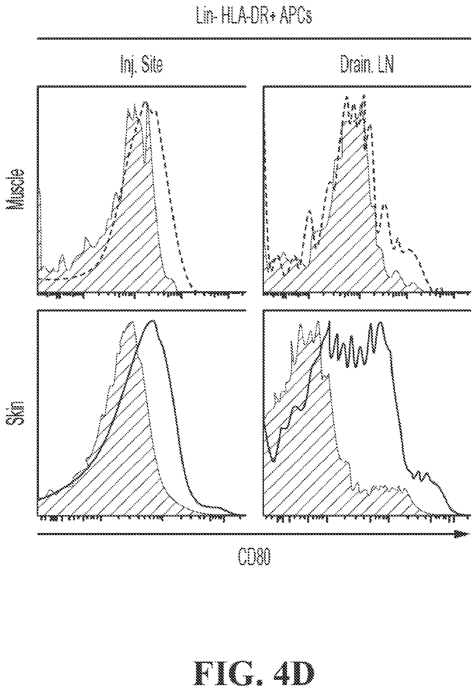

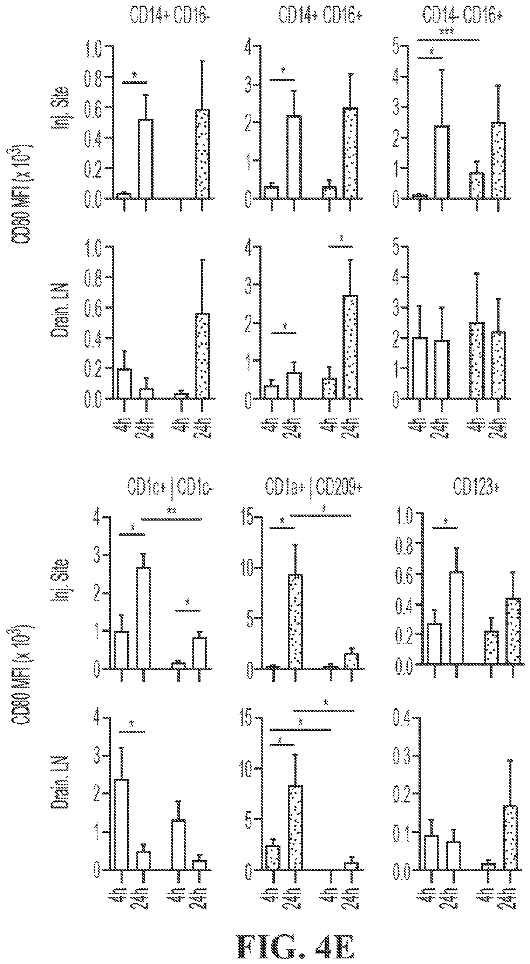

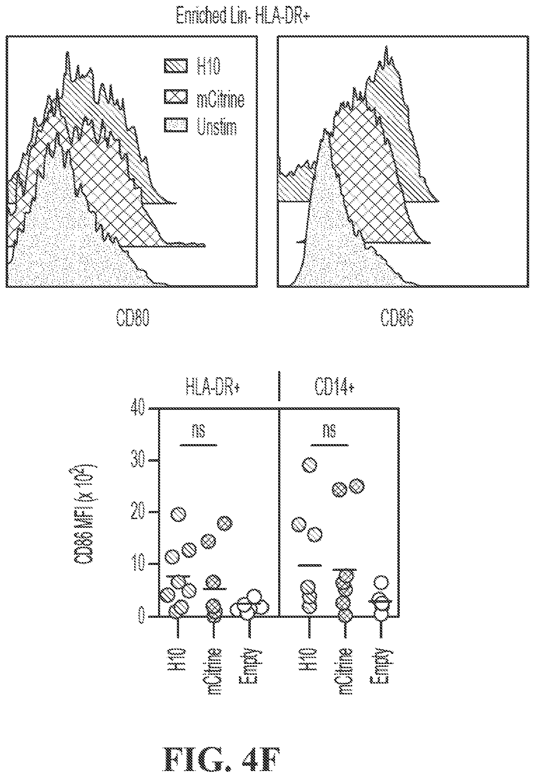

**FIGs 4A-4F** show LNP/mRNA induction of cellular activation with a type I IFN response signature. **FIG. 4A** shows *in situ* expression of type I IFN-inducible MxA at the draining LNs of PBS vs. LNP/mRNA injection. **FIG. 4B** shows serum levels of IFN-inducible CXCL10 (IP-10) prior to and 24 hours after LNP/mRNA immunization. **FIG. 4C** shows IFNα production *in vitro* by CD123+PDCs in rhesus PBMCs stimulated with the indicated conditions, (n=5). **FIG. 4D** shows expression of co-stimulatory CD80 at 24h on lineage (CD3/CD8/CD20)- HLA-DR+ antigen presenting cells (APCs) infiltrating muscle and skin injection sites and their draining LNs. The filled histograms represent the PBS control sites and the dashed (I.M.) and solid (I.D.) lines denote LNP/mRNA sites from the same animal. **FIG. 4E** shows compiled background subtracted (vaccine site - PBS site) mean fluorescence intensity (MFI) of CD80 on APC subsets at the injection sites and draining LNs at 4h and 24h in the I.M. and I.D groups (n=4/group). **FIG. 4F** shows CD80 and CD86 expression *in vitro* by enriched human HLA-DR+APCs or CD14+ monocytes in the presence of LNP with mRNA encoding either mCitrine or H10. Bars show the mean background subtracted CD86 MFI, (n≥6). *p<0.05, **p<0.01, ***p<0.001, ns (not significant). Wilcoxon signed rank test.

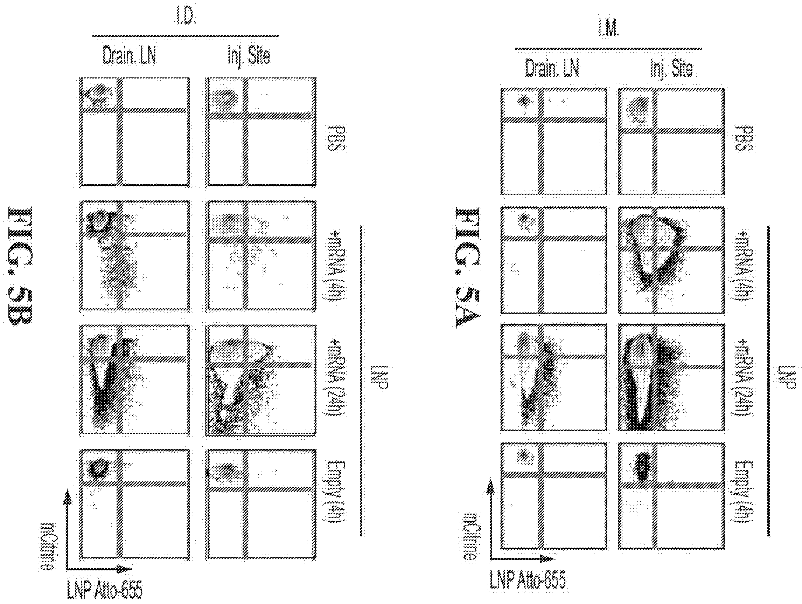

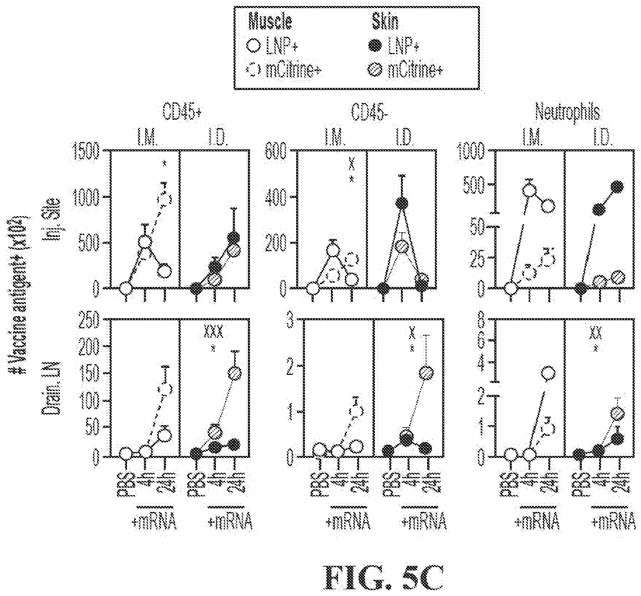

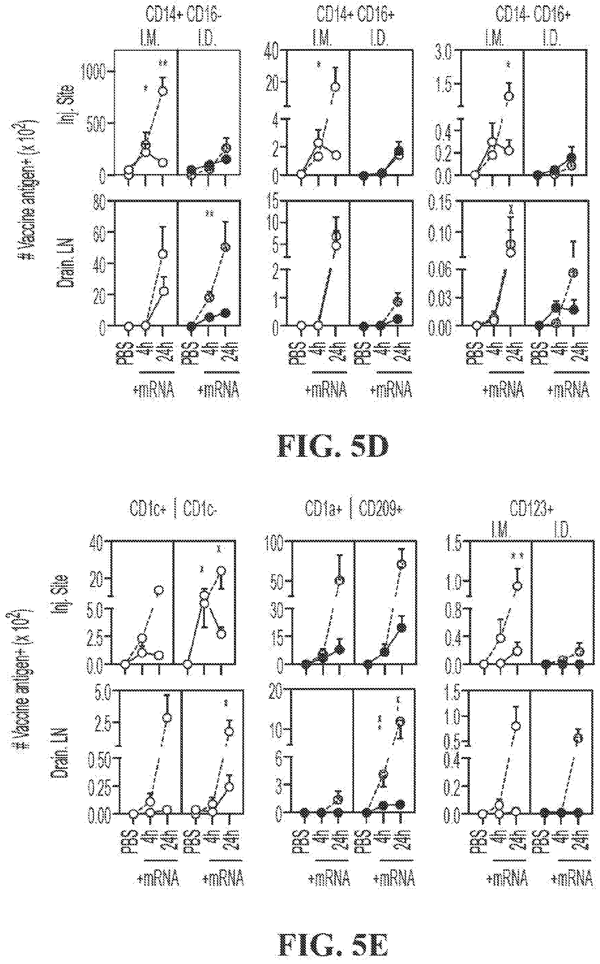

**FIGs. 5A-5E** show efficient LNP uptake and translation of mRNA cargo at the administration sites and their draining LNs. **FIGs. 5A-5B** show uptake of Atto-655-labeled LNP and translation of encapsulated mRNA encoding fluorescent mCitrine by lineage HLA-DR+APCs in suspensions of muscle (**FIG. 5A**) and skin (**FIG. 5B**) injection sites and draining LNs at 4h vs. 24h post-injection. Empty quadrants indicate unlabeled LNP without mRNA. **FIG. 5C** shows compiled data of LNP uptake and mRNA translation by CD45+ immune cells, CD45- tissue cells, and neutrophils. **FIG. 5D** shows complied data of LNP uptake and mRNA translation by CD14+CD16- cells, CD14+CD16+ cells, and CD14- CD16+ cells. **FIG. 5E** shows compiled data of LNP uptake and mRNA translation by CD1c+ | CD1c- cells, CD1a+|CD209+ cells, and CD123+cells. In **FIGs. 5C-5E**, open circles represent I.M. and closed circles denote an I.D. group. Circles in black and grey show mean numbers of LNP+ and mCitrine+ cells, respectively. The level of significance in the number of mCitrine+cells or LNP+cells between the I.M. and I.D groups at the indicated time points are shown with asterisk(s) vs. cross(es) respectively, (n=4/group). *p<0.05, **p<0.01, ***p<0.001. Mann-Whitney test.

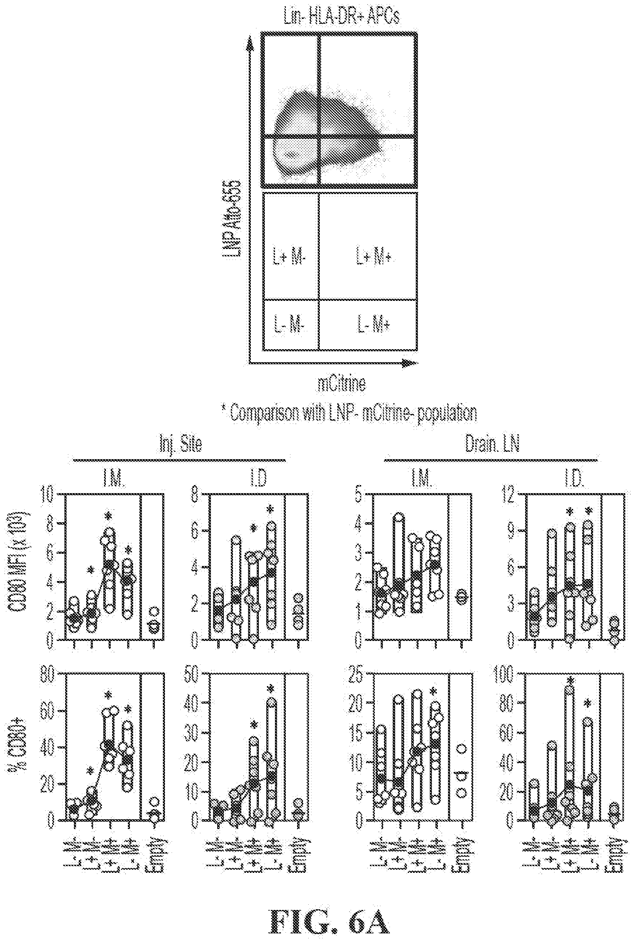

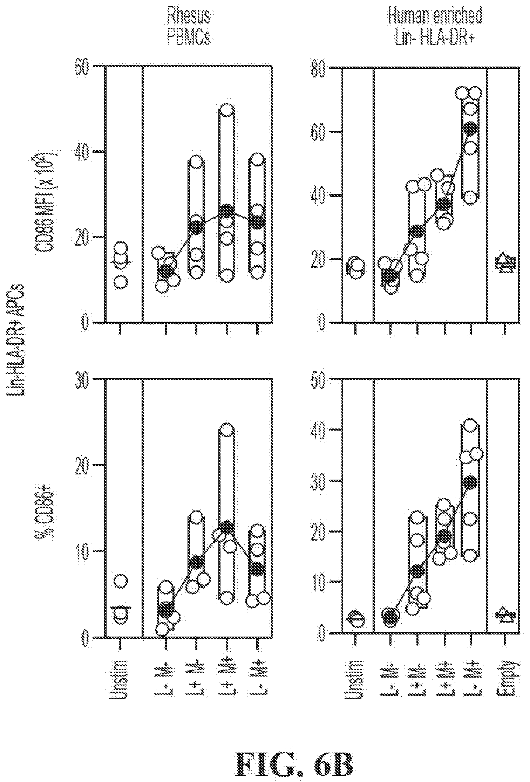

**FIGs. 6A-6B** show IFN responses associated with mRNA translation correlate with mRNA-induced immunity. **FIG. 6A** shows quadrant gates on lineage HLA-DR+APCs according to their Atto-655- labeled LNP (L) uptake and mCitrine mRNA translation (M) at the injection sites and draining LNs. CD80 MFI and % CD80+within LNP- mCitrine- (L-M-), LNP+ mCitrine- (L+ M-), LNP+ mCitrine+ (L+M+), and LNP- mCitrine+ (L- M+) populations were compared to an empty unlabeled LNP injection control. **FIG. 6B** shows CD86 expression according to Atto-655 labeled LNP uptake and mCitrine mRNA translation on lineage- HLA-DR+APCs from rhesus PBMCs and enriched human APCs stimulated with LNP/mCitrine mRNA *in vitro.*

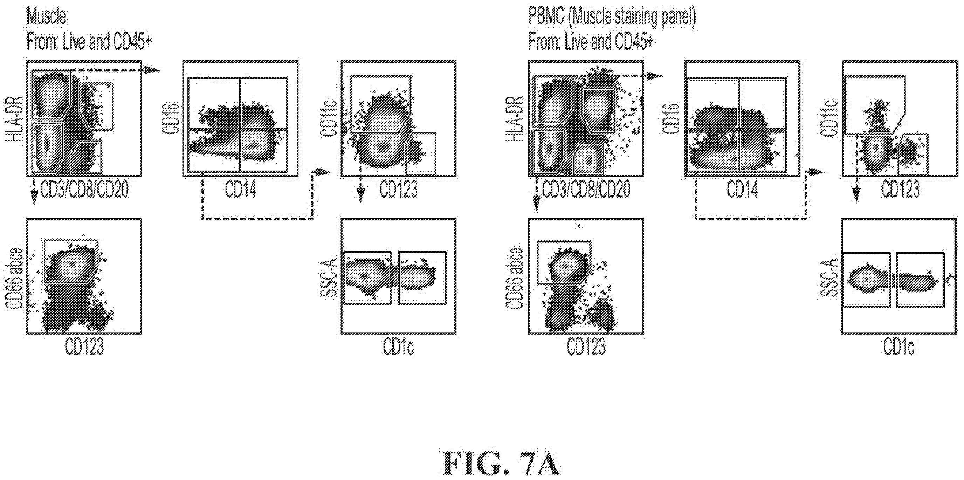

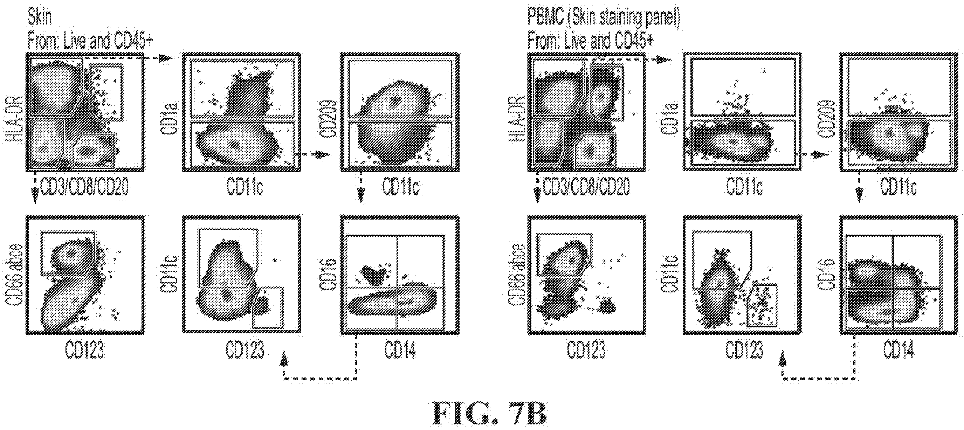

**FIGs. 7A-7B** show flow cytometry plots for gating of immune cell subsets in muscle (**FIG. 7A**) and skin (**FIG. 7B**) cell suspension with PBMC as reference cells.

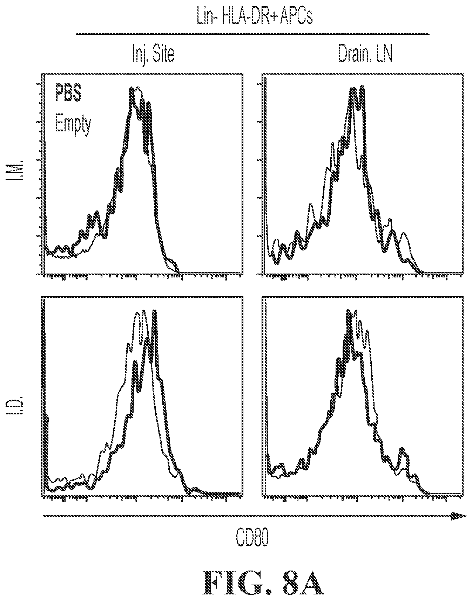

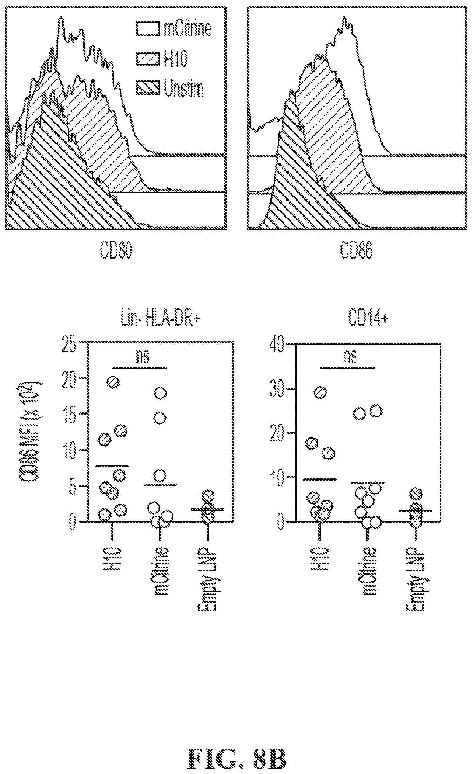

**FIGs. 8A-8B** show that type I IFN response and activation require mRNA cargo. **FIG. 8A** shows histograms of CD80 expression on lineage- HLA-DR+ APCs at the injection sites and draining LNs. Dark gray and light gray histograms indicate PBS and unlabeled empty LNP sites, respectively. **FIG 8B** shows CD80 vs. CD86 cell surface expression on Lin-HLA-DR+ cells vs. CD14+ cells.

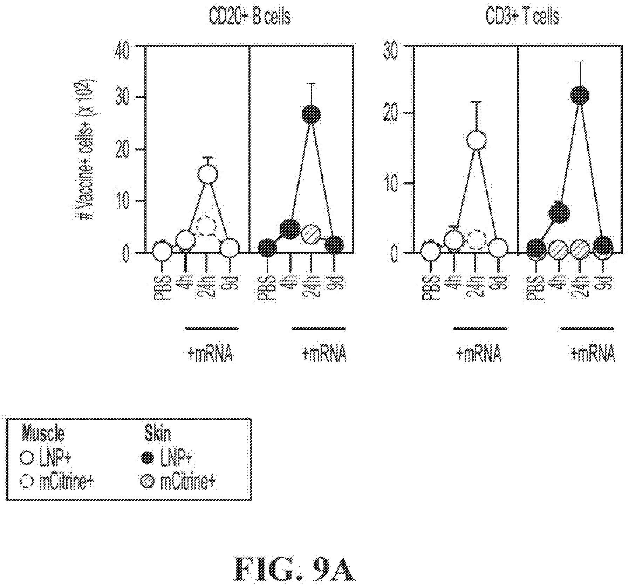

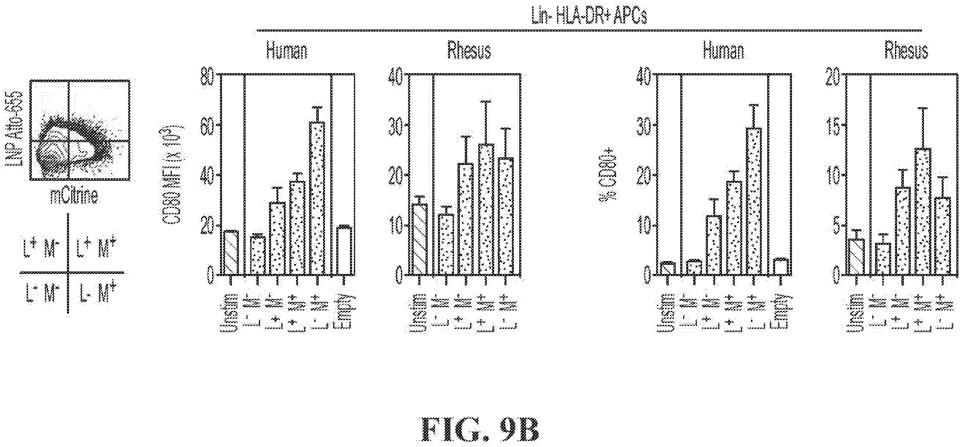

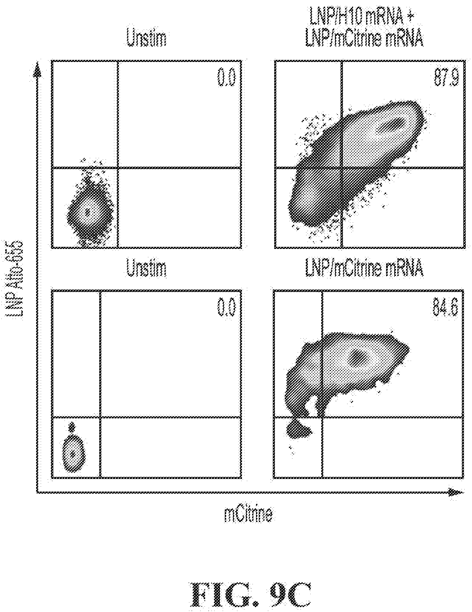

**FIGs. 9A-9C** show T cell and B cell responses to LNP/mRNA delivery. Compiled data on Atto-655 labeled LNP uptake and mCitrine mRNA translation by CD3+T cells and CD20+ B cells in the draining LNs at 4 hour, 24 hour, and 9 days after administration of LNP/mRNA in I.M. vs. I.D. group (n=4/group) is shown. *p<0.05, **p<0.01. Mann-Whitney test. **FIG. 9A** is a graph depicting an analysis of CD20+ B cells versus CD3+T cells at 4 hours, 24 hours and 9 days. **FIG. 9B** is a set of bar graphs depicting CD80+cells in human and Rhesus macaque (rhesus) under the various conditions. **FIG 9C** shows FACS analysis of the study.

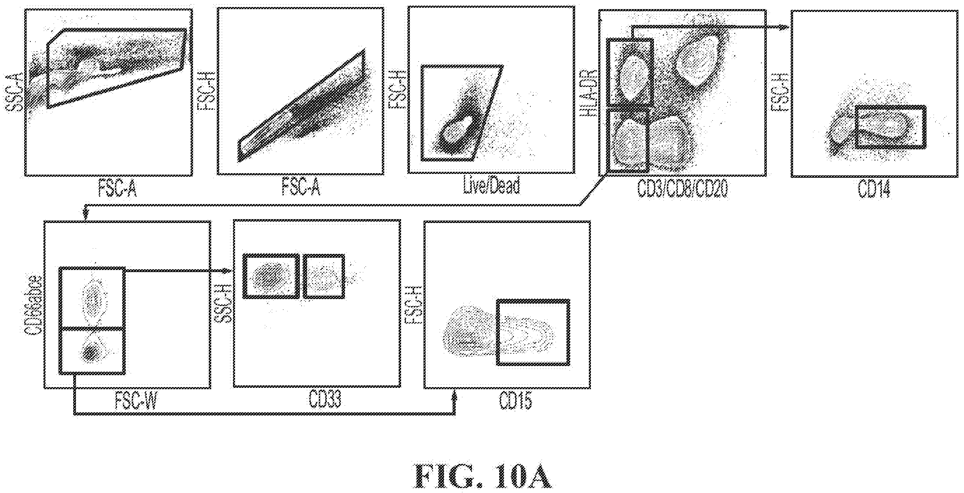

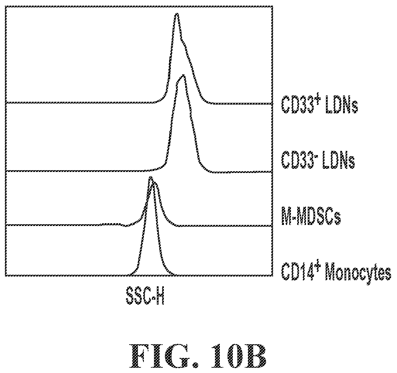

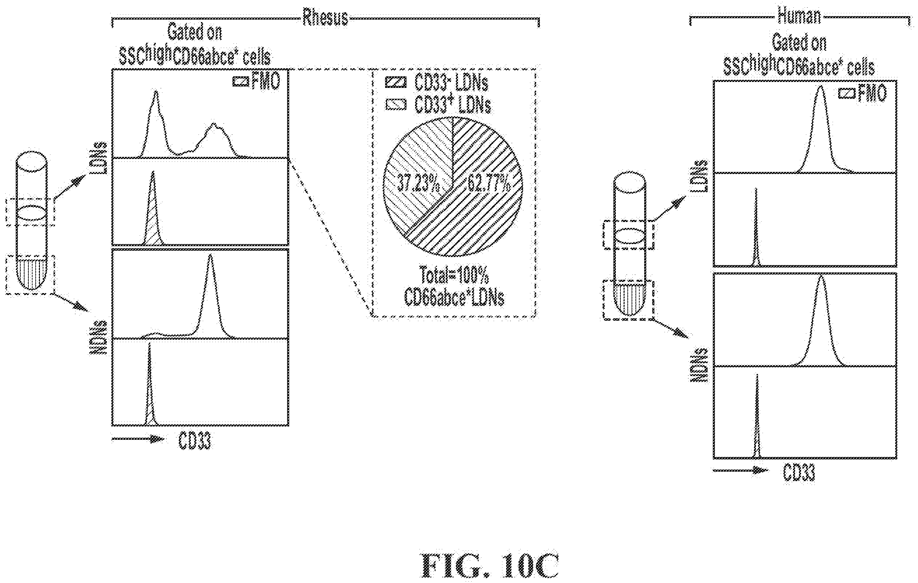

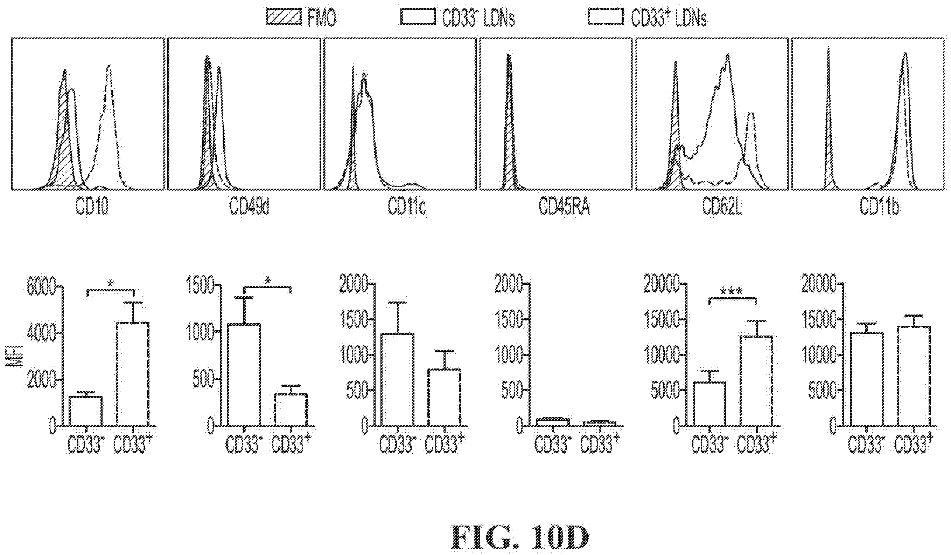

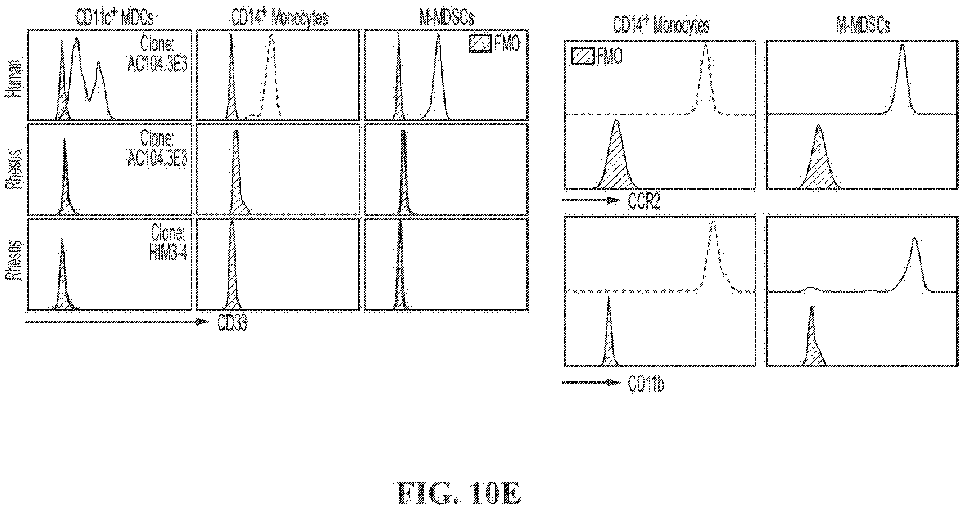

**FIGs. 10A-10E** show the phenotype of MDSCs in rhesus macaques and humans. **FIG. 10A** shows flow plots of CD14+ monocytes, M-MDSCs, CD33+ and CD33- LDNs in rhesus PBMCs. **FIG. 10B** shows a graph of the difference in the side scatter (SSC) parameter of LDNs, M-MDSCs, and monocytes. **FIG. 10C** shows flow plots of CD33 expression on LDNs and NDNs from rhesus (left panel) and human (right panel). **FIG. 10D** shows representative histograms of the indicated markers on rhesus CD33- and CD33+ LDNs (top panel) and mean fluorescence intensities (MFI) of the indicated markers (bottom panel) (mean ± SEM, n ≥ 5). **FIG. 10E** shows flow plots of CD33 expression on MDCs, monocytes, and M-MDSCs from human and rhesus (top panel) and flow plots of CCR2 and CD11b surface expression on M-MDSCs and monocytes from rhesus (bottom panel). Grey histograms represent Fluorescence Minus One (FMO) controls.

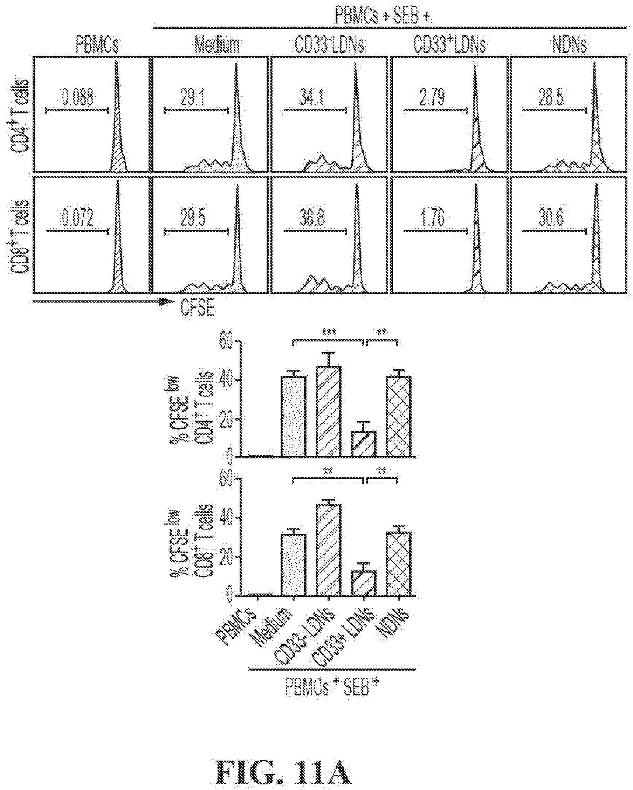

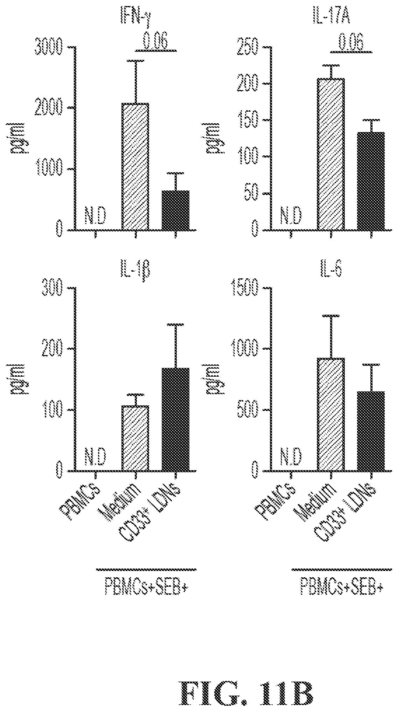

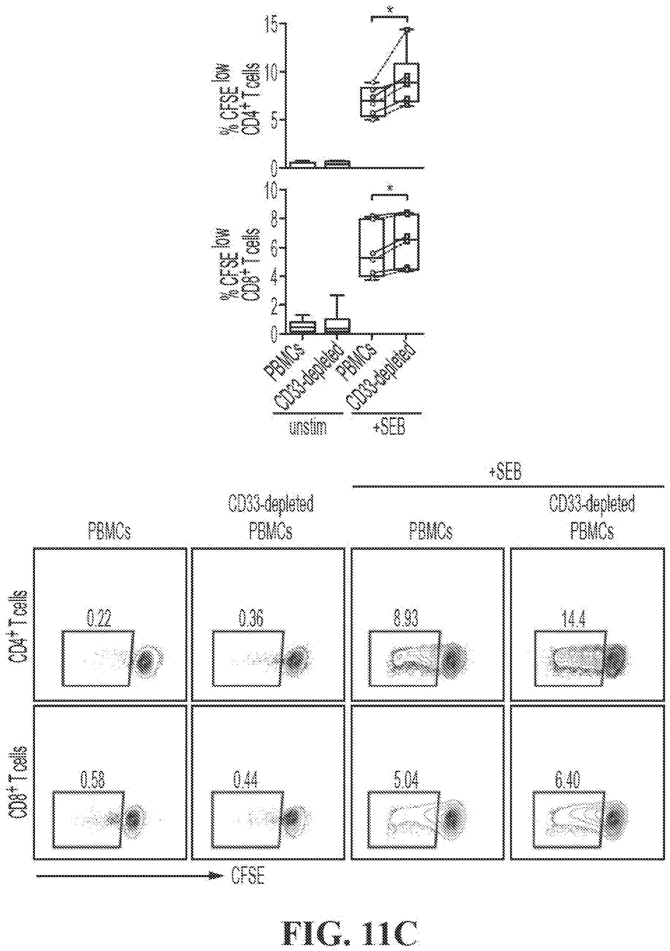

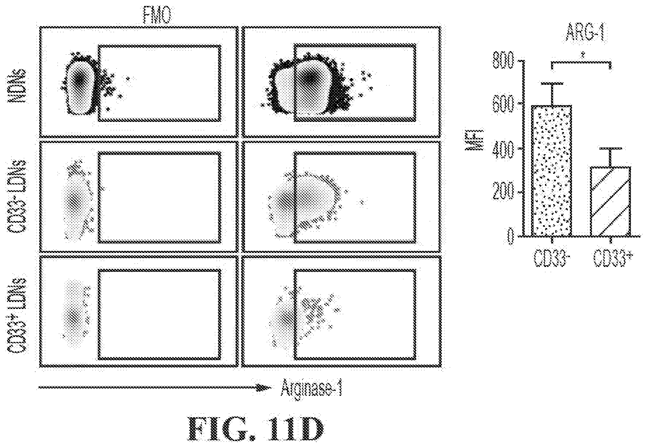

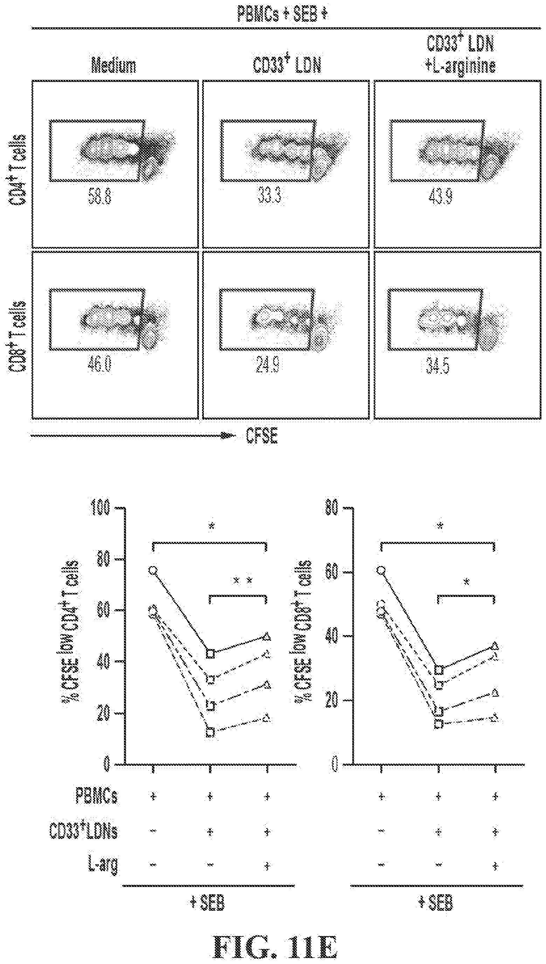

**FIGs. 11A-11E** show that rhesus PMN-MDSCs are CD33+ LDNs with suppressive function partly mediated by release of arginase-1. **FIG. 11A** shows flow plots of rhesus CD33- LDNs, CD33+ LDNs, and NDNs sorted from co-cultures of CFSE-labeled autologous PBMCs in the presence of SEB (left panel) and bar graphs of the percentage of proliferating T cells (n ≥ 5) (right panel). **FIG. 11B** shows graphs of the levels of indicated cytokines in supernatants from cell cultures. **FIG. 11C** shows flow plots of PBMCs or donor-matched PBMCs depleted of CD33+ cells sorted from cultures stimulated with SEB (left panel) and bar graphs of the percentage of proliferating T cells (n=6) (right panel). **FIG. 11D** shows flow plots of intracellular expression of arginase-1 in NDNs, and CD33- and CD33+ LDNs. The MFI of arginase-1 is shown (right graph; n=5). **FIG. 11E** shows flow plots of CD33+ LDNs sorted from co-cultures of CFSE-labeled autologous PBMCs with or without the addition of L-arginine (200 µg/ml) (top panel) and compiled data where each line represents an individual animal (n = 4) (bottom panel). Data is shown as mean ± SEM.

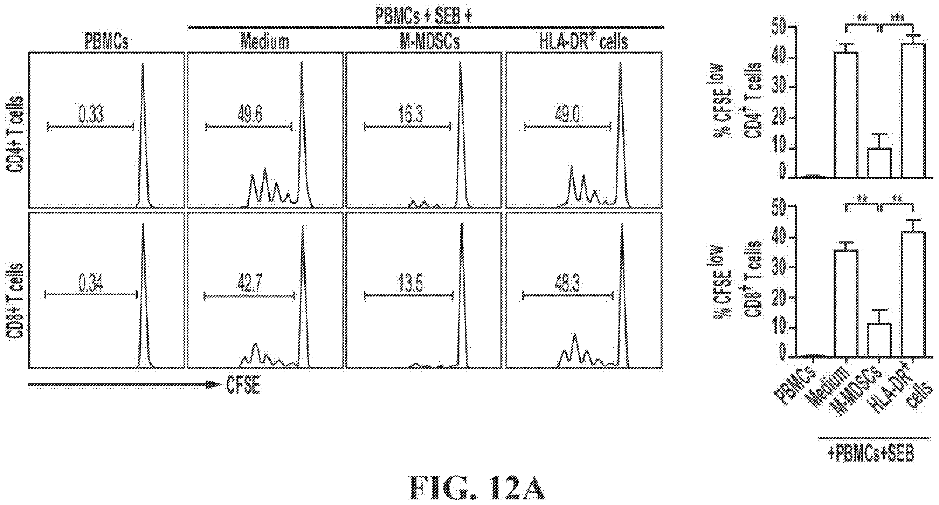

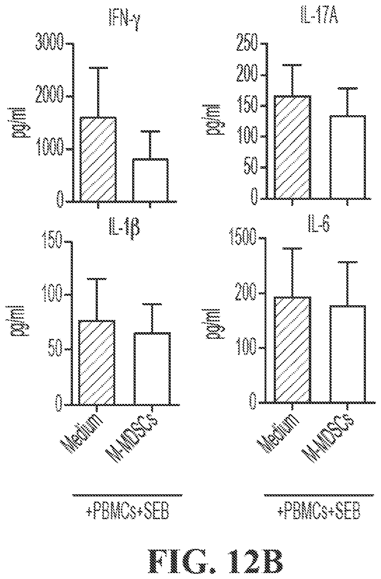

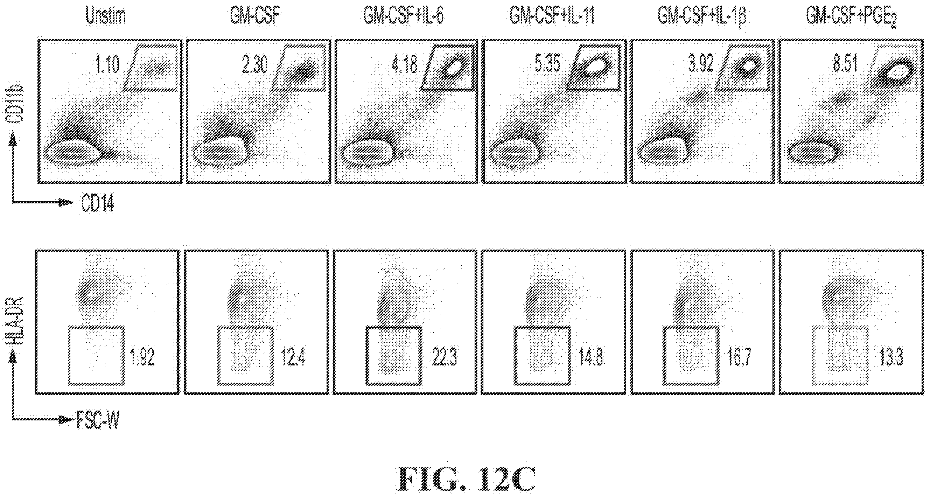

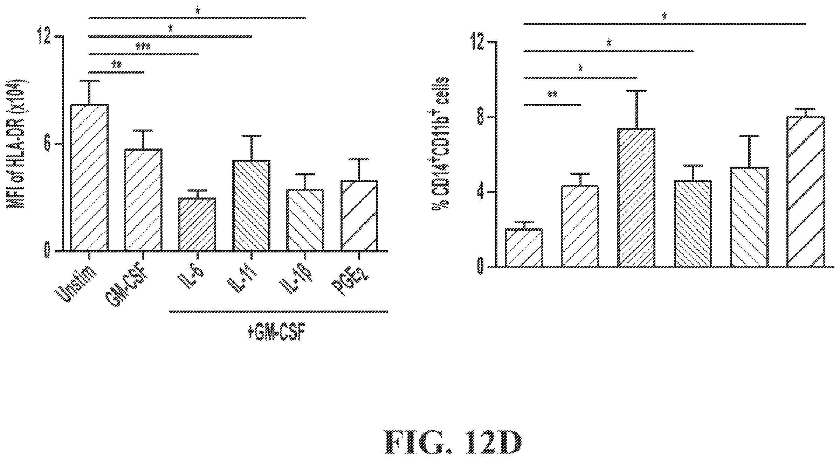

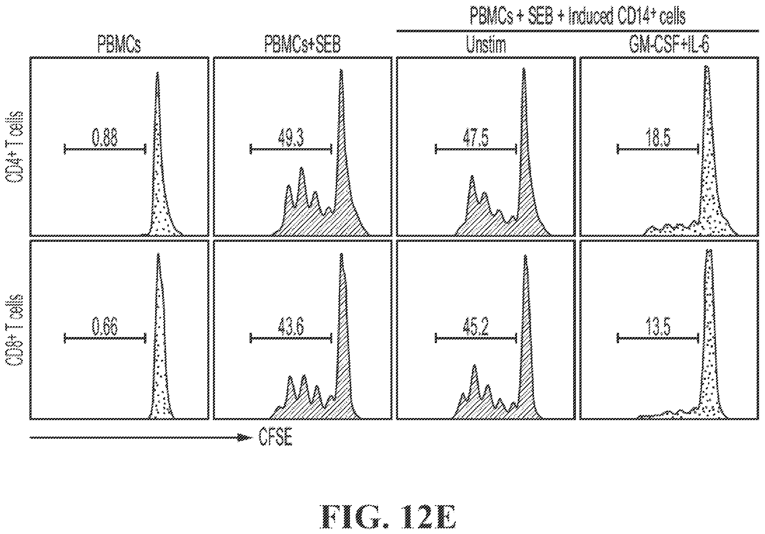

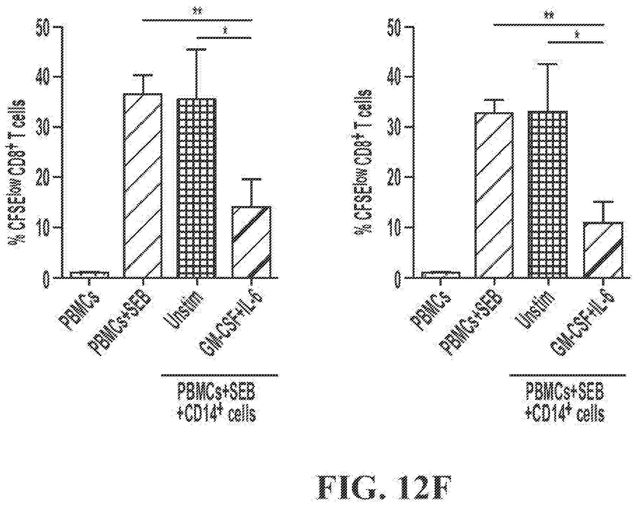

**FIGs. 12A-12F** show functional assessments of rhesus M-MDSCs and the generation of M-MDSC-like cells *in vitro.* **FIG. 12A** shows flow plots of M-MDSCs sorted from co-cultures of CFSE-labeled autologous PBMCs in the presence of SEB (left panel) and bar graphs of the percentage of proliferating T cells (n=4) (right panel). **FIG. 12B** shows graphs of the levels of the indicated cytokines in supernatants from cell cultures. **FIG. 12C** shows representative flow plots of PBMCs cultured alone or with different cytokine cocktails (top panel) and flow plots of the surface expression of HLA-DR on CD14+CD11b+ cells (bottom panel). Numbers in **FIG. 12C** top panel indicate the frequency of CD14+CD11b+ cells. **FIG. 12D** shows a graph of the percentage of generated CD14+CD11b+ cells after culture (n=9) (top panel) and a graph of the MFI of HLA-DR on derived CD14+CD11b+ cells (n=6) (bottom panel). **FIG. 12E** shows flow plots of CD14+ cells sorted from unstimulated or GM-CSF+IL-6-conditioned culture followed by co-culture with CFSE-labeled autologous PBMCs. T cell proliferation from one representative animal is shown. **FIG. 12F** includes bar graphs showing the percentage of proliferating T cells (n=5). Data is shown as mean ± SEM.

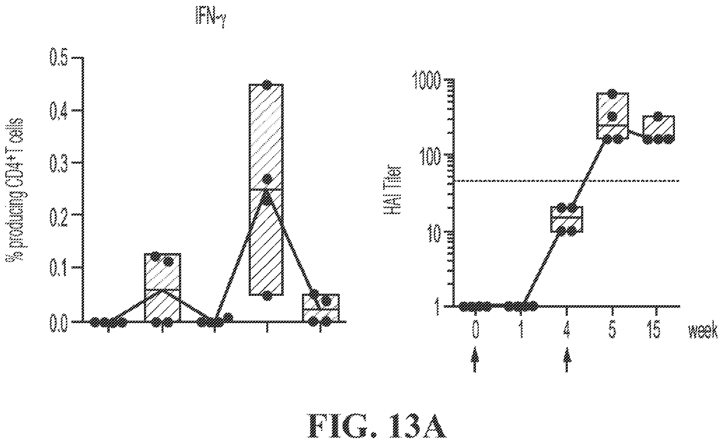

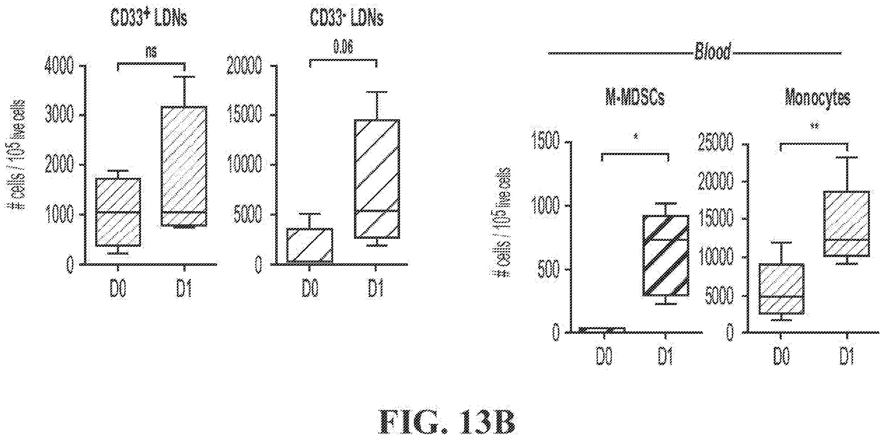

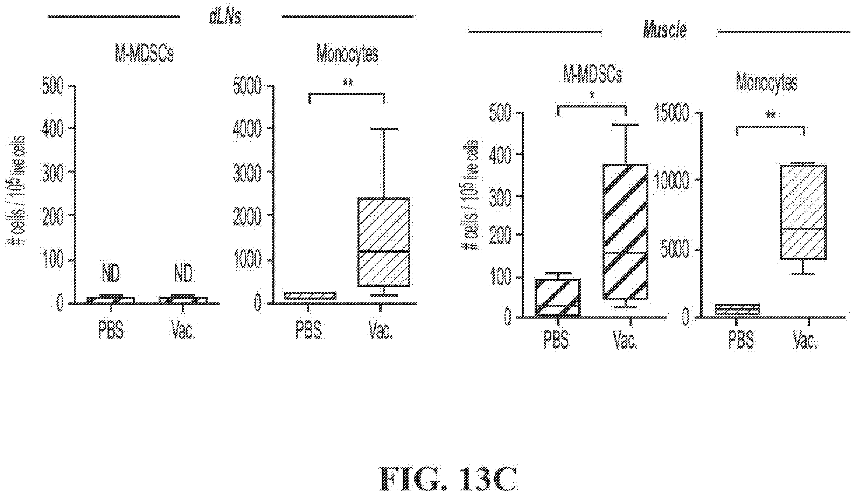

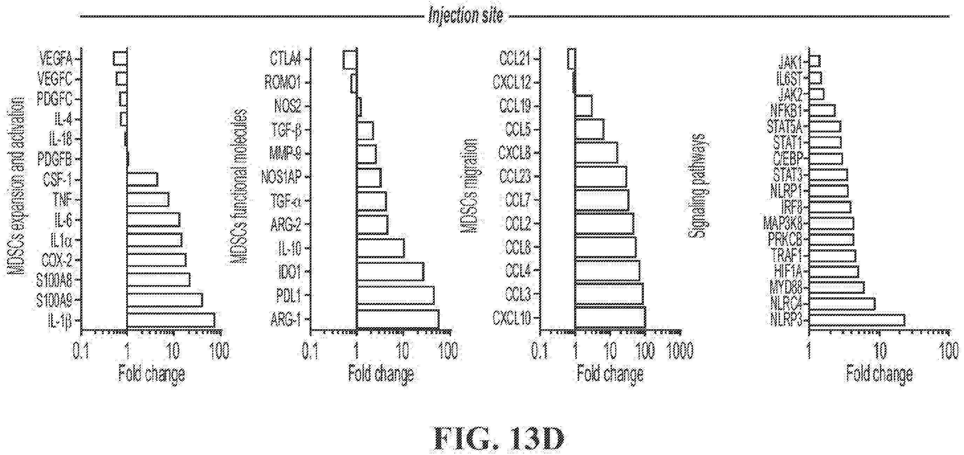

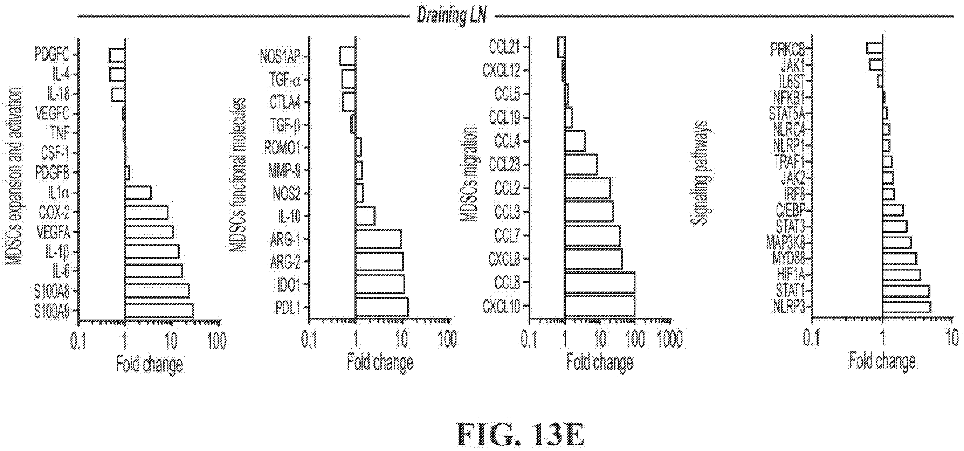

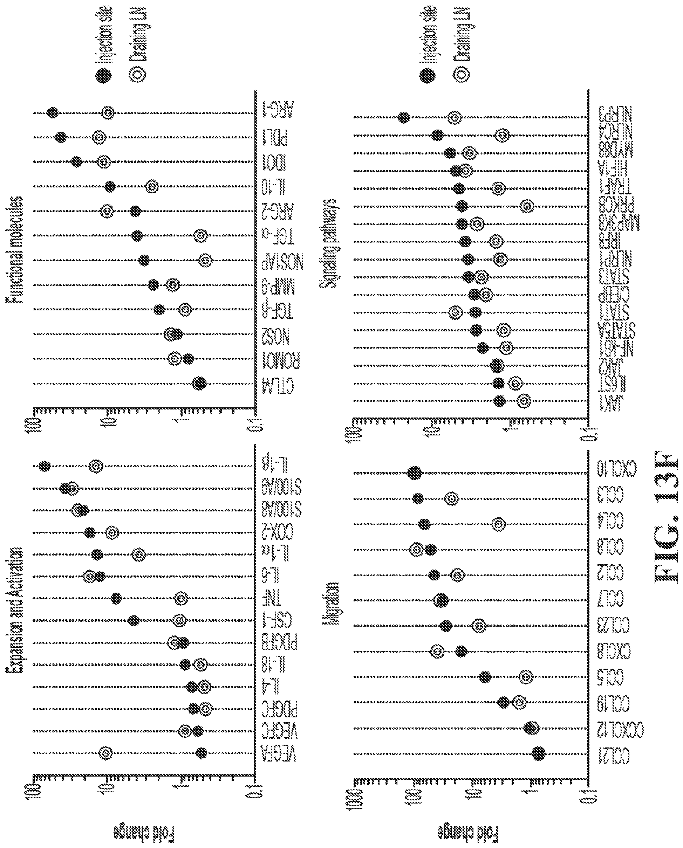

**FIGs. 13A-13F** show the infiltration of MDSCs and elevated expression of MDSC-relevant genes in vaccine injection sites. **FIG. 13A** shows a graph of the percentage of hemagglutinin-specific CD4+ memory T cells producing IFNγ (top panel) and a graph of hemagglutination inhibition (HAI) antibody titers (bottom panel). Rhesus macaques (n=4) were vaccinated at week 0 and 4 (arrows). The dotted line represents the reported protective level. **FIG. 13B** shows graphs of circulating levels of the indicated cell subsets before and at day 1 after vaccination. **FIG. 13C** shows graphs of the frequencies of M-MDSCs and monocytes in vaccine and PBS injection sites and the corresponding draining lymph nodes (dLNs) at day 1. **FIGs. 13D-13E** shows bar graphs of normalized mean expression levels of individual genes in vaccine injection sites **(****FIG. 13D****)** and dLNs **(****FIG. 13E****)** depicted as fold change relative to a PBS control. **FIG. 13F** shows graphs of the fold change in each gene between vaccine injection sites and dLNs.

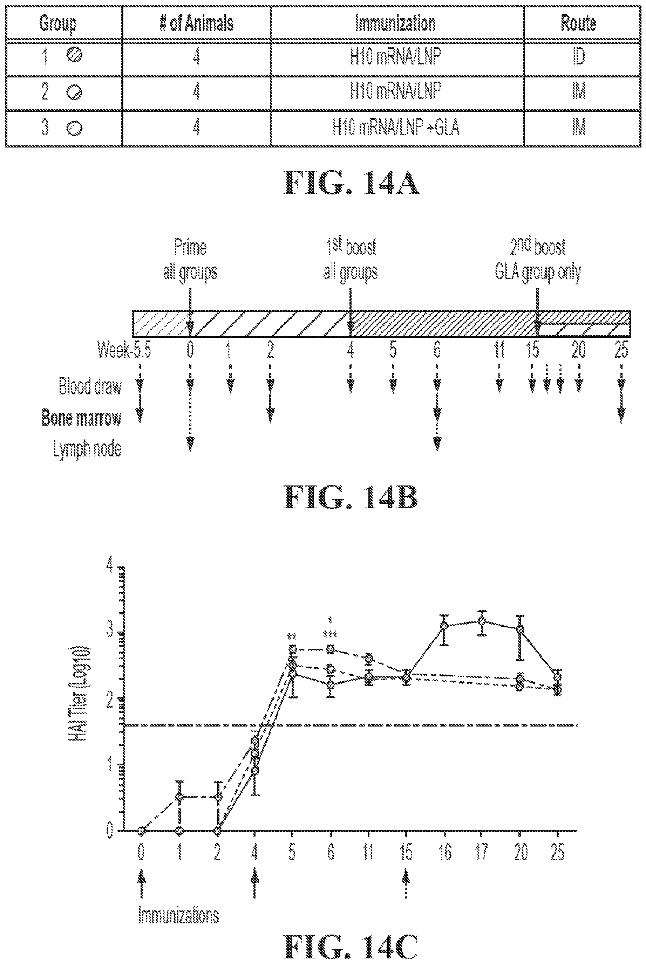

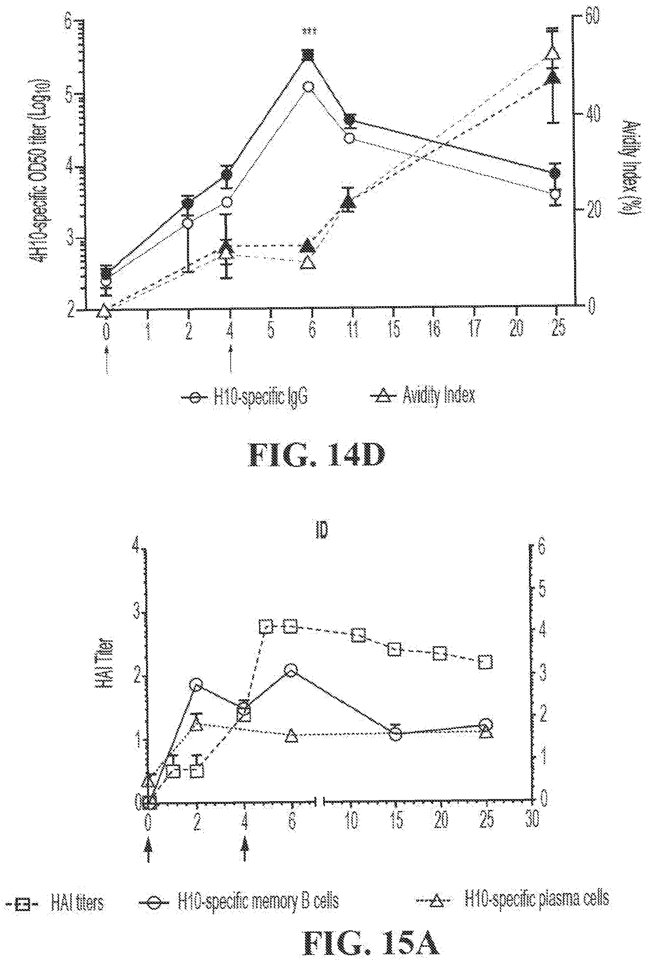

**FIGs. 14A-14D** show that an mRNA vaccine encoding H10 induces protective levels of antibodies. **FIG. 14A** shows immunizion contents of subject groups either intramuscularly (IM) or intradermally (ID) with an mRNA vaccine encoding full-length HA of H10N8 (A/Jiangxi-Donghu/346/2013) (H10) formulated in LNP. **FIG. 14B** shows the immunization schedule. **FIG. 14C** is a graph showing all animals induced neutralizing antibody titers against HA above the accepted level of protection for seasonal influenza vaccination, as measured by hemagglutination inhibition assay (HAl). **FIG. 14D** is a graph showing that H10-specific IgG antibody titers were induced both in the IM and ID groups already after prime immunization, and were increasing at the time of the second immunization and peaked two weeks thereafter.

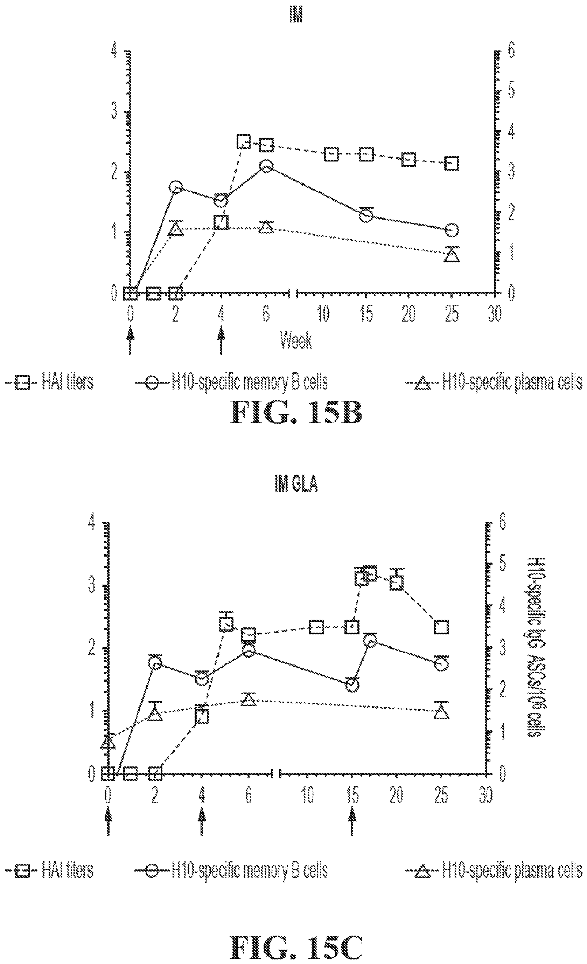

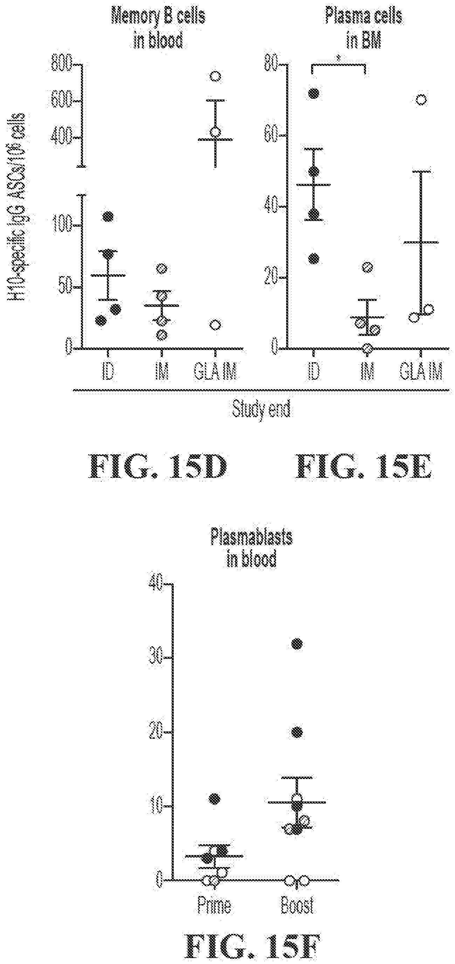

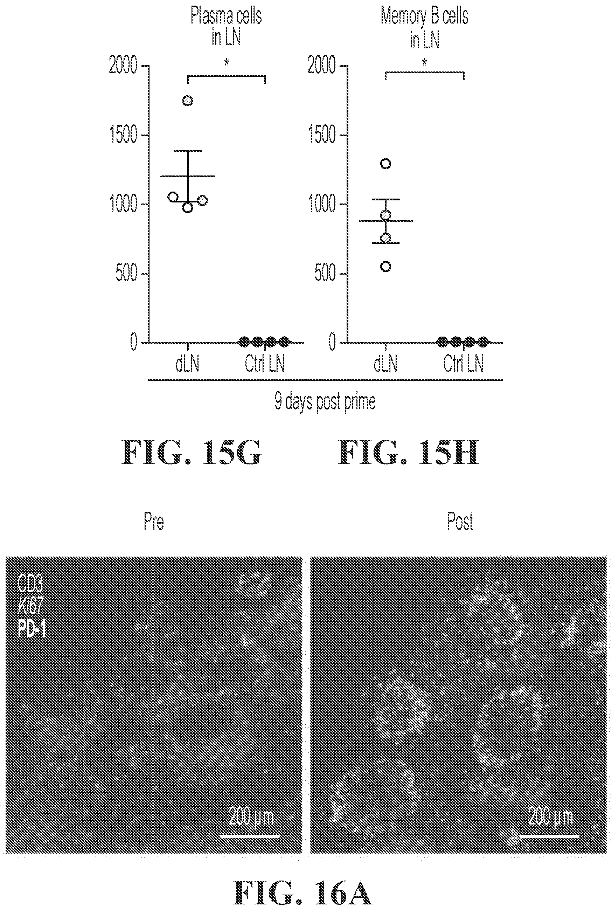

**FIGs. 15A-15H** show rapid and sustained B cell responses after mRNA vaccination. **FIG. 15A-15C** are graphs showing characterization of the kinetics of the B cell responses at the cellular level, and the frequency of H10-specific memory B cells as determined by ELISpot. **FIG. 15D** is a graph showing that, by study end at 25 weeks, the IM and ID groups showed similar levels of circulating H10-specific memory B cells, whereas the GLA group had higher levels due to a more recent boost. **FIG. 15E** shows that the number of H10-specific PCs declined to significantly lower levels in the IM group compared to the ID group at study end. **FIG. 15F** shows that the numbers of H10-specific plasmablasts in some of the animals one week after immunization were close to the limit of detection after the prime immunization but increased to readily detectable levels after the boost immunization. **FIGs. 15G-15H** are graphs showing that priming of vaccine-specific T cells occurs in the vaccine-draining LNs by collection of vaccine-draining LNs nine days after prime immunization and compared with control LNs that did not drain at the vaccine delivery site.

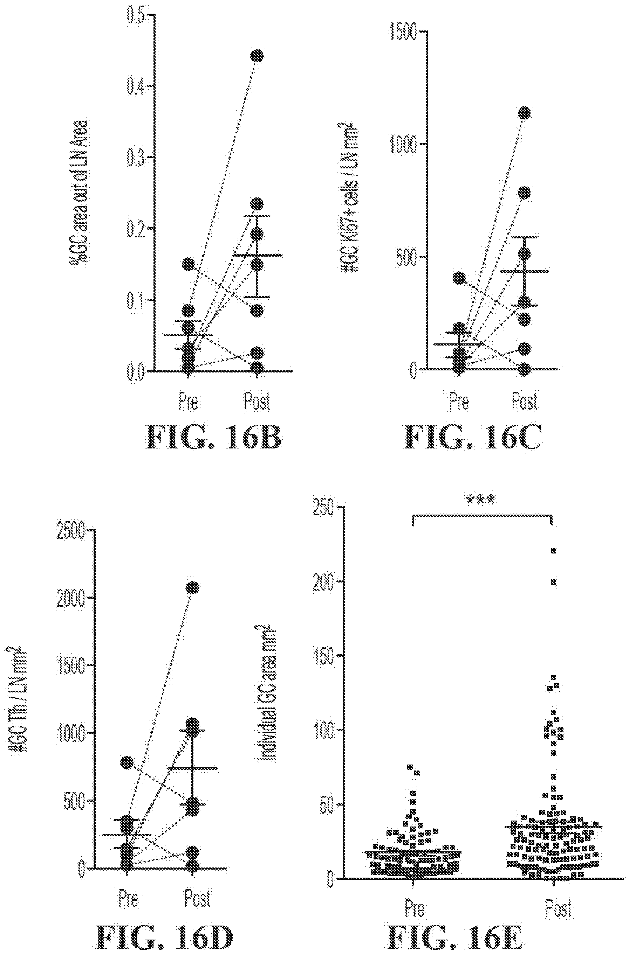

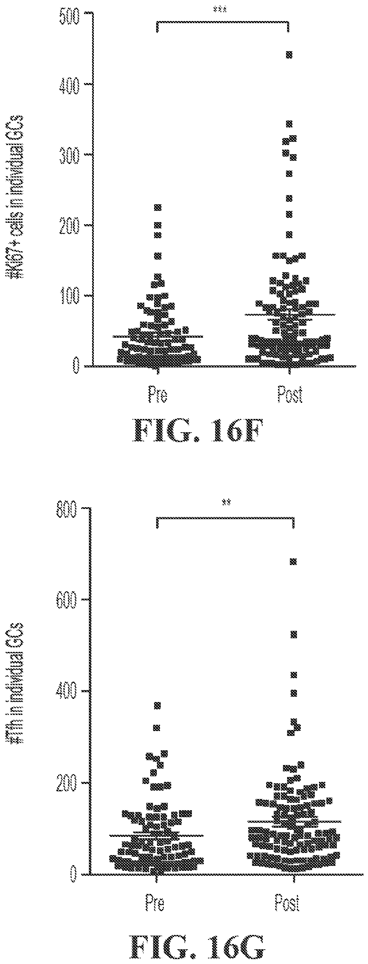

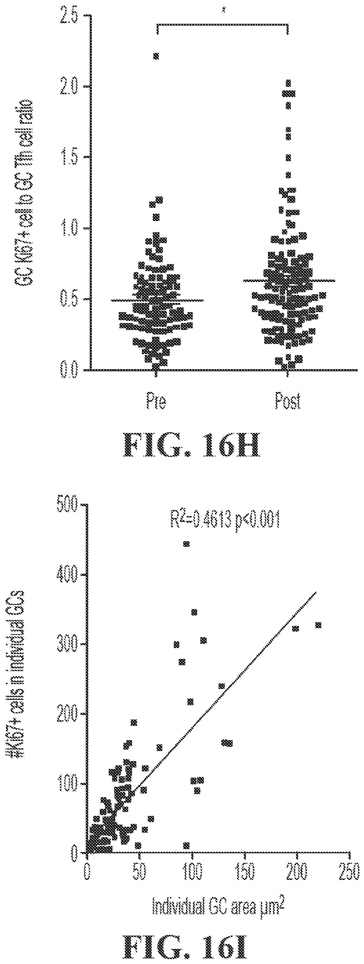

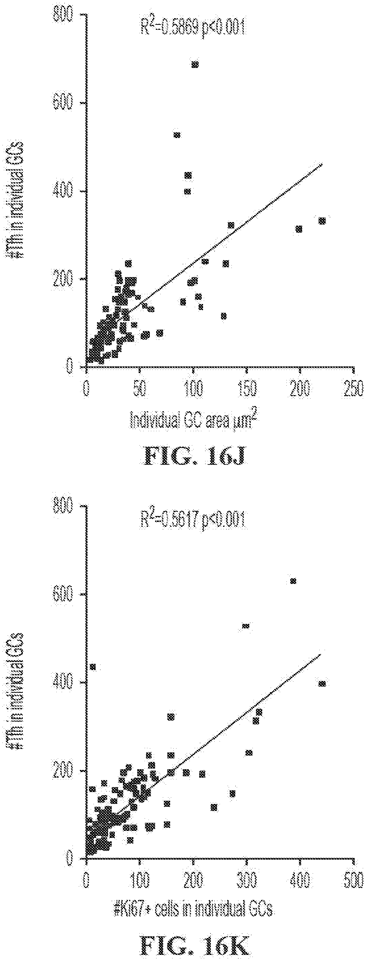

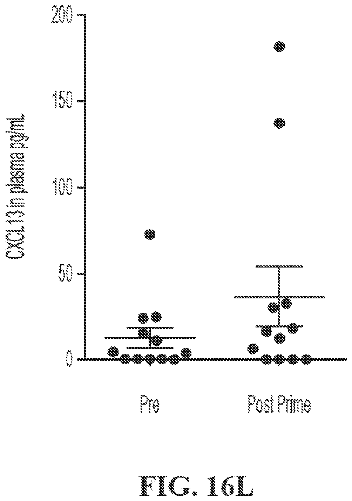

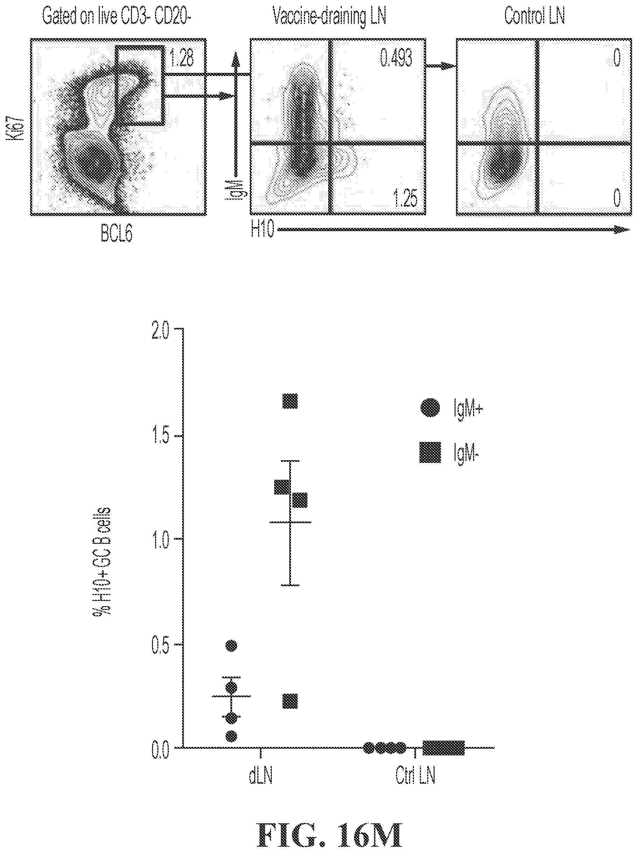

**FIGs. 16A-16M** show germinal center formation in vaccine-draining lymph nodes. **FIG. 16A** is a photo of immunofluorescence and confocal imaging of cryosections quantifing the area of germinal centers (GCs), the numbers of PD-1+ Tfh cells and proliferating Ki67+ cells within individual GCs. **FIG. 16B** shows that, in five out of the seven animals, there was an increase in the GC area/LN area ratio post-immunization. **FIGs. 16C-16D** show that there was also an increase in the number of GC Ki67+cells/LN area ratio and the number of GC Tfh cells/LN area ratio. **FIG. 16E** shows that the area of individual GCs was significantly increased post-vaccination. **FIGs. 16F-16G** show that Tfh cells and GC Ki67+ cells were also observed within individual GCs. **FIG. 16H** shows that a significant increase in the Ki67+ cell/Tfh cell ratio post-vaccination was observed in individual GCs. **FIG. 16I** shows that there was a strong correlation between the number of Ki67+ cells and the area within each GC (p<0.001 R2=0.4613). **FIG. 16J** shows that this was also found for the number of GC Tfh cells and the GC area of individual GCs (p<0.001 R2=0.5869). **FIG. 16K-16L** shows that Tfh cell numbers correlated with Ki67+ cell numbers within each GC (p<0.001 R2=0.5617). **FIG. 16M** shows that no H10-specific GC B cells could be detected in control non-draining LNs, but both unswitched IgM+ and class-switched IgM- BCL6+Ki67+ GC H10-specific B cells were detected in vaccine-draining LNs nine days after prime immunization.

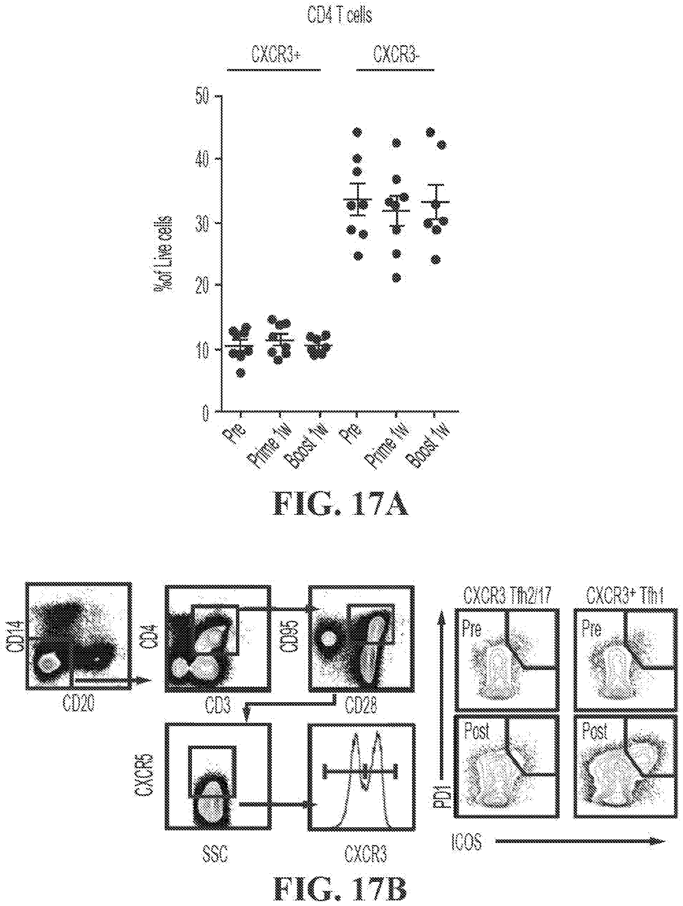

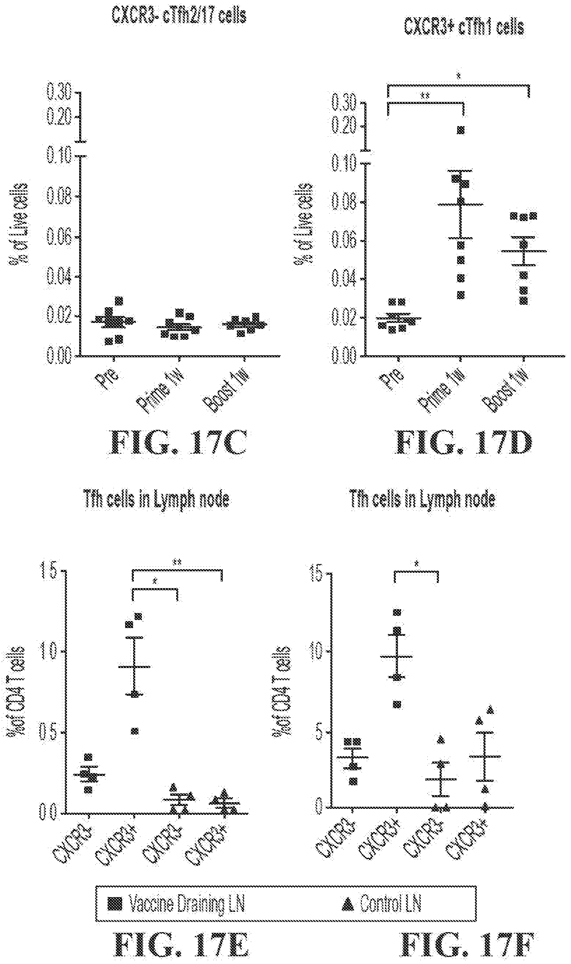

**FIGs. 17A-17F** show that circulating H10-specific ICOS+ PD-1+CXCR3+ T follicular helper cells are induced after vaccination and correlate with antibody avidity. **FIG. 17A** shows that there was no general increase in CXCR3+ or CXCR3-total CD4+ T cells. **FIG. 17B** shows analysis of CXCR5+ICOS+PD-1 +CXCR3+/- cTfh cells within the central memory (CD28+CD95+) CD4+T cell population. **FIGs. 17C-17D** show that, while there was no increase in CXCR3- cTfh cells, there was a significant increase in the number of CXCR3+ cTfh cells both after prime and boost. **FIGs. 17E-17F** show that the frequency of Tfh cells was increased in the vaccine-draining LNs compared to control non-draining LNs and the CXCR3+ Tfh cells in vaccine-draining LNs also expressed Ki67 at higher levels than the CXCR3- Tfh cells in the same LNs as well as compared to CXCR3+/- Tfh cells from the control LNs.

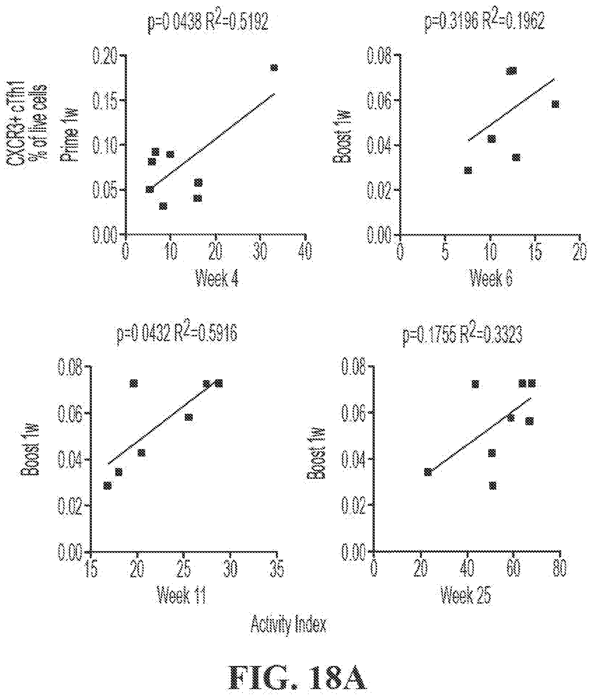

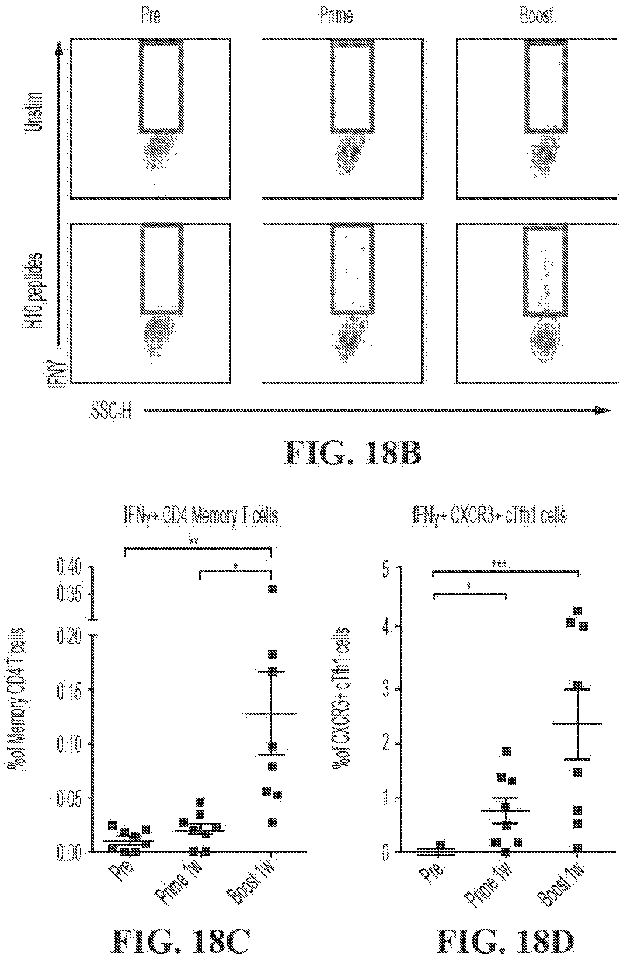

**FIGs. 18A-18D** show that the H10/LNP vaccine administration induces H10-specific cTfh cells of the CXCR3+ Th1-polarized profile that correlates with the avidity of H10-specific IgG antibodies. **FIG. 18A** shows the number of CXCR3+ cTfh cells after boost showed a trend of correlation with antibody avidity at week 6 and 25 and correlated significantly at week 11 (p=0.0432 R2=0.5916). **FIG. 18B** shows the specificity of the cTfh cells -PBMCs stimulated with H10 overlapping peptides for an intracellular cytokine assay. **FIG. 18C** shows there were few H10-specific cells evidenced by IFNγ production within the total CD4+ central memory T cell population one week after prime, but that there was a clear increase one week after boost. **FIG. 18D** shows that a significant increase in the number of IFNγ+CXCR3+ cTfh cells one week after prime and boost could still be observed.

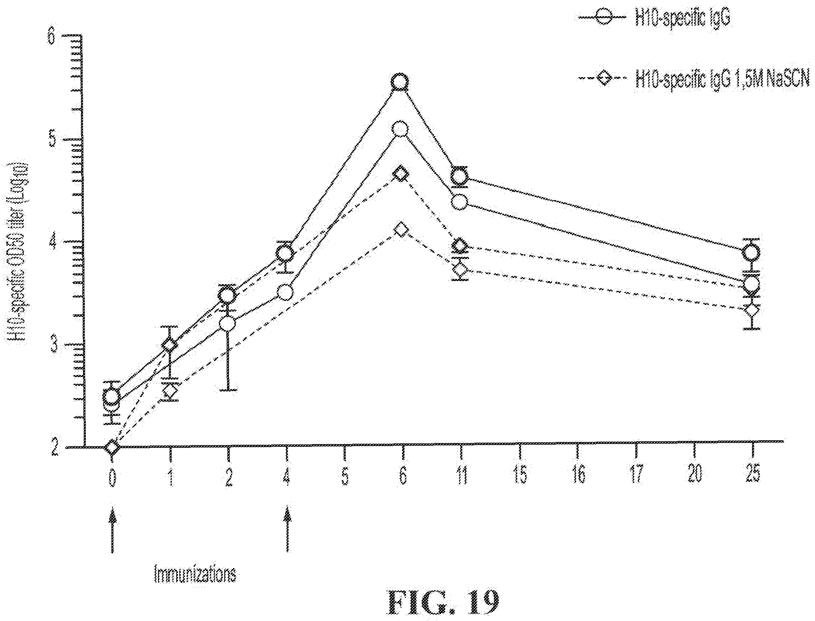

**FIG. 19** is a graph showing H10 specific IgG production.

### DETAILED DESCRIPTION

Although attempts have been made to produce functional RNA vaccines, including mRNA vaccines and self-replicating RNA vaccines, the therapeutic efficacy of these RNA vaccines have not yet been fully established. Quite surprisingly, the inventors have discovered a new metric, differential T cell activation potential (dTCAP), which may be used to determine the quality of a vaccine and whether or not it generates a therapeutic response with minimal to no toxicity. The formulations described herein have demonstrated significant unexpected *in vivo* immune responses sufficient to establish the efficacy of functional mRNA vaccines as prophylactic and therapeutic agents.

Self-replicating RNA vaccines rely on viral replication pathways to deliver enough RNA to a cell to produce an immunogenic response. Likewise, DNA vaccines rely on continuous expression mechanisms to produce therapeutically useful quantities of antigen. It is more challenging to deliver mRNA that does not have a self-replicating feature. Specific formulations have been required to achieve effective delivery. The formulations of the disclosure do not require viral or other artificial replication to produce enough protein to result in a strong immune response. The mRNA of the disclosure are not self-replicating RNA and do not include components necessary for viral replication. The vaccines of the disclosure are capable of providing efficient antigen production with minimal toxicity. Methods for determining optimal formulations for delivering mRNA to achieve these desirable properties have bene discovered according to aspects of the disclosure.

The mRNA vaccines of the disclosure are compositions, including pharmaceutical compositions. The disclosure also encompasses methods for the selection, design, preparation, manufacture, formulation, and/or use of mRNA vaccines. Also provided are systems, processes, devices and kits for the selection, design and/or utilization of the mRNA vaccines described herein.

The invention involves, in some aspects, a novel threshold measurement, the dTCAP, which can be used to evaluate the quality of a vaccine. In the invention, mRNA formulations which have been found to significantly enhance the effectiveness of mRNA vaccines, including chemically modified and unmodified mRNA vaccines and reduced toxicity, can be determined using the dTCAP. Notably, the dTCAP may be used as an indicator of the quality of the vaccine. As used herein, "quality of vaccine" refers to the nature of the immune response generated in response to vaccine administration, i.e., whether the vaccine will lead to enhanced protective immunity against the antigen in the subject with minimal to no sustained toxicity.

Much effort in refining mRNA vaccines has been focused on introducing modifications to increase mRNA stability and reducing innate immune activation that may inhibit translation of encoded proteins (Anderson et al., Nucleic Acids Res., 39(21): 9329-38 (2011); Kariko et al., Immunity, 23(2): 165-75 (2005)). For example, non-replicating mRNA constructs containing modified bases and a donor methyl group on the capped RNA for increased translation efficiency have been developed (Kuge et al., Nucleic Acids Res., 26(13):3208-14 (1998)). Such modified mRNA constructs have shown promise for regenerative (Zangi et al., Nat. Biotechnol., 31(10)898-907 (2013); Warren et al., Cell Stem Cell, 7(5): 618-30 (2010)) and cancer therapies (Kranz et al., Nature, 534(7607): 396-401 (2016); Wang et al., Molecular Therapy: J of ASGT, 21(2):358-67 (2013)). The use of potent delivery systems for mRNA vaccines enables dose reduction without compromising vaccine responses, demonstrating that there is no need for an adjuvant. Formulation of mRNA with liposomes (Kranz et al., Nature, 534(7607): 396-401 (2016)) or protamine (Rettig et al., Blood, 115(22): 4533-4541 (2010)) has been shown to stimulate RNA sensors like TLR7 in mouse dendritic cells (DCs), demonstrating the intrinsic immunogenic property of mRNA. Further, lipid nanoparticles (LNPs) as delivery systems for RNA have already been tested in clinical therapy trials (Coelho et al., N. Engl. J. Med., 369(9): 819-29 (2013)). It has recently been shown that mRNA encoding full-length hemagglutinin H10 of H10N8 (A/Jiangxi-Donghu/346/2013) influenza A virus encapsulated in LNP induces potent and sustained immune responses and neutralization in mice, ferrets and cynomolgus macaques (Bahl et al., Molecular Therapy: J of ASGT, 2017).

The *in vivo* innate immune mechanisms by which mRNA vaccines generate potent adaptive immunity are largely unknown. Information on the specific target cells responsible for translation of the mRNA at the site for administration or in the draining lymph nodes (LNs) is lacking as well as the type of cellular activation of target cells.

A number of conditions associated with inflammation, autoimmune disease and cancer lead to expansion of myeloid-derived suppressor cells (MDSCs) that play a key role in regulating T cell responses. MDSCs represent a heterogeneous population of innate immune cells with three main features: myeloid origin, immature state and suppressive effect of T cell responses in particular (Gabrilovich and Nagaraj, Immunology, 9: 162-64 (2009)). MDSCs interfere with the functions of T cells and NK cells either by direct receptor-mediated cell-cell contact, via the release of suppressive mediators, or disrupting the contact between other innate cell subsets e.g. dendritic cells with T cells or NK cells (Ostrand-Rosenberg et al., Seminars in Cancer Biol., 22: 275-81 (2012)).

Surprisingly it has been found that vaccines comprising an mRNA encoding an antigen formulated in an optimized carrier differentially recruit T cell suppressors (i.e., MDSCs) and T cell activators (e.g., non-MDSC monocytes), an effect which may be measured to determine the quality of a vaccine. Further, the information represents a powerful model to reveal local immune events after administration of encapsulated mRNA vaccines *in vivo* from the injection site to the draining lymph nodes to increase an understanding of how mRNA vaccines target key antigen presenting cells to generate vaccine-specific T cell and B cell responses.

The invention is based, in one aspect, on a new method for characterizing mRNA vaccines. It has been discovered that mRNA formulations can produce migration of unique populations of inflammatory cells at local tissue sites following administration. A set of threshold values associated with the balance between T-cell activation and suppressor cells have been identified. These threshold values can be used as benchmarks to analyze the quality of a putative vaccine formulation. Numerous formulations can be screened and optimized based on their relationship to the threshold values. For instance, each unique mRNA may be matched with a suitable formulation that provides the best quality profile based on this analysis.

Prior to the invention, a suitable assay for identifying both antigenicity and tolerability did not exist. The assays that are currently being used typically evaluate antigenicity and don't evaluate tolerability. Additionally, the assays of the invention can determine whether particular formulations can further enhance antigenicity by reducing the influx of suppressor cells in important tissue sites such as the lymph nodes. The methods of the invention provide a much more accurate and efficient way for characterizing the potential efficacy of vaccines.

Thus, in some aspects, the invention is a method of analyzing a vaccine to determine the quality of the vaccine. The quality is assessed by determining whether a vaccine with a specific formulation meets or exceeds a minimal threshold dTCAP. In other words a quantifiable ratio, absolute value or percentage of key factors in a local inflammatory response driven by local administration of the mRNA vaccine can be determined. The key factors include T-cell activators and suppressors and their relative numbers at different sites in a subject. Ideally, a local immune response at the injection site has a higher value of suppressor cells and lower activator cells and conversely the local immune response at a site such as a draining lymph node has few to no T-cell suppressors and a significant number of activator cells. The most efficacious vaccines may exhibit both of these properties. The present invention is set out in the appended claims.

The threshold dTCAP may be determined to provide a quantitative or qualitative assessment of a vaccine candidate or putative vaccine. As used herein, the threshold dTCAP is a measure of the amount of T cell suppressors and/or T cell activators present in a local population of inflammatory cells at one or more sites exposed to a vaccine. In some embodiments, the sites comprise the injection site and/or a draining lymph node. A threshold dTCAP is reached when the local population of inflammatory cells at an injection site of the vaccine has a higher T cell suppression value (tolerability) than T cell activation value (antigenicity) and/or when the local population of inflammatory cells at a draining lymph node has a lower T cell suppression value than T cell activation value. A threshold dTCAP may be calculated as the amount of T-suppis relative to the T-supp_{LN} (i.e., as a ratio, percent difference, total amount, or normalized to tissue, such as blood) and/or the amount of injection site tolerability (MDSCs) to antigenicity (non-MDSC monocytes) present in the injection site and/or draining lymph node (i.e., as a ratio, percent difference, total amount, or normalized to a tissue, such as blood).

In one embodiment, a representative sample of cells obtained, e.g., from a particular tissue site in a subject, e.g., a patient or an animal that has been injected with a vaccine to be evaluated, are enumerated, e.g., by using specific staining reagents. Using this methodology, different types of inflammatory cells can be quantitated at the particular tissue site. A local population of inflammatory cells is determined at a particular tissue site. For instance, a local population of inflammatory cells can assessed at the injection site, at one or more lymph nodes, including the draining lymph nodes and in blood. Inflammatory cells include suppressor cells, e.g. MDSCs, and activator cells, e.g., monocytes. In one embodiment, MDSCs and monocytes can be detected as described in more detail below.

T cell suppression value refers to an amount of cells capable of suppressing T cell activation in a particular tissue site. The T cell suppression value is a measure of tolerability to the vaccine being tested. When a high number of suppressor cells is present in a tissue site, those cells dampen the local immune response, preventing it from causing toxic inflammation. For instance too much inflammation at the injection site can cause injection site toxicity. Using the methods and formulations described herein it is possible to design specific mRNA vaccines which have minimal to no injection site toxicity.

MDSCs were first identified in cancer to support tumor progression via the dysregulation of immune responses in tumor microenvironment (Kumar et al., Trends in Immunology, 37: 208-20 (2016)). Recent studies have shown that MDSCs also appear in several other conditions including infection, transplantation, autoimmunity and hypertension (Gabrilovich and Nagaraj, Immunology, 9: 162-64 (2009); Shah et al., Circulation Res., 117: 858-69 (2015)). Therefore, the role of MDSCs in immune regulation appears to be widespread although several details of their functions remain elusive. Their suppressive function that contributes to immune deficiency has been proposed to have a protective effect in other contexts evidenced by preventing tissue damage, ameliorating inflammation or even facilitating clearance of pathogens (Pastula and Marcinkiewicz, Int J of Experimental Pathology, 92: 73-8 (2011)). To this end, it is currently unclear whether the numbers of MDSCs increase and influence T cell responses in temporary inflammation, such as after vaccine administration.

MDSCs have been intensively investigated in humans and mice in the recent years (Bronte et al., Nature Communications, 7: 12150 (2016)). In human blood, M-MDSCs can be identified as HLA-DR^{-/low}Lin⁻CD33+CD11b+CD14+CD15- and PMN-MDSCs as HLA-DR-Lin-CD33+CD15+CD14- (Bronte et al., Nature Communications, 7: 12150 (2016)). Notably, human PMN-MDSCs are thought to be a unique subset of neutrophils within the heterogeneous low-density neutrophils (LDNs) that co-segregate with peripheral blood mononuclear cells (PBMCs) after Ficoll centrifugation. This is in contrast to normal density neutrophils (NDNs), which sediment together with erythrocytes and consists of a homogenous population with immune-stimulatory function (Sagiv et al., Cell Reports, 10: 562-73 (2015)). It is currently difficult to discriminate PMN-MDSCs from other LDNs due to limited specific markers available. Human PMN-MDSCs are therefore usually defined as the LDN population as a whole. Other MDSCs are also possible, for example, immature HLA-DR-Lin-CD33+CD15-CD14- progenitors named early-stage (E)-MDSCs were recently described in humans as a subset that may differentiate into M-MDSCs or PMN-MDSCs (Bronte et al., Nature Communications, 7: 12150 (2016); Dumitru et al., Cancer Immunology, 61: 1155-67 (2012)).

The term "differential" as used herein refers to a comparison between two or more data points. Different data points may be, for instance, different numbers or types of cells at one tissue site or at different tissue sites. A differential analysis provides the ability to evaluate multiple pieces of information within a single value, ratio, or percentage. In this instance the differential analysis provides a quantitative value(s) which provides threshold information about the tolerability and antigenicity of a vaccine.

An example of the data that may be used to determine the threshold dTCAP is given in FIGs. 13B-13C. FIG. 13B shows levels of MDSCs and monocytes in blood before and one day after vaccination. These values may be used to normalize the results shown in FIG. 13C (MDSCs and monocytes at the site of injection (muscle) and in draining lymph nodes). Threshold differential T cell activation may be determined in a mammalian subject, including humans and other mammals, including non-human primates.

The threshold dTCAP may be determined by several different methods. It may involve a comparison (ratio, percentage, or absolute number) of a T-suppis to a T-supp_{LN}. It may also involve a comparison of a T cell suppression value to a T cell activation value at the injection site and/or the draining lymph node. For example, the comparison of T-activLN to T-suppLN can be determined. It may also be a normalized comparison of a T cell suppression value and/or a T cell activation value at the same or different tissue sites. Other methods of comparison may be used, to produce a threshold dTCAP score. In the invention, the dTCAP is determined as set out in the appended claims.

Thus, in some embodiments the threshold dTCAP is determined, such that a local population of inflammatory cells at an injection site of the vaccine has a higher T cell suppression value (tolerability) than T cell activation value (antigenicity) and/or when the local population of inflammatory cells at a draining lymph node has a lower T cell suppression value than T cell activation value. In some embodiments, the dTCAP is a ratio of at least 10:1. In other embodiments, the dTCAP is a ratio of at least 6:1, 7:1, 8:1, 9:1, 10:1, 15:1, 20:1, 30:1, 40:1, 50:1, 60:1, 70:1, 80:1, 90:1, 100:1, 125:1, 150:1, 175:1, 200:1, 225:1, 250:1, 275:1, 300:1, 325:1, 350:1, 375:1, 400:1, 425:1, 450:1, 475:1, 500:1, 600:1, 700:1, 800:1, 900:1, or 1000:1.

The threshold dTCAP may be calculated as the amount of T-suppis relative to the T-supp_{LN} (*i.e.*, as a ratio, percent difference, total amount, or normalized to tissue, such as blood) or the amount of injection site tolerability (MDSCs) to antigenicity (non-MDSC monocytes) present in the injection site and/or draining lymph node (*i.e*., as a ratio, percent difference, total amount, or normalized to a tissue, such as blood). The T cell suppression value may, in some embodiments, be a measure of the amount of suppressor cells, i.e., MDSCs present in a sample. As shown in the data described herein the amount of MDSCs in some sites is undetectable. When cells such as MDSCs are undetectable, a baseline minimum can be selected and used to calculate the dTCAP as a ratio or percentage. For instance, when the inflammatory cell being measured is MDSC, a baseline level of 1 MDSC/10⁵ live cells may be used in the calculation.

In some embodiments, the amount of MDSCs present at the injection site is 0-10, 10-100, 10-200, 10-300, 10-400, 10-500, 10-600, 10-700, 10-800, 10-900, 10-1000, 10-1500, 50-100, 50-200, 50-300, 50-400, 50-500, 50-600, 50-700, 50-800, 50-900, 50-1000, 50-1500, 100-200, 100-300, 100-400, 100-500, 100-600, 100-700, 100-800, 100-900, 100-1000, 100-1500, 200-300, 200-400, 200-500, 200-600, 200-700, 200-800, 200-900, 200-1000, 200-1500, 300-400, 300-500, 300-600, 300-700, 300-800, 300-900, 300-1000, 300-1500, 400-500, 400-600, 400-700, 400-800, 400-900, 400-1000, 400-1500, 500-600, 500-700, 500-800, 500-900, 500-1000, 500-1500, 600-700, 600-800, 600-900, 600-1000, 600-1500, 700-800, 700-900, 700-1000, 700-1500, 800-900, 800-1000, 800-1500, 900-1000, 900-1500, or 1000-1500 MDSCs/10⁵ live cells.

In some embodiments, the amount of MDSCs present in the draining lymph node is 0-10, 0-15, 1-2, 1-3, 1-4, 1-5, 1-6, 1-7, 1-8, 1-9, 1-10, 1-11, 1-12, 1-13, 1-14, 1-15, 2-3, 2-4, 2-5, 2-6, 2-7, 2-8, 2-9, 2-10, 2-11, 2-12, 2-13, 2-14, 2-15, 3-4, 3-5, 3-6, 3-7, 3-8, 3-9, 3-10, 3-11, 3-12, 3-13, 3-14, 3-15, 4-5, 4-6, 4-7, 4-8, 4-9, 4-10, 4-11, 4-12, 4-13, 4-14, 4-15, 5-6, 5-7, 5-8, 5-9, 5-10, 5-11, 5-12, 5-13, 5-14, 5-15, 6-7, 6-8, 6-9, 6-10, 6-11, 6-12, 6-13, 6-14, 6-15, 7-8, 7-9, 7-10, 7-11, 7-12, 7-13, 7-14, 7-15, 8-9, 8-10, 8-11, 8-12, 8-13, 8-14, 8-15, 9-10, 9-11, 9-12, 9-13, 9-14, 9-15, 10-11, 10-12, 10-13, 10-14, 10-15, 11-12, 11-13, 11-14, 11-15, 12-13, 12-14, 12-15, 13-14, 13-15, or 14-15 MDSCs/10⁵ live cells.

In some embodiments, the dTCAP is determined as the ratio of normalized T cell suppression values in local populations of inflammatory cells at an injection site to a draining lymph node. For normalization, each value may be normalized to T cell suppression values in local populations of inflammatory cells in tissue (e.g., blood). A normalized value can be generated from raw data by using a Z-score and min-max scaling. A simple method for generating a normalized ratio or percentage from data (normalized against same factor in blood) involves dividing the raw value or average value from the tissue site of interest by the raw value or average value from blood. For example, the average MDSC (or monocyte) concentration in muscle (injection site) may be divided by the average MDSC (or monocyte) concentration in blood to produce a value. A similar calculation can be made for the same factor at the draining lymph nodes.

The dTCAP may also be determined as a percentage of total T cell suppression value. In some embodiments, the percentage of T cell suppressor inflammatory cells at the injection site and the draining lymph node site is 100%.

In some embodiments, the dTCAP is calculated based on a comparison between T cell suppression values and T cell activation values. A T cell activation value refers to an amount of cells capable of activating T cells that are present in a particular body site.

Antigenicity, as measured by the amount of T cell activators, provides valuable information about the efficacy of a vaccine. In contrast, tolerability provides information about the undesirable side effects of a vaccine, such as injection site inflammation.

In some embodiments, the dTCAP is calculated as the ratio of draining lymph node antigenicity to draining lymph node tolerability. The indicator of draining lymph node antigenicity is the amount of T cell activators. In the invention, the T cell activators are T follicular helper cells (Tfh). The indicator of draining lymph node is the amount of inflammation suppressing cells such as MDSCs present at the draining lymph node. In optimal circumstances the amount of MDSCs present at the draining lymph node may even be undetectable. In instances where the amount of MDSCs present at the draining lymph node is undetectable, a baseline minimum value may be used to calculate the ratio. A ratio of draining lymph node antigenicity to draining lymph node tolerability of greater than 1000:1 may indicate the threshold dTCAP. In other embodiments, a ratio of 1000:1, 1250:1, 1500:1, 1750:1, 2000:1, 3000:1, 3500:1, 4000:1, 4500:1, or 5000:1 represent the threshold dTCAP.

In other embodiments, the dTCAP is determined as a percentage of total T cell suppressor cell and T activator cell population at a draining lymph node. In some embodiments, the percentage of T cell suppressor cell and T activator cell at a draining lymph node is 100%.

The level of MDSCs and non-MDSC monocytes can be determined at any point after vaccination. In some embodiments, MDSCs may be measured 1 day after vaccination. In another embodiment, the MDSCs are measured 1, 2, 3, 4, 5, 6, 7, 8, 9, 10, 11, 12, 13, 14, 15, 16, 17, 18, 19, 20, 21, 22, or 23 hours after vaccination. The MDSCs may be measured 1, 2, 3, 4, 5, 6, 7, 8, 9, 10, 12, 14, 16, 18, or 20 days following the vaccination.

The dTCAP, in some embodiments, may be used to indicate the quality of the administered vaccine. Without wising to be bound by theory, it is thought that the inflammation induced by vaccine administration contributes to the expansion of circulating MDSCs, as the inflammatory microenvironment at, for example, a local injection site, recruits circulating MDSCs, which then produce immune suppressive mediators, preventing excess inflammation. With respect to vaccines, inflammation at the injection site is required to induce a sufficient vaccine response, but the inflammation should resolve quickly in order to avoid local or systemic side effects. Therefore, the presence of a threshold level of MDSCs, which produce immune suppressive mediators, can be predictive of a positive vaccine outcome, i.e., immunity against the administered antigen. As shown herein (e.g., Example 2), the MDSCs relative to other non-MDSC monocytes can be used to evaluate vaccine quality.

Quality may also be assessed using additional immunological biomarkers that lead to the production of high titer protective antibodies. Tfh cells and their circulating counterparts have emerged as key players for imprinting antibody responses. In particular, CXCR3+ cTfh cells have been shown to correlate with high-avidity antibodies against influenza after vaccination in humans. It has also been shown that cTfh cell levels can predict the seroconversion in humans to influenza vaccination.

It is demonstrated herein that the mRNA LNP vaccines specifically induced an increase in CXCR3+ cTfh cells, which correlated with IgG avidity and that this phenomenon could be readily measured in order to provide an assay to assess the quality of putative mRNA vaccines. CXCR3+ cTfh cells are important for selecting and expanding B cells of high affinity. It has been proposed that the main function of CXCR3+ cTfh cells is to select memory B cells of high affinity, leading to rapid expansion of this population upon new antigen encounter. Inducing vaccine-specific cTfh cells is a central mechanism in vaccine-mediated protection since these cells facilitate a quick re-stimulation of germinal center (GC) reactions and memory B cells upon antigen re-exposure. The discovery of antigen-specific cells within the CXCR3+ cTfh cell population, demonstrate that this new quality control parameter may be used with mRNA vaccines.

Thus, quality may also be assessed using a measurement of ICOS+PD-1+CXCR3+ T follicular helper cells at a draining lymph node, which provide an assessment of potential antibody avidity. Exemplary assays for detecting and measuring ICOS+PD-1+CXCR3+ T follicular helper cells at a draining lymph node are presented in the Examples.

In one embodiment, Tfh cells can be used as an indicator of T activator cells and the number of Tfh cells can be used to calculate a dTCAP as set forth above.

### I. Vaccines

Embodiments of the present disclosure provide RNA (e.g., mRNA) vaccines that include a polynucleotide encoding one or more antigens formulated in a carrier. mRNA vaccines, as provided herein may be used to induce a balanced immune response, comprising cellular and/or humoral immunity, without many of the risks associated with DNA vaccination. In some embodiments, a vaccine comprises at least one RNA (e.g., mRNA) polynucleotide having an open reading frame encoding an antigen. The mRNA vaccine of the present disclosure comprises a carrier. The term "carrier" denotes an organic or inorganic ingredient, natural or synthetic, with which the mRNA is combined to facilitate administration.

Thus, the invention relates to mRNA vaccines and is set out in the appended claims. The mRNA vaccines provide unique therapeutic alternatives to peptide based or DNA vaccines. When the mRNA vaccine is delivered to a cell, the mRNA will be processed into a polypeptide by the intracellular machinery which can then process the polypeptide into immunosensitive fragments capable of stimulating an immune response against the infectious disease or tumor.

The vaccines described herein include at least one ribonucleic acid (RNA) polynucleotide having an open reading frame encoding at least one antigenic polypeptide or an immunogenic fragment thereof (e.g., an immunogenic fragment capable of inducing an immune response to cancer or infectious disease). As used herein, the term "open reading frame", abbreviated as "ORF", refers to a segment or region of an mRNA molecule that encodes a polypeptide. The ORF comprises a continuous stretch of non-overlapping, in-frame codons, beginning with the initiation codon and ending with a stop codon, and is translated by the ribosome.

The vaccines may be traditional or personalized cancer or infectious disease vaccines. A traditional cancer vaccine, for instance, is a vaccine including a cancer antigen that is known to be found in cancers or tumors generally or in a specific type of cancer or tumor. Antigens that are expressed in or by tumor cells are referred to as "tumor associated antigens". A particular tumor associated antigen may or may not also be expressed in non-cancerous cells. Many tumor mutations are known in the art. Personalized vaccines, for instance, may include RNA encoding for one or more known cancer antigens specific for the tumor or cancer antigens specific for each subject, referred to as neoepitopes or patient specific epitopes or antigens. A "patient specific cancer antigen" is an antigen that has been identified as being expressed in a tumor of a particular patient. The patient specific cancer antigen may or may not be typically present in tumor samples generally. Tumor associated antigens that are not expressed or rarely expressed in non-cancerous cells, or whose expression in non-cancerous cells is sufficiently reduced in comparison to that in cancerous cells and that induce an immune response induced upon vaccination, are referred to as neoepitopes.

The disclosure also encompasses infectious disease vaccines. The antigen of the infectious disease vaccine is a viral or bacterial antigen. In some embodiments the infectious agent is a strain of virus selected from the group consisting of adenovirus; Herpes simplex, type 1; Herpes simplex, type 2; encephalitis virus, papillomavirus, Varicellazoster virus; Epstein-barr virus; Human cytomegalovirus; Human herpes virus, type 8; Human papillomavirus; BK virus; JC virus; Smallpox; polio virus; Hepatitis B virus; Human bocavirus; Parvovirus B19; Human astrovirus; Norwalk virus; coxsackievirus; hepatitis A virus; poliovirus; rhinovirus; Severe acute respiratory syndrome virus; Hepatitis C virus; Yellow Fever virus; Dengue virus; West Nile virus; Rubella virus; Hepatitis E virus; Human Immunodeficiency virus (HIV); Influenza virus; Guanarito virus; Junin virus; Lassa virus; Machupo virus; Sabia virus; Crimean-Congo hemorrhagic fever virus; Ebola virus; Marburg virus; Measles virus; Mumps virus; Parainfluenza virus; Respiratory syncytial virus; Human metapneumovirus; Hendra virus; Nipah virus; Rabies virus; Hepatitis D; Rotavirus; Orbivirus; Coltivirus; Banna virus; Human Enterovirus; Hanta virus; West Nile virus; Middle East Respiratory Syndrome Corona Virus; Japanese encephalitis virus; Vesicular exanthernavirus; and Eastern equine encephalitis.

In other embodiments, the virus is a strain of Influenza A or Influenza B or combinations thereof. In some embodiments, the strain of Influenza A or Influenza B is associated with birds, pigs, horses, dogs, humans or non-human primates. In some embodiments, the antigenic polypeptide encodes a hemagglutinin protein or fragment thereof. In some embodiments, the hemagglutinin protein is H1, H2, H3, H4, H5, H6, H7, H8, H9, H10, H11, H12, H13, H14, H15, H16, H17, H18, or a fragment thereof. In some embodiments, the hemagglutinin protein does not comprise a head domain (HA1). In some embodiments, the hemagglutinin protein comprises a portion of the head domain (HA1). In some embodiments, the hemagglutinin protein does not comprise a cytoplasmic domain. In some embodiments, the hemagglutinin protein comprises a portion of the cytoplasmic domain. In some embodiments, the hemagglutinin protein is truncated. In some embodiments, the truncated hemagglutinin protein comprises a portion of the transmembrane domain. In some embodiments, the amino acid sequence of the hemagglutinin protein or fragment thereof comprises at least 90%, 91%, 92%, 93%, 94%, 95%, 96%, 97% 98%, or 99% identify with any of the known amino acid sequences for influenza antigens. In some embodiments, the virus is selected from the group consisting of H1N1, H3N2, H7N9, and H10N8.

In some embodiments, the infectious agent is a strain of bacteria selected from Tuberculosis (Mycobacterium tuberculosis), clindamycin-resistant Clostridium difficile, fluoroquinolon-resistant Clostridium difficile, methicillin-resistant Staphylococcus aureus (MRSA), multidrug-resistant Enterococcus faecalis, multidrug-resistant Enterococcus faecium, multidrug-resistance Pseudomonas aeruginosa, multidrug-resistant Acinetobacter baumannii, and vancomycin-resistant Staphylococcus aureus (VRSA). In some embodiments, the bacterium is Clostridium difficile.

The mRNA vaccines of the disclosure may include one or more antigens. In some embodiments the mRNA vaccine is composed of 3 or more, 4 or more, 5 or more 6 or more 7 or more, 8 or more, 9 or more antigens. In other embodiments the mRNA vaccine is composed of 1000 or less, 900 or less, 500 or less, 100 or less, 75 or less, 50 or less, 40 or less, 30 or less, 20 or less or 100 or less cancer antigens. In yet other embodiments the mRNA vaccine has 3-100, 5-100, 10-100, 15-100, 20-100, 25-100, 30-100, 35-100, 40-100, 45-100, 50-100, 55-100, 60-100, 65-100, 70-100, 75-100, 80-100, 90-100, 5-50, 10-50, 15-50, 20-50, 25-50, 30-50, 35-50, 40-50, 45-50, 100-150, 100-200, 100-300, 100-400, 100-500, 50-500, 50-800, 50-1,000, or 100-1,000 antigens.

In some embodiments the mRNA vaccines and vaccination methods include epitopes or antigens based on specific mutations (neoepitopes) and those expressed by cancer-germline genes (antigens common to tumors found in multiple patients) or infectious agents.

An epitope, also known as an antigenic determinant, as used herein is a portion of an antigen that is recognized by the immune system in the appropriate context, specifically by antibodies, B cells, or T cells. Epitopes include B cell epitopes and T cell epitopes. B-cell epitopes are peptide sequences which are required for recognition by specific antibody producing B-cells. B cell epitopes refer to a specific region of the antigen that is recognized by an antibody. The portion of an antibody that binds to the epitope is called a paratope. An epitope may be a conformational epitope or a linear epitope, based on the structure and interaction with the paratope. A linear, or continuous, epitope is defined by the primary amino acid sequence of a particular region of a protein. The sequences that interact with the antibody are situated next to each other sequentially on the protein, and the epitope can usually be mimicked by a single peptide. Conformational epitopes are epitopes that are defined by the conformational structure of the native protein. These epitopes may be continuous or discontinuous, i.e. components of the epitope can be situated on disparate parts of the protein, which are brought close to each other in the folded native protein structure.

T-cell epitopes are peptide sequences which, in association with proteins on APC, are required for recognition by specific T-cells. T cell epitopes are processed intracellularly and presented on the surface of APCs, where they are bound to MHC molecules including MHC class II and MHC class I. The peptide epitope may be any length that is reasonable for an epitope. In some embodiments the peptide epitope is 9-30 amino acids. In other embodiments the length is 9- 22, 9-29, 9-28, 9-27, 9-26, 9-25, 9-24, 9-23, 9-21, 9-20, 9-19, 9-18, 10-22, 10-21, 10-20, 11-22, 22-21, 11-20, 12-22, 12-21, 12-20,13-22, 13-21, 13-20, 14-19, 15-18, or 16-17 amino acids.

In some embodiments, the peptide epitopes comprise at least one MHC class I epitope and at least one MHC class II epitope. In some embodiments, at least 10% of the epitopes are MHC class I epitopes. In some embodiments, at least 20% of the epitopes are MHC class I epitopes. In some embodiments, at least 30% of the epitopes are MHC class I epitopes. In some embodiments, at least 40% of the epitopes are MHC class I epitopes. In some embodiments, at least 50%, 60%, 70%, 80%, 90% or 100% of the epitopes are MHC class I epitopes. In some embodiments, at least 10% of the epitopes are MHC class II epitopes. In some embodiments, at least 20% of the epitopes are MHC class II epitopes. In some embodiments, at least 30% of the epitopes are MHC class II epitopes. In some embodiments, at least 40% of the epitopes are MHC class II epitopes. In some embodiments, at least 50%, 60%, 70%, 80%, 90% or 100% of the epitopes are MHC class II epitopes. In some embodiments, the ratio of MHC class I epitopes to MHC class II epitopes is a ratio selected from about 10%:about 90%; about 20%:about 80%; about 30%:about 70%; about 40%:about 60%; about 50%:about 50%; about 60%:about 40%; about 70%:about 30%; about 80%: about 20%; about90%: about 10% MHC class 1: MHC class II epitopes. In some embodiments, the ratio of MHC class II epitopes to MHC class I epitopes is a ratio selected from about 10%:about 90%; about 20%:about 80%; about 30%:about 70%; about 40%: about 60%; about 50%: about 50%; about 60%:about 40%; about 70%:about 30%; about 80%: about 20%; about 90%: about 10% MHC class II: MHC class I epitopes. In some embodiments, at least one of the peptide epitopes of the cancer vaccine is a B cell epitope. In some embodiments, the T cell epitope of the cancer vaccine comprises between 8-11 amino acids. In some embodiments, the B cell epitope of the cancer vaccine comprises between 13-17 amino acids.

### A. mRNAs

Exemplary aspects of the disclosure feature efficacious mRNA vaccines. Described herein are mRNA vaccines designed to achieve particular biologic effects. Exemplary vaccines of the disclosure feature mRNAs encoding a particular antigen of interest (or and mRNA or mRNAs encoding antigens of interest), optionally formulated with additional components designed to facilitate efficacious delivery of mRNAs *in vivo.* In exemplary aspects, the vaccines of the disclosure feature and mRNA or mRNAs encoding antigen(s) of interest, complexed with polymeric or lipid components, or in certain aspects, encapsulated in liposomes, or alternatively, in lipid nanoparticles (LNPs). Chemical modification of mRNAs can facilitate certain desirable properties of vaccines on the disclosure, for example, influencing the type of immune response to the vaccine. For example, appropriate chemical modification of mRNAs can reduce unwanted innate immune responses against mRNA components and/or can facilitate desirable levels of protein expression of the antigen or antigens of interest. Further description of such features of the disclosure is provided *infra.*

### 1. Chemically-modified mRNAs

In some embodiments, the polynucleotide (e.g., a RNA, e.g., an mRNA) of the disclosure comprises a chemically modified nucleobase. The disclosure includes modified polynucleotides comprising a polynucleotide described herein (e.g., a polynucleotide comprising a nucleotide sequence encoding an antigen polypeptide). The modified polynucleotides can be chemically modified and/or structurally modified. When the polynucleotides of the present disclosure are chemically and/or structurally modified the polynucleotides can be referred to as "modified polynucleotides."

The present disclosure provides for modified nucleosides and nucleotides of a polynucleotide (e.g., RNA polynucleotides, such as mRNA polynucleotides) encoding an antigen polypeptide. As used herein, the term "nucleic acid" is used in its broadest sense and encompasses any compound and/or substance that includes a polymer of nucleotides, or derivatives or analogs thereof. These polymers are often referred to as "polynucleotides". Accordingly, as used herein the terms "nucleic acid" and "polynucleotide" are equivalent and are used interchangeably. Exemplary nucleic acids or polynucleotides of the disclosure include, but are not limited to, ribonucleic acids (RNAs), deoxyribonucleic acids (DNAs), DNA-RNA hybrids, RNAi-inducing agents, RNAi agents, siRNAs, shRNAs, mRNAs, modified mRNAs, miRNAs, antisense RNAs, ribozymes, catalytic DNA, RNAs that induce triple helix formation, threose nucleic acids (TNAs), glycol nucleic acids (GNAs), peptide nucleic acids (PNAs), locked nucleic acids (LNAs, including LNA having a β-D-ribo configuration, α-LNA having an α-L-ribo configuration (a diastereomer of LNA), 2'-amino-LNA having a 2'-amino functionalization, and 2'-amino-α-LNA having a 2'-amino functionalization) or hybrids thereof. As used herein, the term "nucleobase" (alternatively "nucleotide base" or "nitrogenous base") refers to a purine or pyrimidine heterocyclic compound found in nucleic acids, including any derivatives or analogs of the naturally occurring purines and pyrimidines that confer improved properties (e.g., binding affinity, nuclease resistance, chemical stability) to a nucleic acid or a portion or segment thereof. Adenine, cytosine, guanine, thymine, and uracil are the nucleobases predominately found in natural nucleic acids. Other natural, non-natural, and/or synthetic nucleobases, as known in the art and/or described herein, can be incorporated into nucleic acids. Nucleoside/Nucleotide: As used herein, the term "nucleoside" refers to a compound containing a sugar molecule (e.g., a ribose in RNA or a deoxyribose in DNA), or derivative or analog thereof, covalently linked to a nucleobase (e.g., a purine or pyrimidine), or a derivative or analog thereof (also referred to herein as "nucleobase"), but lacking an internucleoside linking group (e.g., a phosphate group). As used herein, the term "nucleotide" refers to a nucleoside covalently bonded to an internucleoside linking group (e.g., a phosphate group), or any derivative, analog, or modification thereof that confers improved chemical and/or functional properties (e.g., binding affinity, nuclease resistance, chemical stability) to a nucleic acid or a portion or segment thereof. Modified nucleotides can by synthesized by any useful method, such as, for example, chemically, enzymatically, or recombinantly, to include one or more modified or non-natural nucleosides. Polynucleotides can comprise a region or regions of linked nucleosides. Such regions can have variable backbone linkages. The linkages can be standard phosphodiester linkages, in which case the polynucleotides would comprise regions of nucleotides.

The modified polynucleotides disclosed herein can comprise various distinct modifications. In some embodiments, the modified polynucleotides contain one, two, or more (optionally different) nucleoside or nucleotide modifications. In some embodiments, a modified polynucleotide, introduced to a cell can exhibit one or more desirable properties, e.g., improved protein expression, reduced immunogenicity, or reduced degradation in the cell, as compared to an unmodified polynucleotide.

In some embodiments, a polynucleotide of the present disclosure (e.g., a polynucleotide comprising a nucleotide sequence encoding an antigen polypeptide) is structurally modified, i.e., comprises one or more nucleic acid structure modifications. As used herein, a "structural" modification is one in which two or more linked nucleosides are inserted, deleted, duplicated, inverted or randomized in a polynucleotide without significant chemical modification to the nucleotides themselves. Further, the term "nucleic acid structure" (used interchangeably with "polynucleotide structure") refers to the arrangement or organization of atoms, chemical constituents, elements, motifs, and/or sequence of linked nucleotides, or derivatives or analogs thereof, that comprise a nucleic acid (e.g., an mRNA). The term also refers to the two-dimensional or three-dimensional state of a nucleic acid. Accordingly, the term "RNA structure" refers to the arrangement or organization of atoms, chemical constituents, elements, motifs, and/or sequence of linked nucleotides, or derivatives or analogs thereof, comprising an RNA molecule (e.g., an mRNA) and/or refers to a two-dimensional and/or three dimensional state of an RNA molecule. Nucleic acid structure can be further demarcated into four organizational categories referred to herein as "molecular structure", "primary structure", "secondary structure", and "tertiary structure" based on increasing organizational complexity. Because chemical bonds will necessarily be broken and reformed to effect a structural modification, structural modifications are of a chemical nature and hence are chemical modifications. However, structural modifications will result in a different sequence of nucleotides. For example, the polynucleotide "ATCG" can be chemically modified to "AT-5meC-G". The same polynucleotide can be structurally modified from "ATCG" to "ATCCCG". Here, the dinucleotide "CC" has been inserted, resulting in a structural modification to the polynucleotide.

In some embodiments, the polynucleotides of the present disclosure are chemically modified. As used herein in reference to a polynucleotide, the terms "chemical modification" or, as appropriate, "chemically modified" refer to modification with respect to adenosine (A), guanosine (G), uridine (U), or cytidine (C) ribo- or deoxyribonucleosides in one or more of their position, pattern, percent or population. Generally, herein, these terms are not intended to refer to the ribonucleotide modifications in naturally occurring 5'-terminal mRNA cap moieties.

In some embodiments, the polynucleotides of the present disclosure can have a uniform chemical modification of all or any of the same nucleoside type or a population of modifications produced by mere downward titration of the same starting modification in all or any of the same nucleoside type, or a measured percent of a chemical modification of all any of the same nucleoside type but with random incorporation, such as where all uridines are replaced by a uridine analog, e.g., pseudouridine or 5-methoxyuridine. In another embodiment, the polynucleotides can have a uniform chemical modification of two, three, or four of the same nucleoside type throughout the entire polynucleotide (such as all uridines and all cytosines, etc. are modified in the same way).

Modified nucleotide base pairing encompasses not only the standard adenosine-thymine, adenosine-uracil, or guanosine-cytosine base pairs, but also base pairs formed between nucleotides and/or modified nucleotides comprising non-standard or modified bases, wherein the arrangement of hydrogen bond donors and hydrogen bond acceptors permits hydrogen bonding between a non-standard base and a standard base or between two complementary non-standard base structures. One example of such non-standard base pairing is the base pairing between the modified nucleotide inosine and adenine, cytosine or uracil. Any combination of base/sugar or linker can be incorporated into polynucleotides of the present disclosure.
The skilled artisan will appreciate that, except where otherwise noted, polynucleotide sequences set forth in the instant application will recite "T"s in a representative DNA sequence but where the sequence represents RNA, the "T"s would be substituted for "U"s.

Modifications of polynucleotides (e.g., RNA polynucleotides, such as mRNA polynucleotides) that are useful in the compositions, methods and synthetic processes of the present disclosure include, but are not limited to the following nucleotides, nucleosides, and nucleobases: 2-methylthio-N6-(cis-hydroxyisopentenyl)adenosine; 2-methylthio-N6-methyladenosine; 2-methylthio-N6-threonyl carbamoyladenosine; N6-glycinylcarbamoyladenosine; N6-isopentenyladenosine; N6-methyladenosine; N6-threonylcarbamoyladenosine; 1,2'-O-dimethyladenosine; 1-methyladenosine; 2'-O-methyladenosine; 2'-O-ribosyladenosine (phosphate); 2-methyladenosine; 2-methylthio-N6 isopentenyladenosine; 2-methylthio-N6-hydroxynorvalyl carbamoyladenosine; 2'-O-methyladenosine; 2'-O-ribosyladenosine (phosphate); Isopentenyladenosine; N6-(cis-hydroxyisopentenyl)adenosine; N6,2'-O-dimethyladenosine; N6,2'-O-dimethyladenosine; N6,N6,2'-O-trimethyladenosine; N6,N6-dimethyladenosine; N6-acetyladenosine; N6-hydroxynorvalylcarbamoyladenosine; N6-methyl-N6-threonylcarbamoyladenosine; 2-methyladenosine; 2-methylthio-N6-isopentenyladenosine; 7-deaza-adenosine; N1-methyl-adenosine; N6, N6 (dimethyl)adenine; N6-cis-hydroxy-isopentenyladenosine; α-thio-adenosine; 2 (amino)adenine; 2 (aminopropyl)adenine; 2 (methylthio) N6 (isopentenyl)adenine; 2-(alkyl)adenine; 2-(aminoalkyl)adenine; 2-(aminopropyl)adenine; 2-(halo)adenine; 2-(halo)adenine; 2-(propyl)adenine; 2'-Amino-2'-deoxy-ATP; 2'-Azido-2'-deoxy-ATP; 2'-Deoxy-2'-a-aminoadenosine TP; 2'-Deoxy-2'-a-azidoadenosine TP; 6 (alkyl)adenine; 6 (methyl)adenine; 6-(alkyl)adenine; 6-(methyl)adenine; 7 (deaza)adenine; 8 (alkenyl)adenine; 8 (alkynyl)adenine; 8 (amino)adenine; 8 (thioalkyl)adenine; 8-(alkenyl)adenine; 8-(alkyl)adenine; 8-(alkynyl)adenine; 8-(amino)adenine; 8-(halo)adenine; 8-(hydroxyl)adenine; 8-(thioalkyl)adenine; 8-(thiol)adenine; 8-azido-adenosine; aza adenine; deaza adenine; N6 (methyl)adenine; N6-(isopentyl)adenine; 7-deaza-8-aza-adenosine; 7-methyladenine; 1-Deazaadenosine TP; 2'Fluoro-N6-Bz-deoxyadenosine TP; 2'-OMe-2-Amino-ATP; 2'O-methyl-N6-Bz-deoxyadenosine TP; 2'-a-Ethynyladenosine TP; 2-aminoadenine; 2-Aminoadenosine TP; 2-Amino-ATP; 2'-a-Trifluoromethyladenosine TP; 2-Azidoadenosine TP; 2'-b-Ethynyladenosine TP; 2-Bromoadenosine TP; 2'-b-Trifluoromethyladenosine TP; 2-Chloroadenosine TP; 2'-Deoxy-2',2'-difluoroadenosine TP; 2'-Deoxy-2'-a-mercaptoadenosine TP; 2'-Deoxy-2'-a-thiomethoxyadenosine TP; 2'-Deoxy-2'-b-aminoadenosine TP; 2'-Deoxy-2'-b-azidoadenosine TP; 2'-Deoxy-2'-b-bromoadenosine TP; 2'-Deoxy-2'-b-chloroadenosine TP; 2'-Deoxy-2'-b-fluoroadenosine TP; 2'-Deoxy-2'-b-iodoadenosine TP; 2'-Deoxy-2'-b-mercaptoadenosine TP; 2'-Deoxy-2'-b-thiomethoxyadenosine TP; 2-Fluoroadenosine TP; 2-iodoadenosine TP; 2-Mercaptoadenosine TP; 2-methoxy-adenine; 2-methylthio-adenine; 2-Trifluoromethyladenosine TP; 3-Deaza-3-bromoadenosine TP; 3-Deaza-3-chloroadenosine TP; 3-Deaza-3-fluoroadenosine TP; 3-Deaza-3-iodoadenosine TP; 3-Deazaadenosine TP; 4'-Azidoadenosine TP; 4'-Carbocyclic adenosine TP; 4'-Ethynyladenosine TP; 5'-Homo-adenosine TP; 8-Aza-ATP; 8-bromo-adenosine TP; 8-Trifluoromethyladenosine TP; 9-Deazaadenosine TP; 2-aminopurine; 7-deaza-2,6-diaminopurine; 7-deaza-8-aza-2,6-diaminopurine; 7-deaza-8-aza-2-aminopurine; 2,6-diaminopurine; 7-deaza-8-aza-adenine, 7-deaza-2-aminopurine; 2-thiocytidine; 3-methylcytidine; 5-formylcytidine; 5-hydroxymethylcytidine; 5-methylcytidine; N4-acetylcytidine; 2'-O-methylcytidine; 2'-O-methylcytidine; 5,2'-O-dimethylcytidine; 5-formyl-2'-O-methylcytidine; Lysidine; N4,2'-O-dimethylcytidine; N4-acetyl-2'-O-methylcytidine; N4-methylcytidine; N4,N4-Dimethyl-2'-OMe-Cytidine TP; 4-methylcytidine; 5-aza-cytidine; Pseudo-iso-cytidine; pyrrolo-cytidine; α-thio-cytidine; 2-(thio)cytosine; 2'-Amino-2'-deoxy-CTP; 2'-Azido-2'-deoxy-CTP; 2'-Deoxy-2'-a-aminocytidine TP; 2'-Deoxy-2'-a-azidocytidine TP; 3 (deaza) 5 (aza)cytosine; 3 (methyl)cytosine; 3-(alkyl)cytosine; 3-(deaza) 5 (aza)cytosine; 3-(methyl)cytidine; 4,2'-O-dimethylcytidine; 5 (halo)cytosine; 5 (methyl)cytosine; 5 (propynyl)cytosine; 5 (trifluoromethyl)cytosine; 5-(alkyl)cytosine; 5-(alkynyl)cytosine; 5-(halo)cytosine; 5-(propynyl)cytosine; 5-(trifluoromethyl)cytosine; 5-bromo-cytidine; 5-iodo-cytidine; 5-propynyl cytosine; 6-(azo)cytosine; 6-aza-cytidine; aza cytosine; deaza cytosine; N4 (acetyl)cytosine; 1-methyl-1-deaza-pseudoisocytidine; 1-methyl-pseudoisocytidine; 2-methoxy-5-methyl-cytidine; 2-methoxy-cytidine; 2-thio-5-methyl-cytidine; 4-methoxy-1-methyl-pseudoisocytidine; 4-methoxy-pseudoisocytidine; 4-thio-1-methyl-1-deaza-pseudoisocytidine; 4-thio-1-methyl-pseudoisocytidine; 4-thio-pseudoisocytidine; 5-aza-zebularine; 5-methyl-zebularine; pyrrolo-pseudoisocytidine; Zebularine; (E)-5-(2-Bromo-vinyl)cytidine TP; 2,2'-anhydro-cytidine TP hydrochloride; 2'Fluor-N4-Bz-cytidine TP; 2'Fluoro-N4-Acetyl-cytidine TP; 2'-O-Methyl-N4-Acetyl-cytidine TP; 2'O-methyl-N4-Bz-cytidine TP; 2'-a-Ethynylcytidine TP; 2'-a-Trifluoromethylcytidine TP; 2'-b-Ethynylcytidine TP; 2'-b-Trifluoromethylcytidine TP; 2'-Deoxy-2',2'-difluorocytidine TP; 2'-Deoxy-2'-a-mercaptocytidine TP; 2'-Deoxy-2'-a-thiomethoxycytidine TP; 2'-Deoxy-2'-b-aminocytidine TP; 2'-Deoxy-2'-b-azidocytidine TP; 2'-Deoxy-2'-b-bromocytidine TP; 2'-Deoxy-2'-b-chlorocytidine TP; 2'-Deoxy-2'-b-fluorocytidine TP; 2'-Deoxy-2'-b-iodocytidine TP; 2'-Deoxy-2'-b-mercaptocytidine TP; 2'-Deoxy-2'-b-thiomethoxycytidine TP; 2'-O-Methyl-5-(1-propynyl)cytidine TP; 3'-Ethynylcytidine TP; 4'-Azidocytidine TP; 4'-Carbocyclic cytidine TP; 4'-Ethynylcytidine TP; 5-(1-Propynyl)ara-cytidine TP; 5-(2-Chloro-phenyl)-2-thiocytidine TP; 5-(4-Amino-phenyl)-2-thiocytidine TP; 5-Aminoallyl-CTP; 5-Cyanocytidine TP; 5-Ethynylara-cytidine TP; 5-Ethynylcytidine TP; 5'-Homo-cytidine TP; 5-Methoxycytidine TP; 5-Trifluoromethyl-Cytidine TP; N4-Amino-cytidine TP; N4-Benzoyl-cytidine TP; Pseudoisocytidine; 7-methylguanosine; N2,2'-O-dimethylguanosine; N2-methylguanosine; Wyosine; 1,2'-O-dimethylguanosine; 1-methylguanosine; 2'-O-methylguanosine; 2'-O-ribosylguanosine (phosphate); 2'-O-methylguanosine; 2'-O-ribosylguanosine (phosphate); 7-aminomethyl-7-deazaguanosine; 7-cyano-7-deazaguanosine; Archaeosine; Methylwyosine; N2,7-dimethylguanosine; N2,N2,2'-O-trimethylguanosine; N2, N2,7-trimethylguanosine; N2,N2-dimethylguanosine; N2,7,2'-O-trimethylguanosine; 6-thio-guanosine; 7-deaza-guanosine; 8-oxo-guanosine; N1-methyl-guanosine; α-thio-guanosine; 2 (propyl)guanine; 2-(alkyl)guanine; 2'-Amino-2'-deoxy-GTP; 2'-Azido-2'-deoxy-GTP; 2'-Deoxy-2'-a-aminoguanosine TP; 2'-Deoxy-2'-a-azidoguanosine TP; 6 (methyl)guanine; 6-(alkyl)guanine; 6-(methyl)guanine; 6-methyl-guanosine; 7 (alkyl)guanine; 7 (deaza)guanine; 7 (methyl)guanine; 7-(alkyl)guanine; 7-(deaza)guanine; 7-(methyl)guanine; 8 (alkyl)guanine; 8 (alkynyl)guanine; 8 (halo)guanine; 8 (thioalkyl)guanine; 8-(alkenyl)guanine; 8-(alkyl)guanine; 8-(alkynyl)guanine; 8-(amino)guanine; 8-(halo)guanine; 8-(hydroxyl)guanine; 8-(thioalkyl)guanine; 8-(thiol)guanine; aza guanine; deaza guanine; N (methyl)guanine; N-(methyl)guanine; 1-methyl-6-thio-guanosine; 6-methoxy-guanosine; 6-thio-7-deaza-8-aza-guanosine; 6-thio-7-deaza-guanosine; 6-thio-7-methyl-guanosine; 7-deaza-8-aza-guanosine; 7-methyl-8-oxo-guanosine; N2,N2-dimethyl-6-thio-guanosine; N2-methyl-6-thio-guanosine; 1-Me-GTP; 2'Fluoro-N2-isobutyl-guanosine TP; 2'O-methyl-N2-isobutyl-guanosine TP; 2'-a-Ethynylguanosine TP; 2'-a-Trifluoromethylguanosine TP; 2'-b-Ethynylguanosine TP; 2'-b-Trifluoromethylguanosine TP; 2'-Deoxy-2',2'-difluoroguanosine TP; 2'-Deoxy-2'-a-mercaptoguanosine TP; 2'-Deoxy-2'-a-thiomethoxyguanosine TP; 2'-Deoxy-2'-b-aminoguanosine TP; 2'-Deoxy-2'-b-azidoguanosine TP; 2'-Deoxy-2'-b-bromoguanosine TP; 2'-Deoxy-2'-b-chloroguanosine TP; 2'-Deoxy-2'-b-fluoroguanosine TP; 2'-Deoxy-2'-b-iodoguanosine TP; 2'-Deoxy-2'-b-mercaptoguanosine TP; 2'-Deoxy-2'-b-thiomethoxyguanosine TP; 4'-Azidoguanosine TP; 4'-Carbocyclic guanosine TP; 4'-Ethynylguanosine TP; 5'-Homo-guanosine TP; 8-bromo-guanosine TP; 9-Deazaguanosine TP; N2-isobutyl-guanosine TP; 1-methylinosine; Inosine; 1,2'-O-dimethylinosine; 2'-O-methylinosine; 7-methylinosine; 2'-O-methylinosine; Epoxyqueuosine; galactosyl-queuosine; Mannosylqueuosine; Queuosine; allyamino-thymidine; aza thymidine; deaza thymidine; deoxy-thymidine; 2'-O-methyluridine; 2-thiouridine; 3-methyluridine; 5-carboxymethyluridine; 5-hydroxyuridine; 5-methyluridine; 5-taurinomethyl-2-thiouridine; 5-taurinomethyluridine; Dihydrouridine; Pseudouridine; (3-(3-amino-3-carboxypropyl)uridine; 1-methyl-3-(3-amino-5-carboxypropyl)pseudouridine; 1-methylpseduouridine; 1-ethyl-pseudouridine; 2'-O-methyluridine; 2'-O-methylpseudouridine; 2'-O-methyluridine; 2-thio-2'-O-methyluridine; 3-(3-amino-3-carboxypropyl)uridine; 3,2'-O-dimethyluridine; 3-Methyl-pseudo-Uridine TP; 4-thiouridine; 5-(carboxyhydroxymethyl)uridine; 5-(carboxyhydroxymethyl)uridine methyl ester; 5,2'-O-dimethyluridine; 5,6-dihydro-uridine; 5-aminomethyl-2-thiouridine; 5-carbamoylmethyl-2'-O-methyluridine; 5-carbamoylmethyluridine; 5-carboxyhydroxymethyluridine; 5-carboxyhydroxymethyluridine methyl ester; 5-carboxymethylaminomethyl-2'-O-methyluridine; 5-carboxymethylaminomethyl-2-thiouridine; 5-carboxymethylaminomethyl-2-thiouridine; 5-carboxymethylaminomethyluridine; 5-carboxymethylaminomethyluridine; 5-Carbamoylmethyluridine TP; 5-methoxycarbonylmethyl-2'-O-methyluridine; 5-methoxycarbonylmethyl-2-thiouridine; 5-methoxycarbonylmethyluridine; 5-methyluridine,), 5-methoxyuridine; 5-methyl-2-thiouridine; 5-methylaminomethyl-2-selenouridine; 5-methylaminomethyl-2-thiouridine; 5-methylaminomethyluridine; 5-Methyldihydrouridine; 5-Oxyacetic acid- Uridine TP; 5-Oxyacetic acid-methyl ester-Uridine TP; N1-methyl-pseudo-uracil; N1-ethyl-pseudo-uracil; uridine 5-oxyacetic acid; uridine 5-oxyacetic acid methyl ester; 3-(3-Amino-3-carboxypropyl)-Uridine TP; 5-(iso-Pentenylaminomethyl)- 2-thiouridine TP; 5-(iso-Pentenylaminomethyl)-2'-O-methyluridine TP; 5-(iso-Pentenylaminomethyl)uridine TP; 5-propynyl uracil; α-thio-uridine; 1 (aminoalkylamino-carbonylethylenyl)-2(thio)-pseudouracil; 1 (aminoalkylaminocarbonylethylenyl)-2,4-(dithio)pseudouracil; 1 (aminoalkylaminocarbonylethylenyl)-4 (thio)pseudouracil; 1 (aminoalkylaminocarbonylethylenyl)-pseudouracil; 1 (aminocarbonylethylenyl)-2(thio)-pseudouracil; 1 (aminocarbonylethylenyl)-2,4-(dithio)pseudouracil; 1 (aminocarbonylethylenyl)-4 (thio)pseudouracil; 1 (aminocarbonylethylenyl)-pseudouracil; 1 substituted 2(thio)-pseudouracil; 1 substituted 2,4-(dithio)pseudouracil; 1 substituted 4 (thio)pseudouracil; 1 substituted pseudouracil; 1-(aminoalkylamino-carbonylethylenyl)-2-(thio)-pseudouracil; 1-Methyl-3-(3-amino-3-carboxypropyl) pseudouridine TP; 1-Methyl-3-(3-amino-3-carboxypropyl)pseudo-UTP; 1-Methyl-pseudo-UTP; 1-Ethyl-pseudo-UTP; 2 (thio)pseudouracil; 2' deoxy uridine; 2' fluorouridine; 2-(thio)uracil; 2,4-(dithio)psuedouracil; 2' methyl, 2'amino, 2'azido, 2'fluro-guanosine; 2'-Amino-2'-deoxy-UTP; 2'-Azido-2'-deoxy-UTP; 2'-Azido-deoxyuridine TP; 2'-O-methylpseudouridine; 2' deoxy uridine; 2' fluorouridine; 2'-Deoxy-2'-a-aminouridine TP; 2'-Deoxy-2'-a-azidouridine TP; 2-methylpseudouridine; 3 (3 amino-3 carboxypropyl)uracil; 4 (thio)pseudouracil; 4-(thio )pseudouracil; 4-(thio)uracil; 4-thiouracil; 5 (1,3-diazole-1-alkyl)uracil; 5 (2-aminopropyl)uracil; 5 (aminoalkyl)uracil; 5 (dimethylaminoalkyl)uracil; 5 (guanidiniumalkyl)uracil; 5 (methoxycarbonylmethyl)-2-(thio)uracil; 5 (methoxycarbonylmethyl)uracil; 5 (methyl) 2 (thio)uracil; 5 (methyl) 2,4 (dithio)uracil; 5 (methyl) 4 (thio)uracil; 5 (methylaminomethyl)-2 (thio)uracil; 5 (methylaminomethyl)-2,4 (dithio)uracil; 5 (methylaminomethyl)-4 (thio)uracil; 5 (propynyl)uracil; 5 (trifluoromethyl)uracil; 5-(2-aminopropyl)uracil; 5-(alkyl)-2-(thio)pseudouracil; 5-(alkyl)-2,4 (dithio)pseudouracil; 5-(alkyl)-4 (thio)pseudouracil; 5-(alkyl)pseudouracil; 5-(alkyl)uracil; 5-(alkynyl)uracil; 5-(allylamino)uracil; 5-(cyanoalkyl)uracil; 5-(dialkylaminoalkyl)uracil; 5-(dimethylaminoalkyl)uracil; 5-(guanidiniumalkyl)uracil; 5-(halo)uracil; 5-(l,3-diazole-l-alkyl)uracil; 5-(methoxy)uracil; 5-(methoxycarbonylmethyl)-2-(thio)uracil; 5-(methoxycarbonyl-methyl)uracil; 5-(methyl) 2(thio)uracil; 5-(methyl) 2,4 (dithio )uracil; 5-(methyl) 4 (thio)uracil; 5-(methyl)-2-(thio)pseudouracil; 5-(methyl)-2,4 (dithio)pseudouracil; 5-(methyl)-4 (thio)pseudouracil; 5-(methyl)pseudouracil; 5-(methylaminomethyl)-2 (thio)uracil; 5-(methylaminomethyl)-2,4(dithio )uracil; 5-(methylaminomethyl)-4-(thio)uracil; 5-(propynyl)uracil; 5-(trifluoromethyl)uracil; 5-aminoallyl-uridine; 5-bromo-uridine; 5-iodo-uridine; 5-uracil; 6 (azo)uracil; 6-(azo)uracil; 6-aza-uridine; allyamino-uracil; aza uracil; deaza uracil; N3 (methyl)uracil; P seudo-UTP-1-2-ethanoic acid; Pseudouracil; 4-Thio-pseudo-UTP; 1-carboxymethyl-pseudouridine; 1-methyl-1-deaza-pseudouridine; 1-propynyl-uridine; 1-taurinomethyl-1-methyl-uridine; 1-taurinomethyl-4-thio-uridine; 1-taurinomethyl-pseudouridine; 2-methoxy-4-thio-pseudouridine; 2-thio-1-methyl-1-deaza-pseudouridine; 2-thio-1-methyl-pseudouridine; 2-thio-5-aza-uridine; 2-thio-dihydropseudouridine; 2-thio-dihydrouridine; 2-thio-pseudouridine; 4-methoxy-2-thio-pseudouridine; 4-methoxy-pseudouridine; 4-thio-1-methyl-pseudouridine; 4-thio-pseudouridine; 5-aza-uridine; Dihydropseudouridine; (±)1-(2-Hydroxypropyl)pseudouridine TP; (2R)-1-(2-Hydroxypropyl)pseudouridine TP; (2S)-1-(2-Hydroxypropyl)pseudouridine TP; (E)-5-(2-Bromo-vinyl)ara-uridine TP; (E)-5-(2-Bromo-vinyl)uridine TP; (Z)-5-(2-Bromo-vinyl)ara-uridine TP; (Z)-5-(2-Bromo-vinyl)uridine TP; 1-(2,2,2-Trifluoroethyl)-pseudo-UTP; 1-(2,2,3,3,3-Pentafluoropropyl)pseudouridine TP; 1-(2,2-Diethoxyethyl)pseudouridine TP; 1-(2,4,6-Trimethylbenzyl)pseudouridine TP; 1-(2,4,6-Trimethyl-benzyl)pseudo-UTP; 1-(2,4,6-Trimethyl-phenyl)pseudo-UTP; 1-(2-Amino-2-carboxyethyl)pseudo-UTP; 1-(2-Amino-ethyl)pseudo-UTP; 1-(2-Hydroxyethyl)pseudouridine TP; 1-(2-Methoxyethyl)pseudouridine TP; 1-(3,4-Bis-trifluoromethoxybenzyl)pseudouridine TP; 1-(3,4-Dimethoxybenzyl)pseudouridine TP; 1-(3-Amino-3-carboxypropyl)pseudo-UTP; 1-(3-Amino-propyl)pseudo-UTP; 1-(3-Cyclopropyl-prop-2-ynyl)pseudouridine TP; 1-(4-Amino-4-carboxybutyl)pseudo-UTP; 1-(4-Amino-benzyl)pseudo-UTP; 1-(4-Amino-butyl)pseudo-UTP; 1-(4-Amino-phenyl)pseudo-UTP; 1-(4-Azidobenzyl)pseudouridine TP; 1-(4-Bromobenzyl)pseudouridine TP; 1-(4-Chlorobenzyl)pseudouridine TP; 1-(4-Fluorobenzyl)pseudouridine TP; 1-(4-lodobenzyl)pseudouridine TP; 1-(4-Methanesulfonylbenzyl)pseudouridine TP; 1-(4-Methoxybenzyl)pseudouridine TP; 1-(4-Methoxy-benzyl)pseudo-UTP; 1-(4-Methoxy-phenyl)pseudo-UTP; 1-(4-Methylbenzyl)pseudouridine TP; 1-(4-Methyl-benzyl)pseudo-UTP; 1-(4-Nitrobenzyl)pseudouridine TP; 1-(4-Nitrobenzyl)pseudo-UTP; 1(4-Nitro-phenyl)pseudo-UTP; 1-(4-Thiomethoxybenzyl)pseudouridine TP; 1-(4-Trifluoromethoxybenzyl)pseudouridine TP; 1-(4-Trifluoromethylbenzyl)pseudouridine TP; 1-(5-Amino-pentyl)pseudo-UTP; 1-(6-Amino-hexyl)pseudo-UTP; 1,6-Dimethyl-pseudo-UTP; 1-[3-(2-{2-[2-(2-Aminoethoxy)-ethoxy]-ethoxy}-ethoxy)-propionyl]pseudouridine TP; 1-{3-[2-(2-Aminoethoxy)-ethoxy]-propionyl } pseudouridine TP; 1-Acetylpseudouridine TP; 1-Alkyl-6-(1-propynyl)-pseudo-UTP; 1-Alkyl-6-(2-propynyl)-pseudo-UTP; 1-Alkyl-6-allyl-pseudo-UTP; 1-Alkyl-6-ethynyl-pseudo-UTP; 1-Alkyl-6-homoallyl-pseudo-UTP; 1-Alkyl-6-vinyl-pseudo-UTP; 1-Allylpseudouridine TP; 1-Aminomethyl-pseudo-UTP; 1-Benzoylpseudouridine TP; 1-Benzyloxymethylpseudouridine TP; 1-Benzyl-pseudo-UTP; 1-Biotinyl-PEG2-pseudouridine TP; 1-Biotinylpseudouridine TP; 1-Butyl-pseudo-UTP; 1-Cyanomethylpseudouridine TP; 1-Cyclobutylmethyl-pseudo-UTP; 1-Cyclobutyl-pseudo-UTP; 1-Cycloheptylmethyl-pseudo-UTP; 1-Cycloheptyl-pseudo-UTP; 1-Cyclohexylmethyl-pseudo-UTP; 1-Cyclohexyl-pseudo-UTP; 1-Cyclooctylmethyl-pseudo-UTP; 1-Cyclooctyl-pseudo-UTP; 1-Cyclopentylmethyl-pseudo-UTP; 1-Cyclopentyl-pseudo-UTP; 1-Cyclopropylmethyl-pseudo-UTP; 1-Cyclopropyl-pseudo-UTP; 1-Ethyl-pseudo-UTP; 1-Hexyl-pseudo-UTP; 1-Homoallylpseudouridine TP; 1-Hydroxymethylpseudouridine TP; 1-iso-propyl-pseudo-UTP; 1-Me-2-thio-pseudo-UTP; 1-Me-4-thio-pseudo-UTP; 1-Me-alpha-thio-pseudo-UTP; 1-Methanesulfonylmethylpseudouridine TP; 1-Methoxymethylpseudouridine TP; 1-Methyl-6-(2,2,2-Trifluoroethyl)pseudo-UTP; 1-Methyl-6-(4-morpholino)-pseudo-UTP; 1-Methyl-6-(4-thiomorpholino)-pseudo-UTP; 1-Methyl-6-(substituted phenyl)pseudo-UTP; 1-Methyl-6-amino-pseudo-UTP; 1-Methyl-6-azido-pseudo-UTP; 1-Methyl-6-bromo-pseudo-UTP; 1-Methyl-6-butyl-pseudo-UTP; 1-Methyl-6-chloro-pseudo-UTP; 1-Methyl-6-cyano-pseudo-UTP; 1-Methyl-6-dimethylamino-pseudo-UTP; 1-Methyl-6-ethoxy-pseudo-UTP; 1-Methyl-6-ethylcarboxylate-pseudo-UTP; 1-Methyl-6-ethyl-pseudo-UTP; 1-Methyl-6-fluoro-pseudo-UTP; 1-Methyl-6-formyl-pseudo-UTP; 1-Methyl-6-hydroxyamino-pseudo-UTP; 1-Methyl-6-hydroxy-pseudo-UTP; 1-Methyl-6-iodo-pseudo-UTP; 1-Methyl-6-iso-propyl-pseudo-UTP; 1-Methyl-6-methoxy-pseudo-UTP; 1-Methyl-6-methylamino-pseudo-UTP; 1-Methyl-6-phenyl-pseudo-UTP; 1-Methyl-6-propyl-pseudo-UTP; 1-Methyl-6-tert-butyl-pseudo-UTP; 1-Methyl-6-trifluoromethoxy-pseudo-UTP; 1-Methyl-6-trifluoromethyl-pseudo-UTP; 1-Morpholinomethylpseudouridine TP; 1-Pentyl-pseudo-UTP; 1-Phenyl-pseudo-UTP; 1-Pivaloylpseudouridine TP; 1-Propargylpseudouridine TP; 1-Propyl-pseudo-UTP; 1-propynyl-pseudouridine; 1-p-tolyl-pseudo-UTP; 1-tert-Butyl-pseudo-UTP; 1-Thiomethoxymethylpseudouridine TP; 1-Thiomorpholinomethylpseudouridine TP; 1-Trifluoroacetylpseudouridine TP; 1-Trifluoromethyl-pseudo-UTP; 1-Vinylpseudouridine TP; 2,2'-anhydro-uridine TP; 2'-bromo-deoxyuridine TP; 2'-F-5-Methyl-2'-deoxy-UTP; 2'-OMe-5-Me-UTP; 2'-OMe-pseudo-UTP; 2'-a-Ethynyluridine TP; 2'-a-Trifluoromethyluridine TP; 2'-b-Ethynyluridine TP; 2'-b-Trifluoromethyluridine TP; 2'-Deoxy-2',2'-difluorouridine TP; 2'-Deoxy-2'-a-mercaptouridine TP; 2'-Deoxy-2'-a-thiomethoxyuridine TP; 2'-Deoxy-2'-b-aminouridine TP; 2'-Deoxy-2'-b-azidouridine TP; 2'-Deoxy-2'-b-bromouridine TP; 2'-Deoxy-2'-b-chlorouridine TP; 2'-Deoxy-2'-b-fluorouridine TP; 2'-Deoxy-2'-b-iodouridine TP; 2'-Deoxy-2'-b-mercaptouridine TP; 2'-Deoxy-2'-b-thiomethoxyuridine TP; 2-methoxy-4-thio-uridine; 2-methoxyuridine; 2'-O-Methyl-5-(1-propynyl)uridine TP; 3-Alkyl-pseudo-UTP; 4'-Azidouridine TP; 4'-Carbocyclic uridine TP; 4'-Ethynyluridine TP; 5-(1-Propynyl)ara-uridine TP; 5-(2-Furanyl)uridine TP; 5-Cyanouridine TP; 5-Dimethylaminouridine TP; 5'-Homo-uridine TP; 5-iodo-2'-fluoro-deoxyuridine TP; 5-Phenylethynyluridine TP; 5-Trideuteromethyl-6-deuterouridine TP; 5-TrifluoromethylUridine TP; 5-Vinylarauridine TP; 6-(2,2,2-Trifluoroethyl)-pseudo-UTP; 6-(4-Morpholino)-pseudo-UTP; 6-(4-Thiomorpholino)-pseudo-UTP; 6-(Substituted-Phenyl)-pseudo-UTP; 6-Amino-pseudo-UTP; 6-Azido-pseudo-UTP; 6-Bromo-pseudo-UTP; 6-Butyl-pseudo-UTP; 6-Chloro-pseudo-UTP; 6-Cyano-pseudo-UTP; 6-Dimethylamino-pseudo-UTP; 6-Ethoxy-pseudo-UTP; 6-Ethylcarboxylate-pseudo-UTP; 6-Ethyl-pseudo-UTP; 6-Fluoro-pseudo-UTP; 6-Formyl-pseudo-UTP; 6-Hydroxyamino-pseudo-UTP; 6-Hydroxy-pseudo-UTP; 6-lodo-pseudo-UTP; 6-iso-Propyl-pseudo-UTP; 6-Methoxy-pseudo-UTP; 6-Methylamino-pseudo-UTP; 6-Methyl-pseudo-UTP; 6-Phenyl-pseudo-UTP; 6-Phenyl-pseudo-UTP; 6-Propyl-pseudo-UTP; 6-tert-Butyl-pseudo-UTP; 6-Trifluoromethoxy-pseudo-UTP; 6-Trifluoromethyl-pseudo-UTP; Alpha-thio-pseudo-UTP; Pseudouridine 1-(4-methylbenzenesulfonic acid) TP; Pseudouridine 1-(4-methylbenzoic acid) TP; Pseudouridine TP 1-[3-(2-ethoxy)]propionic acid; Pseudouridine TP 1-[3-{2-(2-[2-(2-ethoxy )-ethoxy]-ethoxy )-ethoxy}]propionic acid; Pseudouridine TP 1-[3-{2-(2-[2-{2(2-ethoxy )-ethoxy}-ethoxy]-ethoxy )-ethoxy}]propionic acid; Pseudouridine TP 1-[3-{2-(2-[2-ethoxy ]-ethoxy)-ethoxy}]propionic acid; Pseudouridine TP 1-[3-{2-(2-ethoxy)-ethoxy}] propionic acid; Pseudouridine TP 1-methylphosphonic acid; Pseudouridine TP 1-methylphosphonic acid diethyl ester; Pseudo-UTP-N1-3-propionic acid; Pseudo-UTP-N1-4-butanoic acid; Pseudo-UTP-N1-5-pentanoic acid; Pseudo-UTP-N1-6-hexanoic acid; Pseudo-UTP-N1-7-heptanoic acid; Pseudo-UTP-N1-methyl-p-benzoic acid; Pseudo-UTP-N1-p-benzoic acid; Wybutosine; Hydroxywybutosine; Isowyosine; Peroxywybutosine; undermodified hydroxywybutosine; 4-demethylwyosine; 2,6-(diamino)purine;1-(aza)-2-(thio)-3-(aza)-phenoxazin-1-yl: 1,3-( diaza)-2-( oxo )-phenthiazin-l-yl;1,3-(diaza)-2-(oxo)-phenoxazin-1-yl;1,3,5-(triaza)-2,6-(dioxa)-naphthalene;2 (amino)purine;2,4,5-(trimethyl)phenyl;2' methyl, 2'amino, 2'azido, 2'fluro-cytidine;2' methyl, 2'amino, 2'azido, 2'fluro-adenine;2'methyl, 2'amino, 2'azido, 2'fluro-uridine;2'-amino-2'-deoxyribose; 2-amino-6-Chloro-purine; 2-aza-inosinyl; 2'-azido-2'-deoxyribose; 2'fluoro-2'-deoxyribose; 2'-fluoro-modified bases; 2'-O-methyl-ribose; 2-oxo-7-aminopyridopyrimidin-3-yl; 2-oxo-pyridopyrimidine-3-yl; 2-pyridinone; 3 nitropyrrole; 3-(methyl)-7-(propynyl)isocarbostyrilyl; 3-(methyl)isocarbostyrilyl; 4-(fluoro)-6-(methyl)benzimidazole; 4-(methyl)benzimidazole; 4-(methyl)indolyl; 4,6-(dimethyl)indolyl; 5 nitroindole; 5 substituted pyrimidines; 5-(methyl)isocarbostyrilyl; 5-nitroindole; 6-(aza)pyrimidine; 6-(azo)thymine; 6-(methyl)-7-(aza)indolyl; 6-chloro-purine; 6-phenyl-pyrrolo-pyrimidin-2-on-3-yl; 7-(aminoalkylhydroxy)-1-(aza)-2-(thio )-3-(aza)-phenthiazin-l-yl; 7-(aminoalkylhydroxy)-1-(aza)-2-(thio)-3-(aza)-phenoxazin-1-yl; 7-(aminoalkylhydroxy)-1,3-(diaza)-2-(oxo)-phenoxazin-1-yl; 7-(aminoalkylhydroxy)-1,3-(diaza)-2-(oxo)-phenthiazin-l-yl; 7-(aminoalkylhydroxy)-l,3-(diaza)-2-(oxo)-phenoxazin-l-yl; 7-(aza)indolyl; 7-(guanidiniumalkylhydroxy)-1-(aza)-2-(thio)-3-(aza)-phenoxazinl-yl; 7-(guanidiniumalkylhydroxy)-1-(aza)-2-(thio)-3-(aza)-phenthiazin-l-yl; 7-(guanidiniumalkylhydroxy)-1-(aza)-2-(thio)-3-(aza)-phenoxazin-1-yl; 7-(guanidiniumalkylhydroxy)-1,3-(diaza)-2-(oxo)-phenoxazin-1-yl; 7-(guanidiniumalkyl-hydroxy)-1,3-(diaza)-2-(oxo)-phenthiazin-l-yl; 7-(guanidiniumalkylhydroxy)-l,3-(diaza)-2-(oxo)-phenoxazin-l-yl; 7-(propynyl)isocarbostyrilyl; 7-(propynyl)isocarbostyrilyl, propynyl-7-(aza)indolyl; 7-deaza-inosinyl; 7-substituted 1-(aza)-2-(thio)-3-(aza)-phenoxazin-1-yl; 7-substituted 1,3-(diaza)-2-(oxo)-phenoxazin-1-yl; 9-(methyl)-imidizopyridinyl; Aminoindolyl; Anthracenyl; bis-ortho-(aminoalkylhydroxy)-6-phenyl-pyrrolo-pyrimidin-2-on-3-yl; bis-ortho-substituted-6-phenyl-pyrrolo-pyrimidin-2-on-3-yl; Difluorotolyl; Hypoxanthine; Imidizopyridinyl; Inosinyl; Isocarbostyrilyl; Isoguanisine; N2-substituted purines; N6-methyl-2-amino-purine; N6-substituted purines; N-alkylated derivative; Napthalenyl; Nitrobenzimidazolyl; Nitroimidazolyl; Nitroindazolyl; Nitropyrazolyl; Nubularine; O6-substituted purines; O-alkylated derivative; ortho-(aminoalkylhydroxy)-6-phenyl-pyrrolo-pyrimidin-2-on-3-yl; ortho-substituted-6-phenyl-pyrrolo-pyrimidin-2-on-3-yl; Oxoformycin TP; para-(aminoalkylhydroxy)-6-phenyl-pyrrolo-pyrimidin-2-on-3-yl; para-substituted-6-phenyl-pyrrolo-pyrimidin-2-on-3-yl; Pentacenyl; Phenanthracenyl; Phenyl; propynyl-7-(aza)indolyl; Pyrenyl; pyridopyrimidin-3-yl; pyridopyrimidin-3-yl, 2-oxo-7-amino-pyridopyrimidin-3-yl; pyrrolo-pyrimidin-2-on-3-yl; Pyrrolopyrimidinyl; Pyrrolopyrizinyl; Stilbenzyl; substituted 1,2,4-triazoles; Tetracenyl; Tubercidine; Xanthine; Xanthosine-5'-TP; 2-thio-zebularine; 5-aza-2-thio-zebularine; 7-deaza-2-amino-purine; pyridin-4-one ribonucleoside; 2-Amino-riboside-TP; Formycin A TP; Formycin B TP; Pyrrolosine TP; 2'-OH-ara-adenosine TP; 2'-OH-ara-cytidine TP; 2'-OH-ara-uridine TP; 2'-OH-ara-guanosine TP; 5-(2-carbomethoxyvinyl)uridine TP; and N6-(19-Amino-pentaoxanonadecyl)adenosine TP.

In some embodiments, the polynucleotide (e.g., RNA polynucleotide, such as mRNA polynucleotide) includes a combination of at least two (e.g., 2, 3, 4 or more) of the aforementioned modified nucleobases.

In some embodiments, the mRNA comprises at least one chemically modified nucleoside. In some embodiments, the at least one chemically modified nucleoside is selected from the group consisting of pseudouridine (ψ), 2-thiouridine (s2U), 4'-thiouridine, 5-methylcytosine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methyluridine, 5-methoxyuridine, 2'-O-methyl uridine, 1-methyl-pseudouridine (m1ψ), 1-ethyl-pseudouridine (e1ψ), 5-methoxy-uridine (mo5U), 5-methyl-cytidine (m5C), α-thio-guanosine, α-thio-adenosine, 5-cyano uridine, 4'-thio uridine 7-deaza-adenine, 1-methyl-adenosine (m1A), 2-methyl-adenine (m2A), N6-methyl-adenosine (m6A), and 2,6-Diaminopurine, (I), 1-methyl-inosine (m1I), wyosine (imG), methylwyosine (mimG), 7-deaza-guanosine, 7-cyano-7-deaza-guanosine (preQ0), 7-aminomethyl-7-deaza-guanosine (preQ1), 7-methyl-guanosine (m7G), 1-methyl-guanosine (m1G), 8-oxo-guanosine, 7-methyl-8-oxo-guanosine, 2,8-dimethyladenosine, 2-geranylthiouridine, 2-lysidine, 2-selenouridine, 3-(3-amino-3-carboxypropyl)-5,6-dihydrouridine, 3-(3-amino-3-carboxypropyl)pseudouridine, 3-methylpseudouridine, 5-(carboxyhydroxymethyl)-2'-O-methyluridine methyl ester, 5-aminomethyl-2-geranylthiouridine, 5-aminomethyl-2-selenouridine, 5-aminomethyluridine, 5-carbamoylhydroxymethyluridine, 5-carbamoylmethyl-2-thiouridine, 5-carboxymethyl-2-thiouridine, 5-carboxymethylaminomethyl-2-geranylthiouridine, 5-carboxymethylaminomethyl-2-selenouridine, 5-cyanomethyluridine, 5-hydroxycytidine, 5-methylaminomethyl-2-geranylthiouridine, 7-aminocarboxypropyl-demethylwyosine, 7-aminocarboxypropylwyosine, 7-aminocarboxypropylwyosine methyl ester, 8-methyladenosine, N4,N4-dimethylcytidine, N6-formyladenosine, N6-hydroxymethyladenosine, agmatidine, cyclic N6-threonylcarbamoyladenosine, glutamyl-queuosine, methylated undermodified hydroxywybutosine, N4,N4,2'-O-trimethylcytidine, geranylated 5-methylaminomethyl-2-thiouridine, geranylated 5-carboxymethylaminomethyl-2-thiouridine, Qbase , preQ0base, preQ1base, and two or more combinations thereof. In some embodiments, the at least one chemically modified nucleoside is selected from the group consisting of pseudouridine, 1-methyl-pseudouridine, 1-ethyl-pseudouridine, 5-methylcytosine, 5-methoxyuridine, and a combination thereof. In some embodiments, the polynucleotide (e.g., RNA polynucleotide, such as mRNA polynucleotide) includes a combination of at least two (e.g., 2, 3, 4 or more) of the aforementioned modified nucleobases.

### 2. mRNA Flanking Regions

In certain aspects, the present disclosure provides nucleic acid molecules, specifically polynucleotides that encode one or more antigens, or functional fragments thereof. Features, which can be considered beneficial in some embodiments of the present disclosure, can be encoded by regions of the polynucleotide and such regions can be upstream (5') or downstream (3') to, or within, a region that encodes a polypeptide. These regions can be incorporated into the polynucleotide before and/or after sequence optimization of the protein encoding region or open reading frame (ORF). It is not required that a polynucleotide contain both a 5' and 3' flanking region. Examples of such features include, but are not limited to, untranslated regions (UTRs), Kozak sequences, an oligo(dT) sequence, and detectable tags and can include multiple cloning sites that can have Xbal recognition.

In some embodiments, a 5' UTR and/or a 3' UTR region can be provided as flanking regions. Multiple 5' or 3' UTRs can be included in the flanking regions and can be the same or of different sequences. Any portion of the flanking regions, including none, can be sequence-optimized and any can independently contain one or more different structural or chemical modifications, before and/or after sequence optimization.

Untranslated regions (UTRs) are nucleic acid sections of a polynucleotide before a start codon (5'UTR) and after a stop codon (3'UTR) that are not translated. In some embodiments, a polynucleotide (e.g., a ribonucleic acid (RNA), e.g., a messenger RNA (mRNA)) of the disclosure comprising an open reading frame (ORF) encoding an antigen polypeptide further comprises UTR (e.g., a 5'UTR or functional fragment thereof, a 3'UTR or functional fragment thereof, or a combination thereof).

A UTR can be homologous or heterologous to the coding region in a polynucleotide. In some embodiments, the UTR is homologous to the ORF encoding the antigen polypeptide. In some embodiments, the UTR is heterologous to the ORF encoding the antigen polypeptide. In some embodiments, the polynucleotide comprises two or more 5'UTRs or functional fragments thereof, each of which have the same or different nucleotide sequences. In some embodiments, the polynucleotide comprises two or more 3'UTRs or functional fragments thereof, each of which have the same or different nucleotide sequences.

In some embodiments, the 5'UTR or functional fragment thereof, 3' UTR or functional fragment thereof, or any combination thereof is sequence optimized.
In some embodiments, the 5'UTR or functional fragment thereof, 3' UTR or functional fragment thereof, or any combination thereof comprises at least one chemically modified nucleobase, e.g., 5-methoxyuracil.

UTRs can have features that provide a regulatory role, e.g., increased or decreased stability, localization and/or translation efficiency. A polynucleotide comprising a UTR can be administered to a cell, tissue, or organism, and one or more regulatory features can be measured using routine methods. In some embodiments, a functional fragment of a 5'UTR or 3'UTR comprises one or more regulatory features of a full length 5' or 3' UTR, respectively.

By engineering the features typically found in abundantly expressed genes of specific target organs, one can enhance the stability and protein production of a polynucleotide. For example, introduction of 5'UTR of liver-expressed mRNA, such as albumin, serum amyloid A, Apolipoprotein A/B/E, transferrin, alpha fetoprotein, erythropoietin, or Factor VIII, can enhance expression of polynucleotides in hepatic cell lines or liver. Likewise, use of 5'UTR from other tissue-specific mRNA to improve expression in that tissue is possible for muscle (e.g., MyoD, Myosin, Myoglobin, Myogenin, Herculin), for endothelial cells (e.g., Tie-1, CD36), for myeloid cells (e.g., C/EBP, AML1, G-CSF, GM-CSF, CD11b, MSR, Fr-1, i-NOS), for leukocytes (e.g., CD45, CD18), for adipose tissue (e.g., CD36, GLUT4, ACRP30, adiponectin) and for lung epithelial cells (e.g., SP-A/B/C/D).

In some embodiments, UTRs are selected from a family of transcripts whose proteins share a common function, structure, feature or property. For example, an encoded polypeptide can belong to a family of proteins (i.e., that share at least one function, structure, feature, localization, origin, or expression pattern), which are expressed in a particular cell, tissue or at some time during development. The UTRs from any of the genes or mRNA can be swapped for any other UTR of the same or different family of proteins to create a new polynucleotide.

In some embodiments, the 5'UTR and the 3'UTR can be heterologous. In some embodiments, the 5'UTR can be derived from a different species than the 3'UTR. In some embodiments, the 3'UTR can be derived from a different species than the 5'UTR.

Co-owned International Patent Application No. PCT/US2014/021522 (Publ. No. WO/2014/164253) provides a listing of exemplary UTRs that can be utilized in the polynucleotide of the present disclosure as flanking regions to an ORF.

Exemplary UTRs of the application include, but are not limited to, one or more 5'UTR and/or 3'UTR derived from the nucleic acid sequence of: a globin, such as an α- or β-globin (e.g., a Xenopus, mouse, rabbit, or human globin); a strong Kozak translational initiation signal; a CYBA (e.g., human cytochrome b-245 a polypeptide); an albumin (e.g., human albumin7); a HSD17B4 (hydroxysteroid (17-β) dehydrogenase); a virus (e.g., a tobacco etch virus (TEV), a Venezuelan equine encephalitis virus (VEEV), a Dengue virus, a cytomegalovirus (CMV) (e.g., CMV immediate early 1 (IE1)), a hepatitis virus (e.g., hepatitis B virus), a sindbis virus, or a PAV barley yellow dwarf virus); a heat shock protein (e.g., hsp70); a translation initiation factor (e.g., eIF4G); a glucose transporter (e.g., hGLUT1 (human glucose transporter 1)); an actin (e.g., human a or β actin); a GAPDH; a tubulin; a histone; a citric acid cycle enzyme; a topoisomerase (e.g., a 5'UTR of a TOP gene lacking the 5' TOP motif (the oligopyrimidine tract)); a ribosomal protein Large 32 (L32); a ribosomal protein (e.g., human or mouse ribosomal protein, such as, for example, rps9); an ATP synthase (e.g., ATP5A1 or the β subunit of mitochondrial H+-ATP synthase); a growth hormone e (e.g., bovine (bGH) or human (hGH)); an elongation factor (e.g., elongation factor 1 a1 (EEF1A1)); a manganese superoxide dismutase (MnSOD); a myocyte enhancer factor 2A (MEF2A); a β-F1-ATPase, a creatine kinase, a myoglobin, a granulocyte-colony stimulating factor (G-CSF); a collagen (e.g., collagen type I, alpha 2 (Col1A2), collagen type I, alpha 1 (Col1A1), collagen type VI, alpha 2 (Col6A2), collagen type VI, alpha 1 (Col6A1)); a ribophorin (e.g., ribophorin I (RPNI)); a low density lipoprotein receptor-related protein (e.g., LRP1); a cardiotrophin-like cytokine factor (e.g., Nnt1); calreticulin (Calr); a procollagen-lysine, 2-oxoglutarate 5-dioxygenase 1 (Plod1); and a nucleobindin (e.g., Nucb1).

In some embodiments, the 5'UTR is selected from the group consisting of a β-globin 5'UTR; a 5'UTR containing a strong Kozak translational initiation signal; a cytochrome b-245 a polypeptide (CYBA) 5'UTR; a hydroxysteroid (17-β) dehydrogenase (HSD17B4) 5'UTR; a Tobacco etch virus (TEV) 5'UTR; a Venezuelen equine encephalitis virus (TEEV) 5'UTR; a 5' proximal open reading frame of rubella virus (RV) RNA encoding nonstructural proteins; a Dengue virus (DEN) 5'UTR; a heat shock protein 70 (Hsp70) 5'UTR; a elF4G 5'UTR; a GLUT1 5'UTR; functional fragments thereof and any combination thereof.

In some embodiments, the 3'UTR is selected from the group consisting of a β-globin 3'UTR; a CYBA 3'UTR; an albumin 3'UTR; a growth hormone (GH) 3'UTR; a VEEV 3'UTR; a hepatitis B virus (HBV) 3'UTR; α-globin 3'UTR; a DEN 3'UTR; a PAV barley yellow dwarf virus (BYDV-PAV) 3'UTR; an elongation factor 1 a1 (EEF1A1) 3'UTR; a manganese superoxide dismutase (MnSOD) 3'UTR; a β subunit of mitochondrial H(+)-ATP synthase (β-mRNA) 3'UTR; a GLUT1 3'UTR; a MEF2A 3'UTR; a β-F1-ATPase 3'UTR; functional fragments thereof and combinations thereof.

Wild-type UTRs derived from any gene or mRNA can be incorporated into the polynucleotides of the disclosure. In some embodiments, a UTR can be altered relative to a wild type or native UTR to produce a variant UTR, e.g., by changing the orientation or location of the UTR relative to the ORF; or by inclusion of additional nucleotides, deletion of nucleotides, swapping or transposition of nucleotides. In some embodiments, variants of 5' or 3' UTRs can be utilized, for example, mutants of wild type UTRs, or variants wherein one or more nucleotides are added to or removed from a terminus of the UTR.

Additionally, one or more synthetic UTRs can be used in combination with one or more non-synthetic UTRs. See, e.g., Mandal and Rossi, Nat. Protoc. 2013 8(3):568-82, and sequences available at addgene.org/Derrick_Rossi/. UTRs or portions thereof can be placed in the same orientation as in the transcript from which they were selected or can be altered in orientation or location. Hence, a 5' and/or 3' UTR can be inverted, shortened, lengthened, or combined with one or more other 5' UTRs or 3' UTRs.

In some embodiments, the polynucleotide comprises multiple UTRs, e.g., a double, a triple or a quadruple 5'UTR or 3'UTR. For example, a double UTR comprises two copies of the same UTR either in series or substantially in series. For example, a double beta-globin 3'UTR can be used (see US2010/0129877).

In some embodiments, the polynucleotides of the disclosure comprise a 5'UTR and/or a 3'UTR selected from any one of the UTRs disclosed herein. The polynucleotides of the disclosure can comprise combinations of features. For example, the ORF can be flanked by a 5'UTR that comprises a strong Kozak translational initiation signal and/or a 3'UTR comprising an oligo(dT) sequence for templated addition of a poly-A tail. A 5'UTR can comprise a first polynucleotide fragment and a second polynucleotide fragment from the same and/or different UTRs (see, e.g., US2010/0293625).

Other non-UTR sequences can be used as regions or subregions within the polynucleotides of the disclosure. For example, introns or portions of intron sequences can be incorporated into the polynucleotides of the disclosure. Incorporation of intronic sequences can increase protein production as well as polynucleotide expression levels. In some embodiments, the polynucleotide of the disclosure comprises an internal ribosome entry site (IRES) instead of or in addition to a UTR (see, e.g., Yakubov et al., Biochem. Biophys. Res. Commun. 2010 394(1):189-193). In some embodiments, the polynucleotide comprises an IRES instead of a 5'UTR sequence. In some embodiments, the polynucleotide comprises an ORF and a viral capsid sequence. In some embodiments, the polynucleotide comprises a synthetic 5'UTR in combination with a non-synthetic 3'UTR.

In some embodiments, the UTR can also include at least one translation enhancer polynucleotide, translation enhancer element, or translational enhancer elements (collectively, "TEE," which refers to nucleic acid sequences that increase the amount of polypeptide or protein produced from a polynucleotide. As a non-limiting example, the TEE can be located between the transcription promoter and the start codon. In some embodiments, the 5'UTR comprises a TEE.

In one aspect, a TEE is a conserved element in a UTR that can promote translational activity of a nucleic acid such as, but not limited to, cap-dependent or cap-independent translation.

In one non-limiting example, the TEE comprises the TEE sequence in the 5'-leader of the Gtx homeodomain protein. See Chappell et al., PNAS 2004 101:9590-9594.

In some embodiments, the polynucleotide of the disclosure comprises one or multiple copies of a TEE. The TEE in a translational enhancer polynucleotide can be organized in one or more sequence segments. A sequence segment can harbor one or more of the TEEs provided herein, with each TEE being present in one or more copies. When multiple sequence segments are present in a translational enhancer polynucleotide, they can be homogenous or heterogeneous. Thus, the multiple sequence segments in a translational enhancer polynucleotide can harbor identical or different types of the TEE provided herein, identical or different number of copies of each of the TEE, and/or identical or different organization of the TEE within each sequence segment. In one embodiment, the polynucleotide of the disclosure comprises a translational enhancer polynucleotide sequence.

In some embodiments, a 5'UTR and/or 3'UTR comprising at least one TEE described herein can be incorporated in a monocistronic sequence such as, but not limited to, a vector system or a nucleic acid vector.

In some embodiments, a 5'UTR and/or 3'UTR of a polynucleotide of the disclosure comprises a TEE or portion thereof described herein. In some embodiments, the TEEs in the 3'UTR can be the same and/or different from the TEE located in the 5'UTR.

In some embodiments, the spacer separating two TEE sequences can include other sequences known in the art that can regulate the translation of the polynucleotide of the disclosure, e.g., miR sequences described herein (e.g., miR binding sites). As a non-limiting example, each spacer used to separate two TEE sequences can include a different miR sequence (e.g., miR binding site).

In some embodiments, a polynucleotide of the disclosure comprises a miR and/or TEE sequence. In some embodiments, the incorporation of a miR sequence and/or a TEE sequence into a polynucleotide of the disclosure can change the shape of the stem loop region, which can increase and/or decrease translation. See e.g., Kedde et al., Nature Cell Biology 2010 12(10):1014-20).

### II. Lipid Nanoparticles (LNPs)

The mRNA vaccines described herein are superior to current vaccines in several ways. In some aspects the vaccine is formulated in a lipid nanoparticle (LNP). The use of LNPs enables the effective delivery of chemically modified or unmodified mRNA vaccines. Both modified and unmodified LNP formulated mRNA vaccines are superior to conventional vaccines by a significant degree. In some embodiments the mRNA vaccines of the disclosure are superior to conventional vaccines by a factor of at least 10 fold, 20 fold, 40 fold, 50 fold, 100 fold, 500 fold or 1,000 fold.

In some aspects the vaccine is formulated in a lipid nanoparticle (LNP). The use of LNPs enables the effective delivery of chemically modified or unmodified mRNA vaccines. Both modified and unmodified LNP formulated mRNA vaccines are superior to conventional vaccines by a significant degree. In some embodiments the mRNA vaccines of the disclosure are superior to conventional vaccines by a factor of at least 10 fold, 20 fold, 40 fold, 50 fold, 100 fold, 500 fold or 1,000 fold.

In one set of embodiments, lipid nanoparticles (LNPs) are provided. In one embodiment, a lipid nanoparticle comprises lipids including an ionizable lipid (such as an ionizable cationic lipid), a structural lipid, a phospholipid, and mRNA. Each of the LNPs described herein may be used as a formulation for the mRNA described herein. In one embodiment, a lipid nanoparticle comprises an ionizable lipid, a structural lipid, a phospholipid, and mRNA. In some embodiments, the LNP comprises an ionizable lipid, a PEG-modified lipid, a phospholipid and a structural lipid. In some embodiments, the LNP has a molar ratio of about 20-60% ionizable lipid: about 5-25% phospholipid: about 25-55% structural lipid; and about 0.5-15% PEG-modified lipid. In some embodiments, the LNP comprises a molar ratio of about 50% ionizable lipid, about 1.5% PEG-modified lipid, about 38.5% structural lipid and about 10% phospholipid. In some embodiments, the LNP comprises a molar ratio of about 55% ionizable lipid, about 2.5% PEG lipid, about 32.5% structural lipid and about 10% phospholipid. In some embodiments, the ionizable lipid is an ionizable amino or cationic lipid and the phospholipid is a neutral lipid, and the structural lipid is a cholesterol. In some embodiments, the LNP has a molar ratio of 50:38.5:10:1.5 of ionizable lipid: cholesterol:DSPC:PEG2000-DMG.

The ionizable lipids described herein (e.g. those having any of Formula (I), (IA), (II), (IIa), (IIb), (IIc), (IId), (IIe), (III), (IV), (V), or (VI) may be advantageously used in lipid nanoparticle compositions for the delivery of vaccines to mammalian cells or organs. In some embodiments, the ionizable lipids have the Formula (I) 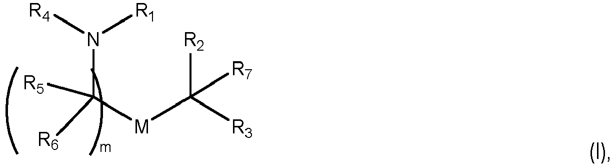 wherein
R₁ is selected from the group consisting of C₅₋₃₀ alkyl, C₅₋₂₀ alkenyl, -R*YR", -YR", and -R"M'R';
R₂ and R₃ are independently selected from the group consisting of H, C₁₋₁₄ alkyl, C₂₋₁₄ alkenyl, -R*YR", -YR", and -R*OR", or R₂ and R₃, together with the atom to which they are attached, form a heterocycle or carbocycle;
R₄ is selected from the group consisting of a C₃₋₆ carbocycle, -(CH₂)ₙQ, -(CH₂)ₙCHQR, -CHQR, -CQ(R)₂, and unsubstituted C₁₋₆ alkyl, where Q is selected from a carbocycle, heterocycle, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, -N(R)R₈, -O(CH₂)ₙOR, -N(R)C(=NR₉)N(R)₂, -N(R)C(=CHR₉)N(R)₂, -OC(O)N(R)₂, -N(R)C(O)OR, -N(OR)C(O)R, -N(OR)S(O)₂R, -N(OR)C(O)OR, -N(OR)C(O)N(R)₂, -N(OR)C(S)N(R)₂, -N(OR)C(=NR₉)N(R)₂, -N(OR)C(=CHR₉)N(R)₂, -C(=NR₉)N(R)₂, -C(=NR₉)R, -C(O)N(R)OR, and -C(R)N(R)₂C(O)OR, and each n is independently selected from 1, 2, 3, 4, and 5;
each R₅ is independently selected from the group consisting of C₁₋₃ alkyl, C₂₋₃ alkenyl, and H;
each R₆ is independently selected from the group consisting of C₁₋₃ alkyl, C₂₋₃ alkenyl, and H;
M and M' are independently selected from -C(O)O-, -OC(O)-, -C(O)N(R')-, -N(R')C(O)-, -C(O)-, -C(S)-, -C(S)S-, -SC(S)-, -CH(OH)-, -P(O)(OR')O-, -S(O)₂-, -S-S-, an aryl group, and a heteroaryl group;
R₇ is selected from the group consisting of C₁₋₃ alkyl, C₂₋₃ alkenyl, and H;
R₈ is selected from the group consisting of C₃₋₆ carbocycle and heterocycle;
R₉ is selected from the group consisting of H, CN, NO₂, C₁₋₆ alkyl, -OR, -S(O)₂R, -S(O)₂N(R)₂, C₂₋₆ alkenyl, C₃₋₆ carbocycle and heterocycle;
each R is independently selected from the group consisting of C₁₋₃ alkyl, C₂₋₃ alkenyl, and H;
each R' is independently selected from the group consisting of C₁₋₁₈ alkyl, C₂₋₁₈ alkenyl, -R*YR", -YR", and H;
each R" is independently selected from the group consisting of C₃₋₁₄ alkyl and C₃₋₁₄ alkenyl;
each R* is independently selected from the group consisting of C₁₋₁₂ alkyl and C₂₋₁₂ alkenyl;
each Y is independently a C₃₋₆ carbocycle;
each X is independently selected from the group consisting of F, Cl, Br, and I; and m is selected from 5, 6, 7, 8, 9, 10, 11, 12, and 13,
or salts or stereoisomers thereof.

In some embodiments, a subset of compounds of Formula (I) includes those in which
R₁ is selected from the group consisting of C₅₋₂₀ alkyl, C₅₋₂₀ alkenyl, -R*YR", -YR", and -R"M'R';
R₂ and R₃ are independently selected from the group consisting of H, C₁₋₁₄ alkyl, C₂₋₁₄ alkenyl, -R*YR", -YR", and -R*OR", or R₂ and R₃, together with the atom to which they are attached, form a heterocycle or carbocycle;
R₄ is selected from the group consisting of a C₃₋₆ carbocycle, -(CH₂)ₙQ, -(CH₂)ₙCHQR, -CHQR, -CQ(R)₂, and unsubstituted C₁₋₆ alkyl, where Q is selected from a carbocycle, heterocycle, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, and -C(R)N(R)₂C(O)OR, and each n is independently selected from 1, 2, 3, 4, and 5;
each R₅ is independently selected from the group consisting of C₁₋₃ alkyl, C₂₋₃ alkenyl, and H;
each R₆ is independently selected from the group consisting of C₁₋₃ alkyl, C₂₋₃ alkenyl, and H;
M and M' are independently selected from -C(O)O-, -OC(O)-, -C(O)N(R')-, -N(R')C(O)-, -C(O)-, -C(S)-, -C(S)S-, -SC(S)-, -CH(OH)-, -P(O)(OR')O-, -S(O)₂-, an aryl group, and a heteroaryl group;
R₇ is selected from the group consisting of C₁₋₃ alkyl, C₂₋₃ alkenyl, and H;
each R is independently selected from the group consisting of C₁₋₃ alkyl, C₂₋₃ alkenyl, and H;
each R' is independently selected from the group consisting of C₁₋₁₈ alkyl, C₂₋₁₈ alkenyl, -R*YR", -YR", and H;
each R" is independently selected from the group consisting of C₃₋₁₄ alkyl and C₃₋₁₄ alkenyl;
each R* is independently selected from the group consisting of C₁₋₁₂ alkyl and C₂₋₁₂ alkenyl;
each Y is independently a C₃₋₆ carbocycle;
each X is independently selected from the group consisting of F, Cl, Br, and I; and m is selected from 5, 6, 7, 8, 9, 10, 11, 12, and 13,
or salts or stereoisomers thereof, wherein alkyl and alkenyl groups may be linear or branched.

In some embodiments, a subset of compounds of Formula (I) includes those in which when R₄ is -(CH₂)ₙQ, -(CH₂)ₙCHQR, -CHQR, or -CQ(R)₂, then (i) Q is not -N(R)₂ when n is 1, 2, 3, 4 or 5, or (ii) Q is not 5, 6, or 7-membered heterocycloalkyl when n is 1 or 2.

In another embodiments, another subset of compounds of Formula (I) includes those in which
R₁ is selected from the group consisting of C₅₋₃₀ alkyl, C₅₋₂₀ alkenyl, -R*YR", -YR", and -R"M'R';
R₂ and R₃ are independently selected from the group consisting of H, C₁₋₁₄ alkyl, C₂₋₁₄ alkenyl, -R*YR", -YR", and -R*OR", or R₂ and R₃, together with the atom to which they are attached, form a heterocycle or carbocycle;
R₄ is selected from the group consisting of a C₃₋₆ carbocycle, -(CH₂)ₙQ, -(CH₂)ₙCHQR, -CHQR, -CQ(R)₂, and unsubstituted C₁₋₆ alkyl, where Q is selected from a C₃₋₆ carbocycle, a 5- to 14-membered heteroaryl having one or more heteroatoms selected from N, O, and S, -OR, -O(CH₂)ₙN(R)₂, -C(O)OR, -OC(O)R, -CX₃, -CX₂H, -CXH₂, -CN, -C(O)N(R)₂, -N(R)C(O)R, -N(R)S(O)₂R, -N(R)C(O)N(R)₂, -N(R)C(S)N(R)₂,
   -CRN(R)₂C(O)OR, -N(R)R₈, -O(CH₂)ₙOR, -N(R)C(=NR₉)N(R)₂, -N(R)C(=CHR₉)N(R)₂,
   -OC(O)N(R)₂, -N(R)C(O)OR, -N(OR)C(O)R, -N(OR)S(O)₂R, -N(OR)C(O)OR, -N(OR)C(O)N(R)₂, -N(OR)C(S)N(R)₂, -N(OR) C(=NR₉)N(R)₂,-N(OR)C(=CHR₉)N(R)₂,
   -C(=NR₉)N(R)₂, -C(=NR₉)R, -C(O)N(R)OR, and a 5- to 14-membered heterocycloalkyl having one or more heteroatoms selected from N, O, and S which is substituted with one or more substituents selected from oxo (=O), OH, amino, and C₁₋₃ alkyl, and each n is independently selected from 1, 2, 3, 4, and 5;
each R₅ is independently selected from the group consisting of C₁₋₃ alkyl, C₂₋₃ alkenyl, and H;
each R₆ is independently selected from the group consisting of C₁₋₃ alkyl, C₂₋₃ alkenyl, and H;
M and M' are independently selected from -C(O)O-, -OC(O)-, -C(O)N(R')-, -N(R')C(O)-, -C(O)-, -C(S)-, -C(S)S-, -SC(S)-, -CH(OH)-, -P(O)(OR')O-, -S(O)₂-, -S-S-, an aryl group, and a heteroaryl group;
R₇ is selected from the group consisting of C₁₋₃ alkyl, C₂₋₃ alkenyl, and H;
R₈ is selected from the group consisting of C₃₋₆ carbocycle and heterocycle;
R₉ is selected from the group consisting of H, CN, NO₂, C₁₋₆ alkyl, -OR, -S(O)₂R, -S(O)₂N(R)₂, C₂₋₆ alkenyl, C₃₋₆ carbocycle and heterocycle;
each R is independently selected from the group consisting of C₁₋₃ alkyl, C₂₋₃ alkenyl, and H;
each R' is independently selected from the group consisting of C₁₋₁₈ alkyl, C₂₋₁₈ alkenyl, -R*YR", -YR", and H;
each R" is independently selected from the group consisting of C₃₋₁₄ alkyl and C₃₋₁₄ alkenyl;
each R* is independently selected from the group consisting of C₁₋₁₂ alkyl and C₂₋₁₂ alkenyl;
each Y is independently a C₃₋₆ carbocycle;
each X is independently selected from the group consisting of F, Cl, Br, and I; and
m is selected from 5, 6, 7, 8, 9, 10, 11, 12, and 13,
or salts or stereoisomers thereof.

In another embodiments, another subset of compounds of Formula (I) includes those in which
R₁ is selected from the group consisting of C₅₋₃₀ alkyl, C₅₋₂₀ alkenyl, -R*YR", -YR", and -R"M'R';
R₂ and R₃ are independently selected from the group consisting of H, C₁₋₁₄ alkyl, C₂₋₁₄ alkenyl, -R*YR", -YR", and -R*OR", or R₂ and R₃, together with the atom to which they are attached, form a heterocycle or carbocycle;
R₄ is selected from the group consisting of a C₃₋₆ carbocycle, -(CH₂)ₙQ, -(CH₂)ₙCHQR, -CHQR, -CQ(R)₂, and unsubstituted C₁₋₆ alkyl, where Q is selected from a C₃₋₆ carbocycle, a 5- to 14-membered heteroaryl having one or more heteroatoms selected from N, O, and S, -OR, -O(CH₂)ₙN(R)₂, -C(O)OR, -OC(O)R, -CX₃, -CX₂H, -CXH₂, -CN, -C(O)N(R)₂, -N(R)C(O)R, -N(R)S(O)₂R, -N(R)C(O)N(R)₂, -N(R)C(S)N(R)₂, -CRN(R)₂C(O)OR, and a 5- to 14-membered heterocycloalkyl having one or more heteroatoms selected from N, O, and S which is substituted with one or more substituents selected from oxo (=O), OH, amino, and C₁₋₃ alkyl, and each n is independently selected from 1, 2, 3, 4, and 5;
each R₅ is independently selected from the group consisting of C₁₋₃ alkyl, C₂₋₃ alkenyl, and H;
each R₆ is independently selected from the group consisting of C₁₋₃ alkyl, C₂₋₃ alkenyl, and H;
M and M' are independently selected from -C(O)O-, -OC(O)-, -C(O)N(R')-, -N(R')C(O)-, -C(O)-, -C(S)-, -C(S)S-, -SC(S)-, -CH(OH)-, -P(O)(OR')O-, -S(O)₂-, an aryl group, and a heteroaryl group;
R₇ is selected from the group consisting of C₁₋₃ alkyl, C₂₋₃ alkenyl, and H;
each R is independently selected from the group consisting of C₁₋₃ alkyl, C₂₋₃ alkenyl, and H;
each R' is independently selected from the group consisting of C₁₋₁₈ alkyl, C₂₋₁₈ alkenyl, -R*YR", -YR", and H;
each R" is independently selected from the group consisting of C₃₋₁₄ alkyl and C₃₋₁₄ alkenyl;
each R* is independently selected from the group consisting of C₁₋₁₂ alkyl and C₂₋₁₂ alkenyl;
each Y is independently a C₃₋₆ carbocycle;
each X is independently selected from the group consisting of F, Cl, Br, and I; and
m is selected from 5, 6, 7, 8, 9, 10, 11, 12, and 13,
or salts or stereoisomers thereof.

In yet another embodiments, another subset of compounds of Formula (I) includes those in which
R₁ is selected from the group consisting of C₅₋₂₀ alkyl, C₅₋₂₀ alkenyl, -R*YR", -YR", and -R"M'R';
R₂ and R₃ are independently selected from the group consisting of H, C₁₋₁₄ alkyl, C₂₋₁₄ alkenyl, -R*YR", -YR", and -R*OR", or R₂ and R₃, together with the atom to which they are attached, form a heterocycle or carbocycle;
R₄ is selected from the group consisting of a C₃₋₆ carbocycle, -(CH₂)ₙQ, -(CH₂)ₙCHQR, -CHQR, -CQ(R)₂, and unsubstituted C₁₋₆ alkyl, where Q is selected from a C₃₋₆ carbocycle, a 5- to 14-membered heterocycle having one or more heteroatoms selected from N, O, and S, -OR, -O(CH₂)ₙN(R)₂, -C(O)OR, -OC(O)R, -CX₃, -CX₂H, -CXH₂, -CN, -C(O)N(R)₂, -N(R)C(O)R, -N(R)S(O)₂R, -N(R)C(O)N(R)₂, -N(R)C(S)N(R)₂, -CRN(R)₂C(O)OR, -N(R)R₈, -O(CH₂)ₙOR, -N(R)C(=NR₉)N(R)₂, -N(R)C(=CHR₉)N(R)₂, -OC(O)N(R)₂, -N(R)C(O)OR, -N(OR)C(O)R, -N(OR)S(O)₂R, -N(OR)C(O)OR, -N(OR)C(O)N(R)₂, -N(OR)C(S)N(R)₂, -N(OR)C(=NR₉)N(R)₂, -N(OR)C(=CHR₉)N(R)₂, -C(=NR₉)R, -C(O)N(R)OR, and -C(=NR₉)N(R)₂, and each n is independently selected from 1, 2, 3, 4, and 5; and when Q is a 5- to 14-membered heterocycle and (i) R₄ is -(CH₂)ₙQ in which n is 1 or 2, or (ii) R₄ is -(CH₂)ₙCHQR in which n is 1, or (iii) R₄ is -CHQR, and -CQ(R)₂, then Q is either a 5- to 14-membered heteroaryl or 8- to 14-membered heterocycloalkyl;
each R₅ is independently selected from the group consisting of C₁₋₃ alkyl, C₂₋₃ alkenyl, and H;
each R₆ is independently selected from the group consisting of C₁₋₃ alkyl, C₂₋₃ alkenyl, and H;
M and M' are independently selected from -C(O)O-, -OC(O)-, -C(O)N(R')-, -N(R')C(O)-, -C(O)-, -C(S)-, -C(S)S-, -SC(S)-, -CH(OH)-, -P(O)(OR')O-, -S(O)₂-, -S-S-, an aryl group, and a heteroaryl group;
R₇ is selected from the group consisting of C₁₋₃ alkyl, C₂₋₃ alkenyl, and H;
R₈ is selected from the group consisting of C₃₋₆ carbocycle and heterocycle;
R₉ is selected from the group consisting of H, CN, NO₂, C₁₋₆ alkyl, -OR, -S(O)₂R, -S(O)₂N(R)₂, C₂₋₆ alkenyl, C₃₋₆ carbocycle and heterocycle;
each R is independently selected from the group consisting of C₁₋₃ alkyl, C₂₋₃ alkenyl, and H;
each R' is independently selected from the group consisting of C₁₋₁₈ alkyl, C₂₋₁₈ alkenyl, -R*YR", -YR", and H;
each R" is independently selected from the group consisting of C₃₋₁₄ alkyl and C₃₋₁₄ alkenyl;
each R* is independently selected from the group consisting of C₁₋₁₂ alkyl and C₂₋₁₂ alkenyl;
each Y is independently a C₃₋₆ carbocycle;
each X is independently selected from the group consisting of F, C!, Br, and I; and
m is selected from 5, 6, 7, 8, 9, 10, 11, 12, and 13,
or salts or stereoisomers thereof.

In yet another embodiments, another subset of compounds of Formula (I) includes those in which
R₁ is selected from the group consisting of C₅₋₂₀ alkyl, C₅₋₂₀ alkenyl, -R*YR", -YR", and -R"M'R';
R₂ and R₃ are independently selected from the group consisting of H, C₁₋₁₄ alkyl, C₂₋₁₄ alkenyl, -R*YR", -YR", and -R*OR", or R₂ and R₃, together with the atom to which they are attached, form a heterocycle or carbocycle;
R₄ is selected from the group consisting of a C₃₋₆ carbocycle, -(CH₂)ₙQ, -(CH₂)ₙCHQR, -CHQR, -CQ(R)₂, and unsubstituted C₁₋₆ alkyl, where Q is selected from a C₃₋₆ carbocycle, a 5- to 14-membered heterocycle having one or more heteroatoms selected from N, O, and S, -OR, -O(CH₂)ₙN(R)₂, -C(O)OR, -OC(O)R, -CX₃, -CX₂H, -CXH₂, -CN, -C(O)N(R)₂, -N(R)C(O)R, -N(R)S(O)₂R, -N(R)C(O)N(R)₂, -N(R)C(S)N(R)₂, -CRN(R)₂C(O)OR, and each n is independently selected from 1, 2, 3, 4, and 5; and when Q is a 5- to 14-membered heterocycle and (i) R₄ is -(CH₂)ₙQ in which n is 1 or 2, or (ii) R₄ is -(CH₂)ₙCHQR in which n is 1, or (iii) R₄ is -CHQR, and -CQ(R)₂, then Q is either a 5- to 14-membered heteroaryl or 8- to 14-membered heterocycloalkyl;
each R₅ is independently selected from the group consisting of C₁₋₃ alkyl, C₂₋₃ alkenyl, and H;
each R₆ is independently selected from the group consisting of C₁₋₃ alkyl, C₂₋₃ alkenyl, and H;
M and M' are independently selected from -C(O)O-, -OC(O)-, -C(O)N(R')-, -N(R')C(O)-, -C(O)-, -C(S)-, -C(S)S-, -SC(S)-, -CH(OH)-, -P(O)(OR')O-, -S(O)₂-, an aryl group, and a heteroaryl group;
R₇ is selected from the group consisting of C₁₋₃ alkyl, C₂₋₃ alkenyl, and H;
each R is independently selected from the group consisting of C₁₋₃ alkyl, C₂₋₃ alkenyl, and H;
each R' is independently selected from the group consisting of C₁₋₁₈ alkyl, C₂₋₁₈ alkenyl, -R*YR", -YR", and H;
each R" is independently selected from the group consisting of C₃₋₁₄ alkyl and C₃₋₁₄ alkenyl;
each R* is independently selected from the group consisting of C₁₋₁₂ alkyl and C₂₋₁₂ alkenyl;
each Y is independently a C₃₋₆ carbocycle;
each X is independently selected from the group consisting of F, Cl, Br, and I; and
m is selected from 5, 6, 7, 8, 9, 10, 11, 12, and 13,
or salts or stereoisomers thereof.

In still another embodiments, another subset of compounds of Formula (I) includes those in which
R₁ is selected from the group consisting of C₅₋₃₀ alkyl, C₅₋₂₀ alkenyl, -R*YR", -YR", and -R"M'R';
R₂ and R₃ are independently selected from the group consisting of H, C₁₋₁₄ alkyl, C₂₋₁₄ alkenyl, -R*YR", -YR", and -R*OR", or R₂ and R₃, together with the atom to which they are attached, form a heterocycle or carbocycle;
R₄ is selected from the group consisting of a C₃₋₆ carbocycle, -(CH₂)ₙQ, -(CH₂)ₙCHQR, -CHQR, -CQ(R)₂, and unsubstituted C₁₋₆ alkyl, where Q is selected from a C₃₋₆ carbocycle, a 5- to 14-membered heteroaryl having one or more heteroatoms selected from N, O, and S, -OR, -O(CH₂)ₙN(R)₂, -C(O)OR, -OC(O)R, -CX₃, -CX₂H, -CXH₂, -CN, -C(O)N(R)₂, -N(R)C(O)R, -N(R)S(O)₂R, -N(R)C(O)N(R)₂, -N(R)C(S)N(R)₂, -CRN(R)₂C(O)OR, -N(R)R₈, -O(CH₂)ₙOR, -N(R)C(=NR₉)N(R)₂, -N(R)C(=CHR₉)N(R)₂, -OC(O)N(R)₂, -N(R)C(O)OR, -N(OR)C(O)R, -N(OR)S(O)₂R, -N(OR)C(O)OR, -N(OR)C(O)N(R)₂, -N(OR)C(S)N(R)₂, -N(OR)C(=NR₉)N(R)₂, -N(OR)C(=CHR₉)N(R)₂, -C(=NR₉)R, -C(O)N(R)OR, and -C(=NR₉)N(R)₂, and each n is independently selected from 1, 2, 3, 4, and 5;
each R₅ is independently selected from the group consisting of C₁₋₃ alkyl, C₂₋₃ alkenyl, and H;
each R₆ is independently selected from the group consisting of C₁₋₃ alkyl, C₂₋₃ alkenyl, and H;
M and M' are independently selected from -C(O)O-, -OC(O)-, -C(O)N(R')-, -N(R')C(O)-, -C(O)-, -C(S)-, -C(S)S-, -SC(S)-, -CH(OH)-, -P(O)(OR')O-, -S(O)₂-, -S-S-, an aryl group, and a heteroaryl group;
R₇ is selected from the group consisting of C₁₋₃ alkyl, C₂₋₃ alkenyl, and H;
R₈ is selected from the group consisting of C₃₋₆ carbocycle and heterocycle;
R₉ is selected from the group consisting of H, CN, NO₂, C₁₋₆ alkyl, -OR, -S(O)₂R, -S(O)₂N(R)₂, C₂₋₆ alkenyl, C₃₋₆ carbocycle and heterocycle;
each R is independently selected from the group consisting of C₁₋₃ alkyl, C₂₋₃ alkenyl, and H;
each R' is independently selected from the group consisting of C₁₋₁₈ alkyl, C₂₋₁₈ alkenyl, -R*YR", -YR", and H;
each R" is independently selected from the group consisting of C₃-₁₄ alkyl and C₃₋₁₄ alkenyl;
each R* is independently selected from the group consisting of C₁₋₁₂ alkyl and C₂₋₁₂ alkenyl;
each Y is independently a C₃₋₆ carbocycle;
each X is independently selected from the group consisting of F, Cl, Br, and I; and
m is selected from 5, 6, 7, 8, 9, 10, 11, 12, and 13,
or salts or stereoisomers thereof.

In certain embodiments, a subset of compounds of Formula (I) includes those of Formula (IA): 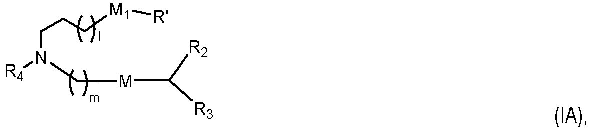
or a salt or stereoisomer thereof, wherein I is selected from 1, 2, 3, 4, and 5; m is selected from 5, 6, 7, 8, and 9; M₁ is a bond or M'; R₄ is unsubstituted C₁₋₃ alkyl, or -(CH₂)ₙQ, in which Q is OH, -NHC(S)N(R)₂, -NHC(O)N(R)₂, -N(R)C(O)R, -N(R)S(O)₂R, -N(R)R₈, -NHC(=NR₉)N(R)₂, -NHC(=CHR₉)N(R)₂, -OC(O)N(R)₂, -N(R)C(O)OR, heteroaryl, or heterocycloalkyl; M and M' are independently selected from -C(O)O-, -OC(O)-, -C(O)N(R')-, -P(O)(OR')O-, -S-S-, an aryl group, and a heteroaryl group; and
R₂ and R₃ are independently selected from the group consisting of H, C₁₋₁₄ alkyl, and C₂₋₁₄ alkenyl.

In certain embodiments, a subset of compounds of Formula (I) includes those of Formula (II): 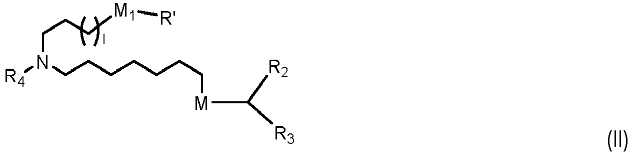
or a salt or stereoisomer thereof, wherein I is selected from 1, 2, 3, 4, and 5; M₁ is a bond or M'; R₄ is unsubstituted C₁₋₃ alkyl, or -(CH₂)ₙQ, in which n is 2, 3, or 4, and Q is OH, -NHC(S)N(R)₂, -NHC(O)N(R)₂, -N(R)C(O)R, -N(R)S(O)₂R, -N(R)R₈, -NHC(=NR₉)N(R)₂, -NHC(=CHR₉)N(R)₂, -OC(O)N(R)₂, -N(R)C(O)OR, heteroaryl, or heterocycloalkyl; M and M' are independently selected from -C(O)O-, -OC(O)-, -C(O)N(R')-, -P(O)(OR')O-, -S-S-, an aryl group, and a heteroaryl group; and
R₂ and R₃ are independently selected from the group consisting of H, C₁₋₁₄ alkyl, and C₂₋₁₄ alkenyl.

In some embodiments, the compound of formula (I) is of the formula (iia), 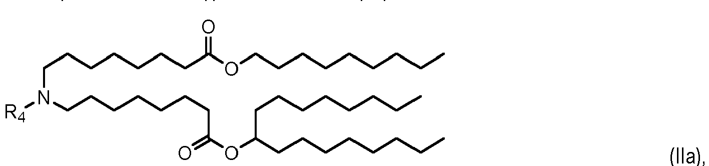 or a salt thereof, wherein R₄ is as described above.

In some embodiments, the compound of formula (I) is of the formula (lib), 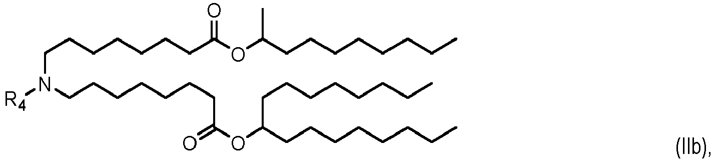 or a salt thereof, wherein R₄ is as described above.

In some embodiments, the compound of formula (I) is of the formula (IIc), 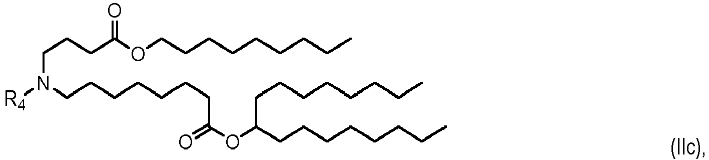 or a salt thereof, wherein R₄ is as described above.

In some embodiments, the compound of formula (I) is of the formula (lie): 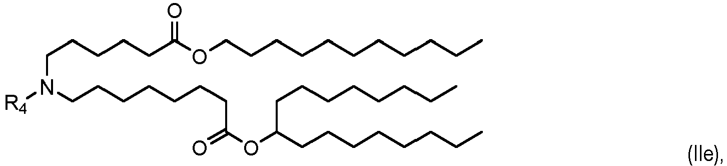 or a salt thereof, wherein R₄ is as described above.

In some embodiments, the compound of formula (I) is of the formula (Ild), 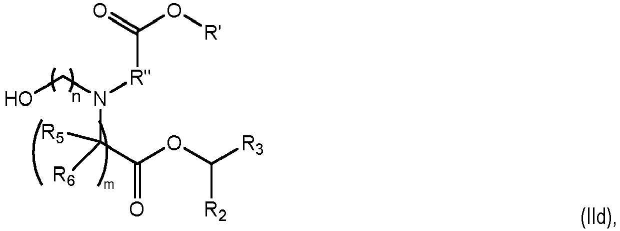 or a salt thereof, wherein R₂ and R₃ are independently selected from the group consisting of C₅₋₁₄ alkyl and C₅₋₁₄ alkenyl, n is selected from 2, 3, and 4, and R', R", R₅, R₆ and m are as defined above.

As used herein, the term "alkyl" or "alkyl group" means a linear or branched, saturated hydrocarbon including one or more carbon atoms (e.g., one, two, three, four, five, six, seven, eight, nine, ten, eleven, twelve, thirteen, fourteen, fifteen, sixteen, seventeen, eighteen, nineteen, twenty, or more carbon atoms).

The notation "C₁₋₁₄ alkyl" means a linear or branched, saturated hydrocarbon including 1-14 carbon atoms. An alkyl group can be optionally substituted.

As used herein, the term "alkenyl" or "alkenyl group" means a linear or branched hydrocarbon including two or more carbon atoms (e.g., two, three, four, five, six, seven, eight, nine, ten, eleven, twelve, thirteen, fourteen, fifteen, sixteen, seventeen, eighteen, nineteen, twenty, or more carbon atoms) and at least one double bond.

The notation "C₂₋₁₄ alkenyl" means a linear or branched hydrocarbon including 2-14 carbon atoms and at least one double bond. An alkenyl group can include one, two, three, four, or more double bonds. For example, C₁₈ alkenyl can include one or more double bonds. A C₁₈ alkenyl group including two double bonds can be a linoleyl group. An alkenyl group can be optionally substituted.

As used herein, the term "carbocycle" or "carbocyclic group" means a mono- or multi-cyclic system including one or more rings of carbon atoms. Rings can be three, four, five, six, seven, eight, nine, ten, eleven, twelve, thirteen, fourteen, or fifteen membered rings.

The notation "C₃₋₆ carbocycle" means a carbocycle including a single ring having 3-6 carbon atoms. Carbocycles can include one or more double bonds and can be aromatic (e.g., aryl groups). Examples of carbocycles include cyclopropyl, cyclopentyl, cyclohexyl, phenyl, naphthyl, and 1,2-dihydronaphthyl groups. Carbocycles can be optionally substituted.

As used herein, the term "heterocycle" or "heterocyclic group" means a mono- or multi-cyclic system including one or more rings, where at least one ring includes at least one heteroatom. Heteroatoms can be, for example, nitrogen, oxygen, or sulfur atoms. Rings can be three, four, five, six, seven, eight, nine, ten, eleven, or twelve membered rings. Heterocycles can include one or more double bonds and can be aromatic (e.g., heteroaryl groups). Examples of heterocycles include imidazolyl, imidazolidinyl, oxazolyl, oxazolidinyl, thiazolyl, thiazolidinyl, pyrazolidinyl, pyrazolyl, isoxazolidinyl, isoxazolyl, isothiazolidinyl, isothiazolyl, morpholinyl, pyrrolyl, pyrrolidinyl, furyl, tetrahydrofuryl, thiophenyl, pyridinyl, piperidinyl, quinolyl, and isoquinolyl groups. Heterocycles can be optionally substituted.

As used herein, a "biodegradable group" is a group that can facilitate faster metabolism of a lipid in a subject. A biodegradable group can be, but is not limited to, -C(O)O-, -OC(O)-, -C(O)N(R')-, -N(R')C(O)-, -C(O)-, -C(S)-, -C(S)S-, -SC(S)-, -CH(OH)-, -P(O)(OR')O-, -S(O)₂-, an aryl group, and a heteroaryl group.

As used herein, an "aryl group" is a carbocyclic group including one or more aromatic rings. Examples of aryl groups include phenyl and naphthyl groups.

As used herein, a "heteroaryl group" is a heterocyclic group including one or more aromatic rings. Examples of heteroaryl groups include pyrrolyl, furyl, thiophenyl, imidazolyl, oxazolyl, and thiazolyl. Both aryl and heteroaryl groups can be optionally substituted. For example, M and M' can be selected from the non-limiting group consisting of optionally substituted phenyl, oxazole, and thiazole. In the formulas herein, M and M' can be independently selected from the list of biodegradable groups above.

Alkyl, alkenyl, and cyclyl (e.g., carbocyclyl and heterocyclyl) groups can be optionally substituted unless otherwise specified. Optional substituents can be selected from the group consisting of, but are not limited to, a halogen atom (e.g., a chloride, bromide, fluoride, or iodide group), a carboxylic acid (e.g., -C(O)OH), an alcohol (e.g., a hydroxyl, -OH), an ester (e.g., -C(O)OR or -OC(O)R), an aldehyde (e.g., -C(O)H), a carbonyl (e.g., -C(O)R, alternatively represented by C=O), an acyl halide (e.g., -C(O)X, in which X is a halide selected from bromide, fluoride, chloride, and iodide), a carbonate (e.g., -OC(O)OR), an alkoxy (e.g., -OR), an acetal (e.g., -C(OR)₂R"", in which each OR are alkoxy groups that can be the same or different and R"" is an alkyl or alkenyl group), a phosphate (e.g., P(O)₄³⁻), a thiol (e.g., -SH), a sulfoxide (e.g., -S(O)R), a sulfinic acid (e.g., -S(O)OH), a sulfonic acid (e.g., -S(O)₂OH), a thial (e.g., -C(S)H), a sulfate (e.g., S(O)₄² ), a sulfonyl (e.g., -S(O)₂-), an amide (e.g., -C(O)NR₂, or -N(R)C(O)R), an azido (e.g., -N₃), a nitro (e.g., -NO₂), a cyano (e.g., -CN), an isocyano (e.g., -NC), an acyloxy (e.g., -OC(O)R), an amino (e.g., -NR₂, -NRH, or -NH₂), a carbamoyl (e.g., -OC(O)NR₂, -OC(O)NRH, or -OC(O)NH₂), a sulfonamide (e.g., -S(O)₂NR₂, -S(O)₂NRH, -S(O)₂NH₂, -N(R)S(O)₂R, -N(H)S(O)₂R, -N(R)S(O)₂H, or -N(H)S(O)₂H), an alkyl group, an alkenyl group, and a cyclyl (e.g., carbocyclyl or heterocyclyl) group.

In any of the preceding, R is an alkyl or alkenyl group, as defined herein. In some embodiments, the substituent groups themselves can be further substituted with, for example, one, two, three, four, five, or six substituents as defined herein. For example, a C₁₋₆ alkyl group can be further substituted with one, two, three, four, five, or six substituents as described herein.

The compounds of any one of formulae (I), (IA), (II), (IIa), (IIb), (IIc), (IId), and (IIe) include one or more of the following features when applicable.

In some embodiments, R₄ is selected from the group consisting of a C₃₋₆ carbocycle, -(CH₂)ₙQ, -(CH₂)ₙCHQR, -CHQR, and -CQ(R)₂, where Q is selected from a C₃₋₆ carbocycle, 5- to 14- membered aromatic or non-aromatic heterocycle having one or more heteroatoms selected from N, O, S, and P, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, and -C(R)N(R)₂C(O)OR, and each n is independently selected from 1, 2, 3, 4, and 5.

In another embodiment, R₄ is selected from the group consisting of a C₃₋₆ carbocycle, -(CH₂)ₙQ, -(CH₂)ₙCHQR, -CHQR, and -CQ(R)₂, where Q is selected from a C₃₋₆ carbocycle, a 5- to 14-membered heteroaryl having one or more heteroatoms selected from N, O, and S, -OR, -O(CH₂)ₙN(R)₂, -C(O)OR, -OC(O)R, -CX₃, -CX₂H, -CXH₂, -CN, -C(O)N(R)₂, -N(R)C(O)R, -N(R)S(O)₂R, -N(R)C(O)N(R)₂, -N(R)C(S)N(R)₂, -C(R)N(R)₂C(O)OR, and a 5- to 14-membered heterocycloalkyl having one or more heteroatoms selected from N, O, and S which is substituted with one or more substituents selected from oxo (=O), OH, amino, and C₁₋₃ alkyl, and each n is independently selected from 1, 2, 3, 4, and 5.

In another embodiment, R₄ is selected from the group consisting of a C₃₋₆ carbocycle, -(CH₂)ₙQ, -(CH₂)ₙCHQR, -CHQR, and -CQ(R)₂, where Q is selected from a C₃₋₆ carbocycle, a 5- to 14-membered heterocycle having one or more heteroatoms selected from N, O, and S, -OR, -O(CH₂)ₙN(R)₂, -C(O)OR, -OC(O)R, -CX₃, -CX₂H, -CXH₂, -CN, -C(O)N(R)₂, -N(R)C(O)R, -N(R)S(O)₂R, -N(R)C(O)N(R)₂, -N(R)C(S)N(R)₂, -C(R)N(R)₂C(O)OR, and each n is independently selected from 1, 2, 3, 4, and 5; and when Q is a 5- to 14-membered heterocycle and (i) R₄ is -(CH₂)ₙQ in which n is 1 or 2, or (ii) R₄ is -(CH₂)ₙCHQR in which n is 1, or (iii) R₄ is -CHQR, and -CQ(R)₂, then Q is either a 5- to 14-membered heteroaryl or 8- to 14-membered heterocycloalkyl.

In another embodiment, R₄ is selected from the group consisting of a C₃₋₆ carbocycle, -(CH₂)ₙQ, -(CH₂)ₙCHQR, -CHQR, and -CQ(R)₂, where Q is selected from a C₃₋₆ carbocycle, a 5- to 14-membered heteroaryl having one or more heteroatoms selected from N, O, and S, -OR, -O(CH₂)ₙN(R)₂, -C(O)OR, -OC(O)R, -CX₃, -CX₂H, -CXH₂, -CN, -C(O)N(R)₂, -N(R)C(O)R, -N(R)S(O)₂R, -N(R)C(O)N(R)₂, -N(R)C(S)N(R)₂, -C(R)N(R)₂C(O)OR, and each n is independently selected from 1, 2, 3, 4, and 5.

In another embodiment, R₄ is unsubstituted C₁₋₄ alkyl, e.g., unsubstituted methyl.

In certain embodiments, the disclosure provides a compound having the Formula (I), wherein R₄ is -(CH₂)ₙQ or -(CH₂)ₙCHQR, where Q is -N(R)₂, and n is selected from 3, 4, and 5.

In certain embodiments, the disclosure provides a compound having the Formula (I), wherein R₄ is selected from the group consisting of -(CH₂)ₙQ, -(CH₂)ₙCHQR, -CHQR, and -CQ(R)₂, where Q is -N(R)₂, and n is selected from 1, 2, 3, 4, and 5.

In certain embodiments, the disclosure provides a compound having the Formula (I), wherein R₂ and R₃ are independently selected from the group consisting of C₂₋₁₄ alkyl, C₂₋₁₄ alkenyl, -R*YR", -YR", and -R*OR", or R₂ and R₃, together with the atom to which they are attached, form a heterocycle or carbocycle, and R₄ is -(CH₂)ₙQ or -(CH₂)ₙCHQR, where Q is -N(R)₂, and n is selected from 3, 4, and 5.

In certain embodiments, R₂ and R₃ are independently selected from the group consisting of C₂₋₁₄ alkyl, C₂₋₁₄ alkenyl, -R*YR", -YR", and -R*OR", or R₂ and R₃, together with the atom to which they are attached, form a heterocycle or carbocycle.

In some embodiments, R₁ is selected from the group consisting of C₅₋₂₀ alkyl and C₅₋₂₀ alkenyl.

In other embodiments, R₁ is selected from the group consisting of -R*YR", -YR", and -R"M'R'.

In certain embodiments, R₁ is selected from -R*YR" and -YR". In some embodiments, Y is a cyclopropyl group. In some embodiments, R* is C₈ alkyl or C₈ alkenyl. In certain embodiments, R" is C₃₋₁₂ alkyl. For example, R" can be C₃ alkyl. For example, R" can be C₄₋₈ alkyl (e.g., C₄, C₅, C₆, C₇, or C₈ alkyl).

In some embodiments, R₁ is C₅₋₂₀ alkyl. In some embodiments, R₁ is C₆ alkyl. In some embodiments, R₁ is C₈ alkyl. In other embodiments, R₁ is C₉ alkyl. In certain embodiments, R₁ is C₁₄ alkyl. In other embodiments, R₁ is C₁₈ alkyl.

In some embodiments, R₁ is C₅₋₂₀ alkenyl. In certain embodiments, R₁ is C₁₈ alkenyl. In some embodiments, R₁ is linoleyl.

In certain embodiments, R₁ is branched (e.g., decan-2-yl, undecan-3-yl, dodecan-4-yl, tridecan-5-yl, tetradecan-6-yl, 2-methylundecan-3-yl, 2-methyldecan-2-yl, 3-methylundecan-3-yl, 4-methyldodecan-4-yl, or heptadeca-9-yl). In certain embodiments, R₁ is

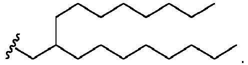

In certain embodiments, R₁ is unsubstituted C₅₋₂₀ alkyl or C₅₋₂₀ alkenyl. In certain embodiments, R' is substituted C₅₋₂₀ alkyl or C₅₋₂₀ alkenyl (e.g., substituted with a C₃₋₆ carbocycle such as 1-cyclopropylnonyl).

In other embodiments, R₁ is -R'M'R'.

In some embodiments, R' is selected from -R*YR" and -YR". In some embodiments, Y is C₃₋₈ cycloalkyl. In some embodiments, Y is C₆₋₁₀ aryl. In some embodiments, Y is a cyclopropyl group. In some embodiments, Y is a cyclohexyl group. In certain embodiments, R* is C₁ alkyl.

In some embodiments, R" is selected from the group consisting of C₃₋₁₂ alkyl and C₃₋₁₂ alkenyl. In some embodiments, R" adjacent to Y is C₁ alkyl. In some embodiments, R" adjacent to Y is C₄₋₉ alkyl (e.g., C₄, C₅, C₆, C₇ or C₈ or C₉ alkyl).

In some embodiments, R' is selected from C₄ alkyl and C₄ alkenyl. In certain embodiments, R' is selected from C₅ alkyl and C₅ alkenyl. In some embodiments, R' is selected from C₆ alkyl and C₆ alkenyl. In some embodiments, R' is selected from C₇ alkyl and C₇ alkenyl. In some embodiments, R' is selected from C₉ alkyl and C₉ alkenyl.

In other embodiments, R' is selected from C₁₁ alkyl and C₁₁ alkenyl. In other embodiments, R' is selected from C₁₂ alkyl, C₁₂ alkenyl, C₁₃ alkyl, C₁₃ alkenyl, C₁₄ alkyl, C₁₄ alkenyl, C₁₅ alkyl, C₁₅ alkenyl, C₁₆ alkyl, C₁₆ alkenyl, C₁₇ alkyl, C₁₇ alkenyl, C₁₈ alkyl, and C₁₈ alkenyl. In certain embodiments, R' is branched (e.g., decan-2-yl, undecan-3-yl, dodecan-4-yl, tridecan-5-yl, tetradecan-6-yl, 2-methylundecan-3-yl, 2-methyldecan-2-yl, 3-methylundecan-3-yl, 4-methyldodecan-4-yl or heptadeca-9-yl). In certain embodiments, R' is

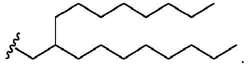

In certain embodiments, R' is unsubstituted C₁₋₁₈ alkyl. In certain embodiments, R' is substituted C₁₋₁₈ alkyl (e.g., C₁₋₁₅ alkyl substituted with a C₃₋₆ carbocycle such as 1-cyclopropylnonyl).

In some embodiments, R" is selected from the group consisting of C₃₋₁₄ alkyl and C₃₋₁₄ alkenyl. In some embodiments, R" is C₃ alkyl, C₄ alkyl, C₅ alkyl, C₆ alkyl, C₇ alkyl, or C₈ alkyl. In some embodiments, R" is C₉ alkyl, C₁₀ alkyl, C₁₁ alkyl, C₁₂ alkyl, C₁₃ alkyl, or C₁₄ alkyl.

In some embodiments, M' is -C(O)O-. In some embodiments, M' is -OC(O)-.

In other embodiments, M' is an aryl group or heteroaryl group. For example, M' can be selected from the group consisting of phenyl, oxazole, and thiazole.

In some embodiments, M is -C(O)O- In some embodiments, M is -OC(O)-. In some embodiments, M is -C(O)N(R')-. In some embodiments, M is -P(O)(OR')O-.

In other embodiments, M is an aryl group or heteroaryl group. For example, M can be selected from the group consisting of phenyl, oxazole, and thiazole.

In some embodiments, M is the same as M'. In other embodiments, M is different from M'.

In some embodiments, each R₅ is H. In certain such embodiments, each R₆ is also H.

In some embodiments, R₇ is H. In other embodiments, R₇ is C₁₋₃ alkyl (e.g., methyl, ethyl, propyl, or i-propyl).

In some embodiments, R₂ and R₃ are independently C₅₋₁₄ alkyl or C₅₋₁₄ alkenyl.

In some embodiments, R₂ and R₃ are the same. In some embodiments, R₂ and R₃ are C₈ alkyl. In certain embodiments, R₂ and R₃ are C₂ alkyl. In other embodiments, R₂ and R₃ are C₃ alkyl. In some embodiments, R₂ and R₃ are C₄ alkyl. In certain embodiments, R₂ and R₃ are C₅ alkyl. In other embodiments, R₂ and R₃ are C₆ alkyl. In some embodiments, R₂ and R₃ are C₇ alkyl.

In other embodiments, R₂ and R₃ are different. In certain embodiments, R₂ is C₈ alkyl. In some embodiments, R₃ is C₁₋₇ (e.g., C₁, C₂, C₃, C₄, C₅, C₆, or C₇ alkyl) or C₉ alkyl.

In some embodiments, R₇ and R₃ are H.

In certain embodiments, R₂ is H.

In some embodiments, m is 5, 7, or 9.

In some embodiments, R₄ is selected from -(CH₂)ₙQ and -(CH₂)ₙCHQR.

In some embodiments, Q is selected from the group consisting of -OR, -OH, -O(CH₂)ₙN(R)₂, -OC(O)R, -CX₃, -CN, -N(R)C(O)R, -N(H)C(O)R, -N(R)S(O)₂R, -N(H)S(O)₂R, -N(R)C(O)N(R)₂, -N(H)C(O)N(R)₂, -N(H)C(O)N(H)(R), -N(R)C(S)N(R)₂, -N(H)C(S)N(R)₂, -N(H)C(S)N(H)(R), -C(R)N(R)₂C(O)OR, a carbocycle, and a heterocycle.

In certain embodiments, Q is -OH.

In certain embodiments, Q is a substituted or unsubstituted 5- to 10- membered heteroaryl, e.g., Q is an imidazole, a pyrimidine, a purine, 2-amino-1,9-dihydro-6H-purin-6-one-9-yl (or guanin-9-yl), adenin-9-yl, cytosin-1-yl, or uracil-1-yl. In certain embodiments, Q is a substituted 5- to 14-membered heterocycloalkyl, e.g., substituted with one or more substituents selected from oxo (=O), OH, amino, and C₁₋₃ alkyl. For example, Q is 4-methylpiperazinyl, 4-(4-methoxybenzyl)piperazinyl, or isoindolin-2-yl-1,3-dione.

In certain embodiments, Q is an unsubstituted or substituted C₆₋₁₀ aryl (such as phenyl) or C₃₋₆ cycloalkyl.

In some embodiments, n is 1. In other embodiments, n is 2. In further embodiments, n is 3. In certain other embodiments, n is 4. For example, R₄ can be -(CH₂)₂OH. For example, R₄ can be -(CH₂)₃OH. For example, R₄ can be -(CH₂)₄OH. For example, R₄ can be benzyl. For example, R₄ can be 4-methoxybenzyl.

In some embodiments, R₄ is a C₃₋₆ carbocycle. In some embodiments, R₄ is a C₃₋₆ cycloalkyl. For example, R₄ can be cyclohexyl optionally substituted with e.g., OH, halo, C₁₋₆ alkyl, etc. For example, R₄ can be 2-hydroxycyclohexyl.

In some embodiments, R is H.

In some embodiments, R is unsubstituted C₁₋₃ alkyl or unsubstituted C₂₋₃ alkenyl. For example, R₄ can be -CH₂CH(OH)CH₃ or -CH₂CH(OH)CH₂CH₃.

In some embodiments, R is substituted C₁₋₃ alkyl, e.g., CH₂OH. For example, R₄ can be -CH₂CH(OH)CH₂OH.

In some embodiments, R₂ and R₃, together with the atom to which they are attached, form a heterocycle or carbocycle. In some embodiments, R₂ and R₃, together with the atom to which they are attached, form a 5- to 14-membered aromatic or non-aromatic heterocycle having one or more heteroatoms selected from N, O, S, and P. In some embodiments, R₂ and R₃, together with the atom to which they are attached, form an optionally substituted C₃₋₂₀ carbocycle (e.g., C₃₋₁₈ carbocycle, C₃₋₁₅ carbocycle, C₃₋₁₂ carbocycle, or C₃₋₁₀ carbocycle), either aromatic or non-aromatic. In some embodiments, R₂ and R₃, together with the atom to which they are attached, form a C₃₋₆ carbocycle. In other embodiments, R₂ and R₃, together with the atom to which they are attached, form a C₆ carbocycle, such as a cyclohexyl or phenyl group. In certain embodiments, the heterocycle or C₃₋₆ carbocycle is substituted with one or more alkyl groups (e.g., at the same ring atom or at adjacent or non-adjacent ring atoms). For example, R₂ and R₃, together with the atom to which they are attached, can form a cyclohexyl or phenyl group bearing one or more C₅ alkyl substitutions. In certain embodiments, the heterocycle or C₃₋₆ carbocycle formed by R₂ and R₃, is substituted with a carbocycle groups. For example, R₂ and R₃, together with the atom to which they are attached, can form a cyclohexyl or phenyl group that is substituted with cyclohexyl. In some embodiments, R₂ and R₃, together with the atom to which they are attached, form a C₇₋₁₅ carbocycle, such as a cycloheptyl, cyclopentadecanyl, or naphthyl group.

In some embodiments, R₄ is selected from -(CH₂)ₙQ and -(CH₂)ₙCHQR. In some embodiments, Q is selected from the group consisting of -OR, -OH, -O(CH₂)ₙN(R)₂, -OC(O)R, -CX₃, -CN, -N(R)C(O)R, -N(H)C(O)R, -N(R)S(O)₂R, -N(H)S(O)₂R, -N(R)C(O)N(R)₂, -N(H)C(O)N(R)₂, -N(H)C(O)N(H)(R), -N(R)C(S)N(R)₂, -N(H)C(S)N(R)₂, -N(H)C(S)N(H)(R), and a heterocycle. In other embodiments, Q is selected from the group consisting of an imidazole, a pyrimidine, and a purine.

In some embodiments, R₂ and R₃, together with the atom to which they are attached, form a heterocycle or carbocycle. In some embodiments, R₂ and R₃, together with the atom to which they are attached, form a C₃₋₆ carbocycle, such as a phenyl group. In certain embodiments, the heterocycle or C₃₋₆ carbocycle is substituted with one or more alkyl groups (e.g., at the same ring atom or at adjacent or non-adjacent ring atoms). For example, R₂ and R₃, together with the atom to which they are attached, can form a phenyl group bearing one or more C₅ alkyl substitutions.

In some embodiments, the LNP has an ionizable amino lipid selected from any of Compounds 1- 232 disclosed in PCT publication WO/2017/049245 published on March 23, 2017 and salts or stereoisomers thereof.

Ionizable lipids can be selected from the non-limiting group consisting of
3-(didodecylamino)-N1,N1,4-tridodecyl-1-piperazineethanamine (KL10), N1-[2-(didodecylamino)ethyl]-N1,N4,N4-tridodecyl-1,4-piperazinediethanamine (KL22), 14,25-ditridecyl-15,18,21,24-tetraaza-octatriacontane (KL25),
1,2-dilinoleyloxy-N,N-dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl 4-(dimethylamino)butanoate (DLin-MC3-DMA),
2,2-dilinoleyl-4-(2-dimethylaminoethyl)-[1,3]-dioxolane (DLin-KC2-DMA), 1,2-dioleyloxy-N,N-dimethylaminopropane (DODMA), (13Z,165Z)-N,N-dimethyl-3-nonydocosa-13-16-dien-1-amine (L608),
2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA),
(2R)-2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA (2R)), and
(2S)-2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA (2S)). In addition to these, an ionizable amino lipid can also be a lipid including a cyclic amine group.

The lipid composition of the pharmaceutical composition disclosed herein can comprise one or more phospholipids, for example, one or more saturated or (poly)unsaturated phospholipids or a combination thereof. In general, phospholipids comprise a phospholipid moiety and one or more fatty acid moieties.

A phospholipid moiety can be selected, for example, from the non-limiting group consisting of phosphatidyl choline, phosphatidyl ethanolamine, phosphatidyl glycerol, phosphatidyl serine, phosphatidic acid, 2-lysophosphatidyl choline, and a sphingomyelin.

A fatty acid moiety can be selected, for example, from the non-limiting group consisting of lauric acid, myristic acid, myristoleic acid, palmitic acid, palmitoleic acid, stearic acid, oleic acid, linoleic acid, alpha-linolenic acid, erucic acid, phytanoic acid, arachidic acid, arachidonic acid, eicosapentaenoic acid, behenic acid, docosapentaenoic acid, and docosahexaenoic acid.

Particular phospholipids can facilitate fusion to a membrane. For example, a cationic phospholipid can interact with one or more negatively charged phospholipids of a membrane (e.g., a cellular or intracellular membrane). Fusion of a phospholipid to a membrane can allow one or more elements (e.g., a therapeutic agent) of a lipid-containing composition (e.g., LNPs) to pass through the membrane permitting, e.g., delivery of the one or more elements to a target tissue.

Non-natural phospholipid species including natural species with modifications and substitutions including branching, oxidation, cyclization, and alkynes are also contemplated. For example, a phospholipid can be functionalized with or cross-linked to one or more alkynes (e.g., an alkenyl group in which one or more double bonds is replaced with a triple bond). Under appropriate reaction conditions, an alkyne group can undergo a copper-catalyzed cycloaddition upon exposure to an azide. Such reactions can be useful in functionalizing a lipid bilayer of a nanoparticle composition to facilitate membrane permeation or cellular recognition or in conjugating a nanoparticle composition to a useful component such as a targeting or imaging moiety (e.g., a dye).

Phospholipids include, but are not limited to, glycerophospholipids such as phosphatidylcholines, phosphatidylethanolamines, phosphatidylserines, phosphatidylinositols, phosphatidy glycerols, and phosphatidic acids. Phospholipids also include phosphosphingolipid, such as sphingomyelin.

In certain embodiments, a phospholipid useful or potentially useful in the present disclosure is an analog or variant of DSPC.

In certain embodiments, a phospholipid useful or potentially useful in the present disclosure comprises a modified phospholipid head (e.g., a modified choline group). In certain embodiments, a phospholipid with a modified head is DSPC, or analog thereof, with a modified quaternary amine.

In certain embodiments, a phospholipid useful or potentially useful in the present disclosure comprises a modified tail. In certain embodiments, a phospholipid useful or potentially useful in the present disclosure is DSPC, or analog thereof, with a modified tail. As described herein, a "modified tail" may be a tail with shorter or longer aliphatic chains, aliphatic chains with branching introduced, aliphatic chains with substituents introduced, aliphatic chains wherein one or more methylenes are replaced by cyclic or heteroatom groups, or any combination thereof.

In certain embodiments, an alternative lipid is used in place of a phospholipid of the disclosure.

The LNPs disclosed herein can comprise one or more structural lipids. As used herein, the term "structural lipid" refers to sterols and also to lipids containing sterol moieties.

Incorporation of structural lipids in the lipid nanoparticle may help mitigate aggregation of other lipids in the particle. Structural lipids can be selected from the group including but not limited to, cholesterol, fecosterol, sitosterol, ergosterol, campesterol, stigmasterol, brassicasterol, tomatidine, tomatine, ursolic acid, alpha-tocopherol, hopanoids, phytosterols, steroids, and mixtures thereof. In some embodiments, the structural lipid is a sterol. As defined herein, "sterols" are a subgroup of steroids consisting of steroid alcohols. In certain embodiments, the structural lipid is a steroid. In certain embodiments, the structural lipid is cholesterol. In certain embodiments, the structural lipid is an analog of cholesterol. In certain embodiments, the structural lipid is alpha-tocopherol.

In one embodiment, the amount of the structural lipid (e.g., an sterol such as cholesterol) in the lipid composition of a pharmaceutical composition disclosed herein ranges from about 20 mol % to about 60 mol %, from about 25 mol % to about 55 mol %, from about 30 mol % to about 50 mol %, or from about 35 mol % to about 45 mol %.

In one embodiment, the amount of the structural lipid (e.g., an sterol such as cholesterol) in the lipid composition disclosed herein ranges from about 25 mol % to about 30 mol %, from about 30 mol % to about 35 mol %, or from about 35 mol % to about 40 mol %.

In one embodiment, the amount of the structural lipid (e.g., a sterol such as cholesterol) in the lipid composition disclosed herein is about 24 mol %, about 29 mol %, about 34 mol %, or about 39 mol %.

In some embodiments, the amount of the structural lipid (e.g., an sterol such as cholesterol) in the lipid composition disclosed herein is at least about 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 mol %.

The lipid composition of a pharmaceutical composition disclosed herein can comprise one or more a polyethylene glycol (PEG) lipid.

As used herein, the term "PEG-lipid" refers to polyethylene glycol (PEG)-modified lipids. Non-limiting examples of PEG-lipids include PEG-modified phosphatidylethanolamine and phosphatidic acid, PEG-ceramide conjugates (e.g., PEG-CerC14 or PEG-CerC20), PEG-modified dialkylamines and PEG-modified 1,2-diacyloxypropan-3-amines. Such lipids are also referred to as PEGylated lipids. For example, a PEG lipid can be PEG-c-DOMG, PEG-DMG, PEG-DLPE, PEG-DMPE, PEG-DPPC, or a PEG-DSPE lipid.

In some embodiments, the PEG-lipid includes, but not limited to 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-disteryl glycerol (PEG-DSG), PEG-dipalmetoleyl, PEG-dioleyl, PEG-distearyl, PEG-diacylglycamide (PEG-DAG), PEG-dipalmitoyl phosphatidylethanolamine (PEG-DPPE), or PEG-I,2-dimyristyloxlpropyl-3-amine (PEG-c-DMA).

In one embodiment, the PEG-lipid is selected from the group consisting of a PEG-modified phosphatidylethanolamine, a PEG-modified phosphatidic acid, a PEG-modified ceramide, a PEG-modified dialkylamine, a PEG-modified diacylglycerol, a PEG-modified dialkylglycerol, and mixtures thereof.

In some embodiments, the lipid moiety of the PEG-lipids includes those having lengths of from about C₁₄ to about C₂₂, preferably from about C₁₄ to about C₁₆. In some embodiments, a PEG moiety, for example an mPEG-NH₂, has a size of about 1000, 2000, 5000, 10,000, 15,000 or 20,000 daltons. In one embodiment, the PEG-lipid is PEG₂ₖ-DMG.

In one embodiment, the lipid nanoparticles described herein can comprise a PEG lipid which is a non-diffusible PEG. Non-limiting examples of non-diffusible PEGs include PEG-DSG and PEG-DSPE.

PEG-lipids are known in the art, such as those described in U.S. Patent No. 8158601 and International Publ. No. WO 2015/130584 A2.

In one embodiment, PEG lipids useful in the present disclosure can be PEGylated lipids described in International Publication No. WO2012/099755. Any of these exemplary PEG lipids described herein may be modified to comprise a hydroxyl group on the PEG chain. In certain embodiments, the PEG lipid is a PEG-OH lipid. As generally defined herein, a "PEG-OH lipid" (also referred to herein as "hydroxy-PEGylated lipid") is a PEGylated lipid having one or more hydroxyl (-OH) groups on the lipid. In certain embodiments, the PEG-OH lipid includes one or more hydroxyl groups on the PEG chain. In certain embodiments, a PEG-OH or hydroxy-PEGylated lipid comprises an -OH group at the terminus of the PEG chain. Each possibility represents a separate embodiment of the present disclosure.

In one embodiment, the amount of PEG-lipid in the lipid composition of a pharmaceutical composition disclosed herein ranges from about 0.1 mol % to about 5 mol %, from about 0.5 mol % to about 5 mol %, from about 1 mol % to about 5 mol %, from about 1.5 mol % to about 5 mol %, from about 2 mol % to about 5 mol %, from about 0.1 mol % to about 4 mol %, from about 0.5 mol % to about 4 mol %, from about 1 mol % to about 4 mol %, from about 1.5 mol % to about 4 mol %, from about 2 mol % to about 4 mol %, from about 0.1 mol % to about 3 mol %, from about 0.5 mol % to about 3 mol %, from about 1 mol % to about 3 mol %, from about 1.5 mol % to about 3 mol %, from about 2 mol % to about 3 mol %, from about 0.1 mol % to about 2 mol %, from about 0.5 mol % to about 2 mol %, from about 1 mol % to about 2 mol %, from about 1.5 mol % to about 2 mol %, from about 0.1 mol % to about 1.5 mol %, from about 0.5 mol % to about 1.5 mol %, or from about 1 mol % to about 1.5 mol %.

In one embodiment, the amount of PEG-lipid in the lipid composition disclosed herein is about 2 mol %. In one embodiment, the amount of PEG-lipid in the lipid composition disclosed herein is about 1.5 mol %.

In one embodiment, the amount of PEG-lipid in the lipid composition disclosed herein is at least about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5 mol %.

In some aspects, the lipid composition of the pharmaceutical compositions disclosed herein does not comprise a PEG-lipid.

The lipid composition of a pharmaceutical composition disclosed herein can include one or more components in addition to those described above. For example, the lipid composition can include one or more permeability enhancer molecules, carbohydrates, polymers, surface altering agents (e.g., surfactants), or other components. For example, a permeability enhancer molecule can be a molecule described by U.S. Patent Application Publication No. 2005/0222064. Carbohydrates can include simple sugars (e.g., glucose) and polysaccharides (e.g., glycogen and derivatives and analogs thereof).

A polymer can be included in and/or used to encapsulate or partially encapsulate a pharmaceutical composition disclosed herein (e.g., a pharmaceutical composition in lipid nanoparticle form). A polymer can be biodegradable and/or biocompatible. A polymer can be selected from, but is not limited to, polyamines, polyethers, polyamides, polyesters, polycarbamates, polyureas, polycarbonates, polystyrenes, polyimides, polysulfones, polyurethanes, polyacetylenes, polyethylenes, polyethyleneimines, polyisocyanates, polyacrylates, polymethacrylates, polyacrylonitriles, and polyarylates.

The ratio between the lipid composition and the polynucleotide range can be from about 10:1 to about 60:1 (wt/wt).

In some embodiments, the ratio between the lipid composition and the polynucleotide can be about 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, 50:1, 51:1, 52:1, 53:1, 54:1, 55:1, 56:1, 57:1, 58:1, 59:1 or 60:1 (wt/wt). In some embodiments, the wt/wt ratio of the lipid composition to the polynucleotide encoding a therapeutic agent is about 20:1 or about 15:1.

In one embodiment, the lipid nanoparticles described herein can comprise polynucleotides (e.g., mRNA) in a lipid:polynucleotide weight ratio of 5:1, 10:1, 15:1, 20:1, 25:1, 30:1, 35:1, 40:1, 45:1, 50:1, 55:1, 60:1 or 70:1, or a range or any of these ratios such as, but not limited to, 5:1 to about 10:1, from about 5:1 to about 15:1, from about 5:1 to about 20:1, from about 5:1 to about 25:1, from about 5:1 to about 30:1, from about 5:1 to about 35:1, from about 5:1 to about 40:1, from about 5:1 to about 45:1, from about 5:1 to about 50:1, from about 5:1 to about 55:1, from about 5:1 to about 60:1, from about 5:1 to about 70:1, from about 10:1 to about 15:1, from about 10:1 to about 20:1, from about 10:1 to about 25:1, from about 10:1 to about 30:1, from about 10:1 to about 35:1, from about 10:1 to about 40:1, from about 10:1 to about 45:1, from about 10:1 to about 50:1, from about 10:1 to about 55:1, from about 10:1 to about 60:1, from about 10:1 to about 70:1, from about 15:1 to about 20:1, from about 15:1 to about 25:1 ,from about 15:1 to about 30:1, from about 15:1 to about 35:1, from about 15:1 to about 40:1, from about 15:1 to about 45:1, from about 15:1 to about 50:1, from about 15:1 to about 55:1, from about 15:1 to about 60:1 or from about 15:1 to about 70:1.

In one embodiment, the lipid nanoparticles described herein can comprise the polynucleotide in a concentration from approximately 0.1 mg/ml to 2 mg/ml such as, but not limited to, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1.0 mg/ml, 1.1 mg/ml, 1.2 mg/ml, 1.3 mg/ml, 1.4 mg/ml, 1.5 mg/ml, 1.6 mg/ml, 1.7 mg/ml, 1.8 mg/ml, 1.9 mg/ml, 2.0 mg/ml or greater than 2.0 mg/ml.

In some embodiments, the pharmaceutical compositions disclosed herein are formulated as lipid nanoparticles (LNP). Accordingly, the present disclosure also provides nanoparticle compositions comprising (i) a lipid composition comprising a delivery agent such as a compound of Formula (I) or (iii) as described herein, and (ii) a polynucleotide encoding an antigen polypeptide. In such nanoparticle composition, the lipid composition disclosed herein can encapsulate the polynucleotide encoding an antigen polypeptide.

Nanoparticle compositions are typically sized on the order of micrometers or smaller and can include a lipid bilayer. Nanoparticle compositions encompass lipid nanoparticles (LNPs), liposomes (e.g., lipid vesicles), and lipoplexes. For example, a nanoparticle composition can be a liposome having a lipid bilayer with a diameter of 500 nm or less.

Nanoparticle compositions include, for example, lipid nanoparticles (LNPs), liposomes, and lipoplexes. In some embodiments, nanoparticle compositions are vesicles including one or more lipid bilayers. In certain embodiments, a nanoparticle composition includes two or more concentric bilayers separated by aqueous compartments. Lipid bilayers can be functionalized and/or crosslinked to one another. Lipid bilayers can include one or more ligands, proteins, or channels.

In one embodiment, a lipid nanoparticle comprises an ionizable lipid, a structural lipid, a phospholipid, and mRNA. In some embodiments, the LNP comprises an ionizable lipid, a PEG-modified lipid, a phospholipid and a structural lipid. In some embodiments, the LNP has a molar ratio of about 20-60% ionizable lipid: about 5-25% phospholipid: about 25-55% structural lipid; and about 0.5-15% PEG-modified lipid. In some embodiments, the LNP comprises a molar ratio of about 50% ionizable lipid, about 1.5% PEG-modified lipid, about 38.5% structural lipid and about 10% phospholipid. In some embodiments, the LNP comprises a molar ratio of about 55% ionizable lipid, about 2.5% PEG lipid, about 32.5% structural lipid and about 10% phospholipid. In some embodiments, the ionizable lipid is an ionizable amino lipid and the phospholipid is a neutral lipid, and the structural lipid is a cholesterol. In some embodiments, the LNP has a molar ratio of 50:38.5:10:1.5 of ionizable lipid: cholesterol: DSPC: PEG lipid.

In some embodiments, the LNP has a polydispersity value of less than 0.4. In some embodiments, the LNP has a net neutral charge at a neutral pH. In some embodiments, the LNP has a mean diameter of 50-150 nm. In some embodiments, the LNP has a mean diameter of 80-100 nm.

As generally defined herein, the term "lipid" refers to a small molecule that has hydrophobic or amphiphilic properties. Lipids may be naturally occurring or synthetic. Examples of classes of lipids include, but are not limited to, fats, waxes, sterol-containing metabolites, vitamins, fatty acids, glycerolipids, glycerophospholipids, sphingolipids, saccharolipids, and polyketides, and prenol lipids. In some instances, the amphiphilic properties of some lipids lead them to form liposomes, vesicles, or membranes in aqueous media.

In some embodiments, a lipid nanoparticle (LNP) may comprise an ionizable lipid. As used herein, the term "ionizable lipid" has its ordinary meaning in the art and may refer to a lipid comprising one or more charged moieties. In some embodiments, an ionizable lipid may be positively charged or negatively charged. An ionizable lipid may be positively charged, in which case it can be referred to as "cationic lipid". In certain embodiments, an ionizable lipid molecule may comprise an amine group, and can be referred to as an ionizable amino lipids. As used herein, a "charged moiety" is a chemical moiety that carries a formal electronic charge, e.g., monovalent (+1, or -1), divalent (+2, or -2), trivalent (+3, or -3), etc. The charged moiety may be anionic (i.e., negatively charged) or cationic (i.e., positively charged). Examples of positively-charged moieties include amine groups (e.g., primary, secondary, and/or tertiary amines), ammonium groups, pyridinium group, guanidine groups, and imidizolium groups. In a particular embodiment, the charged moieties comprise amine groups. Examples of negatively- charged groups or precursors thereof, include carboxylate groups, sulfonate groups, sulfate groups, phosphonate groups, phosphate groups, hydroxyl groups, and the like. The charge of the charged moiety may vary, in some cases, with the environmental conditions, for example, changes in pH may alter the charge of the moiety, and/or cause the moiety to become charged or uncharged. In general, the charge density of the molecule may be selected as desired.

It should be understood that the terms "charged" or "charged moiety" does not refer to a "partial negative charge" or "partial positive charge" on a molecule. The terms "partial negative charge" and "partial positive charge" are given their ordinary meaning in the art. A "partial negative charge" may result when a functional group comprises a bond that becomes polarized such that electron density is pulled toward one atom of the bond, creating a partial negative charge on the atom. Those of ordinary skill in the art will, in general, recognize bonds that can become polarized in this way.

In some embodiments, the ionizable lipid is an ionizable amino lipid, sometimes referred to in the art as an "ionizable cationic lipid". In one embodiment, the ionizable amino lipid may have a positively charged hydrophilic head and a hydrophobic tail that are connected via a linker structure.

In addition to these, an ionizable lipid may also be a lipid including a cyclic amine group.

In one embodiment, the ionizable lipid may be selected from, but not limited to, an ionizable lipid described in International Publication Nos. WO2013/086354 and WO2013/116126.

In one embodiment, the lipid may be a cleavable lipid such as those described in International Publication No. WO2012/170889. In one embodiment, the lipid may be synthesized by methods known in the art and/or as described in International Publication Nos. WO2013086354.

Nanoparticle compositions can be characterized by a variety of methods. For example, microscopy (e.g., transmission electron microscopy or scanning electron microscopy) can be used to examine the morphology and size distribution of a nanoparticle composition. Dynamic light scattering or potentiometry (e.g., potentiometric titrations) can be used to measure zeta potentials. Dynamic light scattering can also be utilized to determine particle sizes. Instruments such as the Zetasizer Nano ZS (Malvern Instruments Ltd, Malvern, Worcestershire, UK) can also be used to measure multiple characteristics of a nanoparticle composition, such as particle size, polydispersity index, and zeta potential.

Nanoparticle compositions can be characterized by a variety of methods. For example, microscopy (e.g., transmission electron microscopy or scanning electron microscopy) can be used to examine the morphology and size distribution of a nanoparticle composition. Dynamic light scattering or potentiometry (e.g., potentiometric titrations) can be used to measure zeta potentials. Dynamic light scattering can also be utilized to determine particle sizes. Instruments such as the Zetasizer Nano ZS (Malvern Instruments Ltd, Malvern, Worcestershire, UK) can also be used to measure multiple characteristics of a nanoparticle composition, such as particle size, polydispersity index, and zeta potential.

The size of the nanoparticles can help counter biological reactions such as, but not limited to, inflammation, or can increase the biological effect of the polynucleotide.

As used herein, "size" or "mean size" in the context of nanoparticle compositions refers to the mean diameter of a nanoparticle composition.

In one embodiment, the polynucleotide encoding an antigen polypeptide are formulated in lipid nanoparticles having a diameter from about 10 to about 100 nm such as, but not limited to, about 10 to about 20 nm, about 10 to about 30 nm, about 10 to about 40 nm, about 10 to about 50 nm, about 10 to about 60 nm, about 10 to about 70 nm, about 10 to about 80 nm, about 10 to about 90 nm, about 20 to about 30 nm, about 20 to about 40 nm, about 20 to about 50 nm, about 20 to about 60 nm, about 20 to about 70 nm, about 20 to about 80 nm, about 20 to about 90 nm, about 20 to about 100 nm, about 30 to about 40 nm, about 30 to about 50 nm, about 30 to about 60 nm, about 30 to about 70 nm, about 30 to about 80 nm, about 30 to about 90 nm, about 30 to about 100 nm, about 40 to about 50 nm, about 40 to about 60 nm, about 40 to about 70 nm, about 40 to about 80 nm, about 40 to about 90 nm, about 40 to about 100 nm, about 50 to about 60 nm, about 50 to about 70 nm, about 50 to about 80 nm, about 50 to about 90 nm, about 50 to about 100 nm, about 60 to about 70 nm, about 60 to about 80 nm, about 60 to about 90 nm, about 60 to about 100 nm, about 70 to about 80 nm, about 70 to about 90 nm, about 70 to about 100 nm, about 80 to about 90 nm, about 80 to about 100 nm and/or about 90 to about 100 nm.

In one embodiment, the nanoparticles have a diameter from about 10 to 500 nm. In one embodiment, the nanoparticle has a diameter greater than 100 nm, greater than 150 nm, greater than 200 nm, greater than 250 nm, greater than 300 nm, greater than 350 nm, greater than 400 nm, greater than 450 nm, greater than 500 nm, greater than 550 nm, greater than 600 nm, greater than 650 nm, greater than 700 nm, greater than 750 nm, greater than 800 nm, greater than 850 nm, greater than 900 nm, greater than 950 nm or greater than 1000 nm.

In some embodiments, the largest dimension of a nanoparticle composition is 1 µm or shorter (e.g., 1 µm, 900 nm, 800 nm, 700 nm, 600 nm, 500 nm, 400 nm, 300 nm, 200 nm, 175 nm, 150 nm, 125 nm, 100 nm, 75 nm, 50 nm, or shorter).

A nanoparticle composition can be relatively homogenous. A polydispersity index can be used to indicate the homogeneity of a nanoparticle composition, e.g., the particle size distribution of the nanoparticle composition. A small (e.g., less than 0.3) polydispersity index generally indicates a narrow particle size distribution. A nanoparticle composition can have a polydispersity index from about 0 to about 0.25, such as 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, or 0.25. In some embodiments, the polydispersity index of a nanoparticle composition disclosed herein can be from about 0.10 to about 0.20.

In addition to LNPs, the mRNA vaccine may be formulated in other carriers, as long as such carrier is determined to have the threshold differential T-cell activation potential. Known carriers, for instance, may be modified or further formulated to achieve the threshold values described herein. Other carriers include but are not limited to other non-LNP lipid based carriers such as liposomes, lipoids and lipoplexes, particulate or polymeric nanoparticles, peptide carriers, nanoparticle mimics, nanotubes, conjugates, or emulsion delivery systems such as cationic submicron oil-in-water emulsions.

Liposomes are amphiphilic lipids which can form bilayers in an aqueous environment to encapsulate a RNA-containing aqueous core. These lipids can have an anionic, cationic or zwitterionic hydrophilic head group. Liposomes can be formed from a single lipid or from a mixture of lipids. A mixture may comprise (i) a mixture of anionic lipids (ii) a mixture of cationic lipids (iii) a mixture of zwitterionic lipids (iv) a mixture of anionic lipids and cationic lipids (v) a mixture of anionic lipids and zwitterionic lipids (vi) a mixture of zwitterionic lipids and cationic lipids or (vii) a mixture of anionic lipids, cationic lipids and zwitterionic lipids. Similarly, a mixture may comprise both saturated and unsaturated lipids. Exemplary phospholipids include, but are not limited to, phosphatidylethanolamines, phosphatidylcholines, phosphatidylserines, and phosphatidylglycerols. Cationic lipids include, but are not limited to, dioleoyl trimethylammonium propane (DOTAP), 1,2-distearyloxy-N,N-dimethyl-3-aminopropane (DSDMA), 1,2-dioleyloxy-N,Ndimethyl-3-aminopropane (DODMA), 1,2-dilinoleyloxy-N,N-dimethyl-3-aminopropane (DLinDMA), 1,2-dilinolenyloxy-N,N-dimethyl-3-aminopropane (DLenDMA). Zwitterionic lipids include, but are not limited to, acyl zwitterionic lipids and ether zwitterionic lipids. Examples of useful zwitterionic lipids are DPPC, DOPC and dodecylphosphocholine. The lipids can be saturated or unsaturated.

Polymeric microparticles or nanoparticles can also be used to encapsulate or adsorb mRNA. The particles may be substantially non-toxic and biodegradable. The particles useful for delivering mRNA may have an optimal size and zeta potential. For instance, the microparticles may have a diameter in the range of 0.02 µm to 8 µm. In the instances when the composition has a population of micro- or nanoparticles with different diameters, at least 80%, 85%, 90%, or 95% of those particles ideally have diameters in the range of 0.03-7 µm. The particles may also have a zeta potential of between 40-100 mV, in order to provide maximal adsorption of the mRNA to the particles.

Non-toxic and biodegradable polymers include, but are not limited to, poly(ahydroxy acids), polyhydroxy butyric acids, polylactones (including polycaprolactones), polydioxanones, polyvalerolactone, polyorthoesters, polyanhydrides, polycyanoacrylates, tyrosine-derived polycarbonates, polyvinyl-pyrrolidinones or polyester-amides, and combinations thereof. In some embodiments, the particles are formed from poly(ahydroxy acids), such as a poly(lactides) ("PLA"), copolymers of lactide and glycolide such as a poly(D,L-lactide-co-glycolide) ("PLG"), and copolymers of D,L-lactide and caprolactone. Useful PLG polymers include those having a lactide/glycolide molar ratio ranging, for example, from 20:80 to 80:20 e.g. 25:75, 40:60, 45:55, 55:45, 60:40, 75:25. Useful PLG polymers include those having a molecular weight between, for example, 5,000-200,000 Da e.g. between 10,000-100,000, 20,000-70,000, 40,000-50,000 Da.

Oil-in-water emulsions may also be used for delivering mRNA to a subject. Examples of oils useful for making the emulsions include animal (e.g., fish) oil or vegetable oil (e.g. nuts, seeds and grains). The oil may be biodegradable (metabolizable) and biocompatible. Some exemplary oils include tocopherols and squalene, a shark liver oil which is a branched, unsaturated terpenoid and combinations thereof. Terpenoids are branched chain oils that are synthesized biochemically in 5-carbon isoprene units.

The aqueous component of the emulsion can be water or can be water in which additional components have been added. For instance, it may include salts to form a buffer e.g. citrate or phosphate salts, such as sodium salts. Exemplary buffers include: a phosphate buffer; a Tris buffer; a borate buffer; a succinate buffer; a histidine buffer; or a citrate buffer.

The oil-in water emulsions ideally include one or more cationic molecules. For instance, a cationic lipid can be included in the emulsion to provide a positively charged droplet surface to which negatively-charged mRNA can attach. Useful cationic lipids include, but are not limited to: 1,2-dioleoyloxy-3-(trimethylammonio)propane (DOTAP), 3'-[N--(N',N'-Dimethylaminoethane)-carbamoyl]Cholesterol (DC Cholesterol), dimethyldioctadecyl-ammonium (DDA e.g. the bromide), 1,2-Dimyristoyl-3-Trimethyl-AmmoniumPropane (DMTAP), dipalmitoyl(C16:0)trimethyl ammonium propane (DPTAP), distearoyltrimethylammonium propane (DSTAP). Other useful cationic lipids are: benzalkonium chloride (BAK), benzethonium chloride, cetramide (which contains tetradecyltrimethylammonium bromide and possibly small amounts of dedecyltrimethylammonium bromide and hexadecyltrimethyl ammonium bromide), cetylpyridinium chloride (CPC), cetyl trimethylammonium chloride (CTAC), N,N',N'-polyoxyethylene (10)-N-tallow-1,3-diaminopropane, dodecyltrimethylammonium bromide, hexadecyltrimethyl-ammonium bromide, mixed alkyl-trimethyl-ammonium bromide, benzyldimethyldodecylammonium chloride, benzyldimethylhexadecyl-ammonium chloride, benzyltrimethylammonium methoxide, cetyldimethylethylammonium bromide, dimethyldioctadecyl ammonium bromide (DDAB), methylbenzethonium chloride, decamethonium chloride, methyl mixed trialkyl ammonium chloride, methyl trioctylammonium chloride), N,N-dimethyl-N-[2 (2-methyl-4-(1,1,3,3tetramethylbutyl)-phenoxy]-ethoxy)ethyl]-benzenemetha- -naminium chloride (DEBDA), dialkyldimethylammonium salts, [1-(2,3-dioleyloxy)-propyl]-N,N,N,trimethylammonium chloride, 1,2-diacyl-3-(trimethylammonio) propane (acyl group=dimyristoyl, dipalmitoyl, distearoyl, dioleoyl), 1,2-diacyl-3 (dimethylammonio)propane (acyl group=dimyristoyl, dipalmitoyl, distearoyl, dioleoyl), 1,2-dioleoyl-3-(4'-trimethylammonio)butanoyl-sn-glycerol, 1,2-dioleoyl 3-succinyl-sn-glycerol choline ester, cholesteryl (4'-trimethylammonio) butanoate), N-alkyl pyridinium salts (e.g. cetylpyridinium bromide and cetylpyridinium chloride), N-alkylpiperidinium salts, dicationic bolaform electrolytes (C12Me6; C12BU6), dialkylglycetylphosphorylcholine, lysolecithin, L-.alpha.dioleoylphosphatidylethanolamine, cholesterol hemisuccinate choline ester, lipopolyamines, including but not limited to dioctadecylamidoglycylspermine (DOGS), dipalmitoyl phosphatidylethanol-amidospermine (DPPES), lipopoly-L (or D)-lysine (LPLL, LPDL), poly(L (or D)-lysine conjugated to N-glutarylphosphatidylethanolamine, didodecyl glutamate ester with pendant amino group (C GluPhCnN), ditetradecyl glutamate ester with pendant amino group (C14GluCnN+), cationic derivatives of cholesterol, including but not limited to cholesteryl-3.beta.-oxysuccinamidoethylenetrimethylammonium salt, cholesteryl-3.beta.-oxysuccinamidoethylene-dimethylamine, cholesteryl-3.beta.-carboxyamidoethylenetrimethylammonium salt, and cholesteryl-3.beta.-carboxyamidoethylenedimethylamine.

In addition to the oil and cationic lipid, an emulsion can include a non-ionic surfactant and/or a zwitterionic surfactant. Such surfactants include, but are not limited to: the polyoxyethylene sorbitan esters surfactants, especially polysorbate 20 and polysorbate 80; copolymers of ethylene oxide, propylene oxide, and/or butylene oxide, linear block copolymers; octoxynols; (octylphenoxy)polyethoxyethanol; phospholipids such as phosphatidylcholine; polyoxyethylene fatty ethers derived from lauryl, cetyl, stearyl and oleyl alcohols; polyoxyethylene-9-lauryl ether; and sorbitan esters.

The size of the emulsion particle may vary. In some embodiments the particles are in the range 20-750 nm, or for instance, 20-250 nm, 20- 200 nm, 20-150 nm.

### EXAMPLES

### Example 1 - mRNA Vaccines Efficiently Target and Activate APCs in Vivo

Vaccine-induced immunity induced by mRNA encoding full-length H10 formulated in LNP was first evaluated by the levels of neutralizing antibody titers to H10, i.e. hemagglutination inhibition (HAI) (FIG. 1A) and H10-specific CD4+ T cell responses (FIGs. 1B-D). Two groups received a prime and boost immunization by either I.M. or I.D. administration. Some animals in the I.D. group responded with detectable HAI titers after priming, albeit below the reported protective threshold (1:40) (Cox, Human Vaccine Immunotherapy 2013 9(2):405-8) (FIG. 1A). The HAI titers in all groups were significantly increased after the second immunization and remained above protective levels for the remainder for the study period (21 weeks). The titers were significantly higher in the I.D. group compared to the I.M. groups for two weeks after the boost but were thereafter at similar levels. To evaluate any potential enhancement by the addition of an adjuvant, a third group received I.M. administration of the LNP/H10 mRNA vaccine together with a TLR4-using glucopyranosyl lipid adjuvant (GLA). However, the addition of the adjuvant did not enhance the titers above the responses found in the other groups indicating that the mRNA/LNP formulation itself was immunogenic. The GLA group received a third immunization which resulted in a transient increase in HAI titers, which returned after three weeks to similar levels as the other groups.

Consistent with the highest HAI titers found in the I.D. group, the number of H10-specific CD4+T cells was also significantly higher in this group early after boost (FIG. 1C). Again, the addition of GLA to LNP/mRNA did not augment the responses. The polyfunctionality of the H10-specific CD4+ T cells producing IFNγ, IL-2 and TNF or a combination thereof was similar between the groups (FIG. 1D). Conclusively, LNP/mRNA delivered by the I.D. route induced transiently higher HAI titers and CD4+T cell responses directly after boost immunization but the responses were comparable at the later time points (>2 weeks) regardless of the route of administration or addition of the GLA adjuvant.

### A variety of immune cells were found to rapidly accumulate to the site of mRNA injection

As discussed hereinabove, details on the mechanisms of action by which LNP/mRNA-based vaccines generate strong vaccine-responses are largely lacking. Whether the distinct immunization routes lead to targeting of different immune cells and induction of distinct innate immune responses responsible for forming the vaccine-induced immunity is unexplored. The sequence of immune events culminating into vaccine-induced immunity is initiated by transient early local inflammation that ushers immune cells including APCs to the site of vaccine delivery. To analyze the cell infiltration after vaccine administration, rhesus macaques received I.M. or I.D. injections of mRNA encoding for the fluorescent protein mCitrine formulated in Atto655-labeled LNP. Uptake of LNP vs. translation of mRNA (mCitrine) could therefore be differentially detected. Injection of PBS or "empty" non-labeled LNP without mRNA cargo served as controls.

At 4 and 24 hours, biopsies from the sites of injection (skin or muscle) were analyzed for cell infiltration. Compared to the PBS-injection site, there was rapid recruitment of CD45+ immune cells to the LNP/mRNA-injected sites (FIGs. 2A-2B). The level of cell infiltration was higher at 24 hours compared to 4 hours. Cell infiltration was found regardless if the LNP contained mRNA or not indicating that LNP itself was capable of initiating immune cell recruitment. Multiple cell subsets were defined within the CD45+immune cells (FIGs.7A-7B). CD66abce+neutrophils and classical CD14+CD16- monocytes were the most frequent cell type infiltrating both the skin and muscle (FIGs. 2C-2D). Both skin and muscle also showed an increase in intermediate (CD14+CD16+) monocytes described as having efficient antigen uptake and the intermediate as well as non-classical (CD14-/dim CD16+) monocytes reported to produce inflammatory cytokines upon specific TLR stimulation (Cros, Immunity, 33(3):375-86 (2010); Geissmann et al., Immunity, 19(1):71-82 (2003); Lund et al., Frontiers in Immunology, 7: 322 (2016)). However, these monocyte subsets were detected at much lower levels than the classical monocytes. The large infiltration of neutrophils and monocytes at the vaccine delivery sites is in line with previous reports on mouse muscle receiving SAM vaccine in nanoemulsion (Brito, Molecular Therapy: J of ASGT, 22(12): 2118-29 (2014)) and adenoviral vector (Liang et al., J Immunological Methods, 425: 69-78 (2015)).

As for monocytes, DCs also consist of a heterogeneous group of cells; CD11c+ myeloid DCs (MDCs) including the CD1c+MDC subset that are potent inducers of CD4+T cell responses in addition to CD123+plasmacytoid DCs (PDCs) are efficient producers of type I IFNs (Jongbloed et al., J of Experimental Med., 207(6): 1247-60 (2010); Lore et al., J Immunology, 179(3): 1721-29 (2007)). The numbers of MDCs (CD1c+ and CD1c-) as well as PDCs were significantly elevated at both the muscle and skin vaccine injection sites (FIGs. 2E-2F). The skin harbors epidermal CD1a+ Langerhans cells and CD209 (DC-SIGN)+dermal DCs in addition to CD1a+dermal DCs and CD209+ macrophages (McGovern et al., Immunity, 41(3): 465-77 (2014)). The CD209+APCs were found to be especially elevated after LNP/mRNA administration (FIG. 2F).

### The cells accumulating at the injection sites and draining LNs in response to mRNA vaccines are associated with IFN responses as measured by upregulation of genes

Muscle and skin are different in their composition of tissue-specific cells and resident immune cells which may result in differential expression of genes associated with innate immune activation after vaccine exposure. Whether the robust cell infiltration with LNP/mRNA administration found with both I.D. and I.M. administration was accompanied with modulation of genes associated with innate immune activation was analyzed. Transcriptomic analyses of biopsies from the sites of injection (skin and muscle) as well as draining LNs collected at 24 hours after immunization showed that there was significant gene modulation both at the injection sites and LNs compared to the PBS controls. More genes were significantly (p<0.05) altered at the injection site in the LNP/mRNA-injected skin than in muscle compared to the respective PBS-injection control (FIG. 3A). Vice versa, more genes were significantly changed in muscle-draining LNs than in skin-draining LNs compared to the matching PBS-draining LNs (FIG. 3B). A total of 64 upregulated genes were selected and divided by their involvement in inflammation, migration or antigen uptake and presentation (FIGs. 3C-3D). The I.D. and I.M. groups modulated a common set of 50 and 34 genes with log₂ (fold change) ≥2 at the LNP/mRNA injection sites and draining LNs, respectively (FIG. 3E). This suggests that I.M. and I.D. delivery of LNP/mRNA to a large extent induces similar innate immune activation. This may help explain the similar H10-induced adaptive responses in the groups after the prime-boost immunization (FIGs. 1A-1D). However, a few genes were upregulated either in the I.M. or I.D. group including anti-microbial peptides, molecules for migration, inflammatory effectors and their receptors plus antigen acquisition and presentation (FIG. 3E).

Genes associated with inflammatory mediators e.g. IL-1β, MyD88, PTX3, NLRP3 were upregulated in the LNP/mRNA-injected sites and respective draining LNs in comparison to the PBS controls (FIGs. 3F-3G). Although many of them are downstream of various inflammatory pathways, there were a number of genes specifically connected to IFN responses including type I IFN-inducible MX1 (MxA). In addition, for genes associated with cell migration there was high expression of genes encoding the IFN-inducible chemokines CXCL10 (IP-10) and CXCL11 (I-TAC) especially in the skin of the I.D. group and at similar levels in the draining LNs of both groups. In fact, CXCL10 and CXCL11 were the highest upregulated IFN-inducible genes expressed at about 7-11 fold higher levels than the PBS control sites. While upregulation of the IFNα receptor 2 (IFNAR2) gene was detected in both after I.D. and I.M. administration, expression of the IFNα subtype 1/13 and CXCR3 (CXCL10/11 receptor) was increased mainly in the skin. Further, the LDL receptor that was previously shown to mediate ionizable LNP uptake in an ApoE-dependent manner (Akinc et al., Molecular Therapy: J of ASGT, 18(7): 1357-64 (2010); Pardi et al., J Controlled Release, 217: 345-51 (2015)) was upregulated in all animals although ApoE gene was downregulated at 24 hours post immunization (FIG. 3F). Genes mediating in antigen processing (Cathepsin L) and antigen loading (TAP2) were also upregulated along with the T cell co-stimulatory molecules CD80 and CD86 at both injection sites and draining LNs.

### Phenotypic differentiation of APCs by mRNA exposure at injection site and lymph nodes

A type I IFN response on the protein level evidenced by the upregulation of MxA was also observed at the injection sites and draining LNs after LNP/mRNA administration showed (FIG. 4A). MxA expression was exclusively induced by LNP/mRNA exposure since it was not detected in the PBS and empty LNP control sites. In line with the strong upregulation of the IFN-inducible CXCL10 gene at 24 hours after LNP/mRNA administration, the levels of CXCL10 (IP-10) in plasma were also elevated at this time (FIG. 4B). Protamine-complexed RNA and self-replicating RNA have been shown to induce IFNα in vitro (Rettig et al., Blood, 115(22): 4533-41 (2010); Chahal et al., PNAS, 113(29): e4133-42 (2016)) and RNA formulated in liposomes stimulated IFNα in vivo in mice in a TLR7 dependent manner (Kranz et al., Nature, 534(7607): 396-401 (2016); Pollard et al., Molecular Therapy: J of the ASGT, 21(1): 251-9 (2013)). The mRNA used in this study contains modified bases to avoid excessive immune activation mediated by TLR7 ligation. Since PDCs express high levels of TLR7 and are unique in their secretion of high levels of type I IFNs, they were exposedto LNP/mRNA in vitro. It was found that LNP/mRNA but not empty LNP induced low but detectable IFNα production in PDCs (FIG. 4C) but at lower levels compared to the synthetic TLR7/8 agonist R848.

LNP/mRNA may induce cellular activation directly but also in a bystander manner via type I IFNs as previously described (Montoya et al., Blood, 99(9): 3263-71 (2002)). Phenotypic differentiation of APCs was measured. Consistent with the upregulated CD80 gene expression in the injection sites and LNs, it was found that APCs at these sites exhibited upregulated CD80 expression compared to APCs from the donor-matched PBS injection sites (FIG. 4C). The CD14+ CD16+ and the CD14- CD16+ monocytes infiltrating the injection sites and the skin-draining LNs showed the highest upregulation of CD80 (FIG. 4D). In the I.M. group, CD1c+ MDCs were more efficient at upregulating CD80 than CD1c-DCs. CD1a+ APCs responded more strongly than CD209+ APCs both in the LNP/mRNA injected skin and its draining LNs. PDCs in both groups showed increased CD80 expression.

Interestingly, LNP alone, which was sufficient to induce immune cell infiltration to the injection sites (FIGs. 2A-2B), did not show marked CD80 upregulation (FIG. 4E). This suggests that the mRNA cargo is the main inducer of cellular activation in terms of phenotypic activation of APCs. It was confirmed that immune activation was not related to the specific protein encoded by the mRNA, as APCs upregulated CD80 or CD86 with similar efficiency in vitro when LNP contained mRNA encoding H10 or the fluorescent mCitrine protein (FIG. 4E).

### Uptake and translation of mRNA at the injection site and draining LNs in vivo after administration was determined

Next, the immune cells that were targeted by LNP/mRNA at the injection site and draining LNs were characterized. Previous mouse studies showed translation of administered RNA in organs or tissues by in vivo bioluminescence (Geall et al., PNAS, 109(36):14604-9 (2012); Kranz et al., Nature, 534(7607):396-401 (2016); Pardi et al., J Controlled Release, 217: 345-51 (2015); Kallen et al., Hum Vaccine Immunotherapy, 9(10): 2263-76 (2013)) or by histological staining (Brito et al., Molecular Therapy: J of the ASGT, 22(12): 2118-29 (2014); Probst et al., Gene Therapy, 14(15): 1175-80 (2007)). Although translated mRNA is detected by these approaches, they do not specify the infiltrating or tissue resident hematopoietic cells capable of vaccine mRNA translation. Tissue cells (e.g. myocytes, keratinocytes, fibroblasts) targeted by mRNA vaccines likely help create a local inflammation at the injection site, but they do not migrate to draining LNs or partake in priming adaptive responses. In contrast, DCs and monocytes have an essential part in priming and/or maintaining adaptive responses. To be able to determine the target immune cells for the LNP/mRNA vaccine, mRNA encoding for mCitrine was used so that translated protein could be visualized and Atto655-labeled LNP was used so that uptake could be tracked after administration.

A noticeable amount of total APCs, as defined by expression of HLA-DR, were found to contain LNP and/or mCitrine already at 4 hours at the injection sites (FIG. 5A) and in the draining LNs of the I.D. group (FIG. 5B). No LNP+ or mCitrine+ cells were detected at the PBS or empty unlabeled LNP control sites demonstrating that the uptake and translation of LNP/mRNA is restricted to the vaccination sites and their draining LNs. Since the number of mobilized CD45+ immune cells was higher in the LNP/mRNA-injection sites at 24 hours than 4 hours, the level of CD45+ mCitrine+ cells was also more abundant at this time point (FIG. 5C). CD45- non-immune cells that translate mRNA have been detected in the muscle (Brito et al., Molecular Therapy: J of the ASGT, 22(12): 2118-29 (2014)) and skin after mRNA administration in mice (Probst et al., Gene Therapy, 14(15): 1175-80 (2007)). Although it was observed that CD45- cells were able to take up LNP, the translation of mCitrine mRNA was much less efficient compared to CD45+ immune cells (FIG. 5C).

A large portion of the CD45+ cells consisted of neutrophils as shown above. However, although neutrophils were efficient at internalizing LNP they did not show efficient translation of the mRNA cargo (FIG. 5C). In contrast, the classical CD14+CD16- monocytes were found to be most abundant mCitrine+ immune cells at 24 hours after LNP/mRNA delivery and were significantly higher in the draining LNs of the I.D. group already at 4 hours compared to the I.M. group (FIG. 5D). The mCitrine+CD16+monocyte subset was also found to be increased at 24 hours. In addition, CD1c+and CD1c- DCs as well as CD123+ PDCs, CD1a+ and CD209 APCs at the LNP/mRNA injection sites also showed clear mCitrine translation especially at 24 hours. There was overall more mCitrine+ and less LNP+ monocytes and DCs at 24 hours compared to 4 hours. The inverse relation may indicate that a large proportion of the acquired LNP has been degraded intracellularly at 24 hours and the mRNA cargo released and advanced to translation. Detachment or quenching of the Atto-655 dye on the LNPs in vivo is unlikely since LNP was readily detectable in neutrophils at both time points (FIG. 5C).

Uptake of LNPs was reported to be facilitated in presence of ApoE lipoprotein (Akinc et al., Molecular Therapy: J of the ASGT, 18(7): 1357-64 (2010); Pardi et al., J Controlled Release, 217: 345-51 (2015)) with affinity for lipid antigens and present in both sera and tissues. Monocytes and DCs highly express receptors for ApoE-coated LNPs, including LDL-(van den Elzen et al., Nature, 437(7060): 906-10 (2005)) and LDL-like receptors (Ferrer et al., Cytometry: J of the ISAC, 85(7): 601-10 (2014); Hart et al., J Immunology, 172(1): 70-78 (2004)). In addition, B cells also express LDL receptor (De Sanctis et al., Cllin Exp Immunology, 113(2): 206-12 (1998)). B cells were few in comparison to monocytes and DCs at the injection sites but LNP+ mCitrine + B cells were found in the draining LNs in both the I.D. and I.M. groups (FIG. 9A). The size of the LNPs (approx. 100 nm) enables cellular and passive transport to the draining LNs (Bachmann et al., Nat Rev Immunology, 10(11): 787-96 (2010)), which may explain uptake of LNP/mRNA by B cells. As B cells express TLR7 (Gujer et al., Immunology, 134(3): 257-69 (2011)) and present antigens, these features likely facilitate cognate T cell help during the process of B cell differentiation into vaccine-specific antibody secreting cells. mCitrine+T cells were also found in the LNs (FIG. 9A).

### mRNA-Induced immunity was demonstrated to relate to the level of translation and activation

Since monocytes and DCs at the injection sites and draining LNs showed efficient LNP uptake and mRNA translation as well as phenotypic differentiation, it was assessed whether these features co-existed in the same cell. A higher expression of CD80 in the APCs that showed mRNA translation (mCitrine+) was found, which suggests that APCs become activated by mRNA during translation (FIG. 6A). The same pattern was observed in rhesus and human APCs exposed in vitro to LNP/mRNA (FIG. 6B). As expected, there was no CD80 upregulation on cells exposed by empty LNP without mRNA.

As mRNA delivery induced type IIFN responses and upregulation of co-stimulatory molecule expression on APCs was directly associated with mRNA translation in the same cell, it was evaluated whether the magnitude and location of MxA+cells in vivo impacted the level of vaccine-induced immunity. MxA expression was detected within LN tissue particularly in the T cell zone where APCs also are present. It is well established that type I IFNs support both T cell and B cell responses (Montoya et al., Blood, 99(9): 3263-71 (2002); Gujer et al., J leukoc Biology, 89(6): 811-21 (2011)). As expected, no MxA was found in LNs prior immunization but high numbers of MxA+ cells were observed both in the T cell and B cell zones in draining LNs 24 hours after LNP/mRNA administration. Induction of type I IFNs is typically rapid (Asselin-Paturel et al., J Exp Medicine, 201(7): 1157-67 (2005)) but low levels of MxA+cells remained detectable after one week indicating that the IFN response was persistent. In support of this, translation of non-replicating modified mRNA formulated in LNPs has been reported to be detected >1 week after delivery (Pardi et al., J Controlled Release, 217: 345-51 (2015)) which shows that low degree of translation can continue. It was found that the MxA levels in the draining LNs at 24 hours correlated with the magnitude of MxA expression that reoccurred after the boost immunization. The level of MxA expression found in the vaccine-draining LNs one week after the boost immunization also correlated with HAI titers and IFNγ+CD4 T cell responses. Altogether, innate immune responses to mRNA vaccine delivery, such as cell infiltration and activation coinciding with translation of the mRNA and resulting in type I IFN responses appear to be central mechanisms for the induction of robust vaccine-induced immunity.

### Discussion

Modified mRNA vaccines have emerged as a promising vaccine platform and are being evaluated in preclinical studies as well as human trials for infectious diseases (Bogers et al., J Infectious Diseases, 211(6): 947-55 (2015); Brito et al., Molecular Therapy: J of the ASGT, 22(12): 2118-29 (2014); Petsch et al., Nat Biotechnology, 30(12): 1210-6 (2012); Hekele et al., Emerging Microbes & Infections, 2(8): e52 (2013); Geall et al., PNAS, 109(36): 14604-9 (2012)). Despite this, several fundamental aspects of the mechanisms of action of mRNA vaccines are unknown. This study provides insights of that mRNA formulated in LNPs particularly targets APCs at the site of administration and draining LNs leading to rapid translation of the encoded vaccine antigen and generation of vaccine-specific T cell and B cell responses. The APCs that translate the encoded antigen are especially activated. Type I IFN responses are central in the type of innate immune cell activation induced by the mRNA construct and likely play a major determinant in the type of vaccine-specific immunity developed.

Rhesus macaques that more closely resemble humans than mice in terms of anatomy and cell specific receptor expression were used in this study to reveal the early mechanisms dictating vaccine responses to mRNA vaccines. Self-amplifying mRNA constructs have shown to induce vaccine antigen-specific antibodies and CD4+ and CD8+ T cells in non-human primates (Bogers et al., J Infectious Diseases, 211(6): 947-55 (2015)). However, induction of high titers of antibodies required three immunizations of mRNA followed by two heterologous prime (SAM in nanoemulsion) and boost (protein and adjuvant) immunizations (Bogers et al., J Infectious Diseases, 211(6): 947-55 (2015)). Here, it is shown that two immunizations of LNP/H10 mRNA were sufficient to induce antibody titers above the protective level for influenza transmission.

The specific cell subsets were identified that A) internalized the vaccine formulation LNP and B) translated the mRNA cargo detected by mCitrine. A number of prior studies have elegantly demonstrated where mRNA vaccines are disseminated in mice after different routes to administration (Brito et al., Molecular Therapy: J of the ASGT, 22(12): 2118-29 (2014); Akinc et al., Molecular Therapy: J of the ASGT, 18(7): 1357-64 (2010); Pardi et al., J Controlled Release, 217: 345-51 (2015)). In vivo imaging techniques like the luciferase system have mainly been used for this type of investigation. Here, mRNA translation was examined at the cell subset level using multicolor flow cytometry of cell suspensions from the site of injection and draining LNs. Using this method, it was found that the predominate cell populations that were mobilized after mRNA vaccine delivery were neutrophils, monocytes and DCs. Consistent with that these cells are highly endocytic, they all efficiently internalized LNP but only monocytes and DCs showed high translation of the mRNA-encoding protein. The efficient targeting of professional APCs by the mRNA vaccine may therefore be one mechanism by which vaccine-specific responses are rapidly generated. Whether the mRNA encoded protein is loaded onto MHC and presented on the cell surface to T cells remains to be elucidated but it is clear that the APCs that translate protein also specifically undergo maturation characterized by upregulation of co-stimulatory molecules required in the antigen presentation process. Further, type I IFNs have reported to support upregulation of molecules involved in antigen presentation and processing in vitro (Simmons et al., J Immunology, 188(7): 3116-26 (2012)), which is in line with the presently disclosed data on increased gene expression of CD80, TAP2, CTSL and HLA-DR.

It was found that only low numbers of tissue-resident muscle cells or keratinocytes in the skin translated antigen. Intradermal delivery of mRNA in the ear of mice has shown that HLA-DR+APCs can translate mRNA but that most of the cells that translated mRNA in this model was tissue cells as concluded by cell morphology (Probst et al., Gene Therapy, 14(15): 1175-80 (2007)). A consistent finding in the experiments discussed herein as well as in previous studies is that mRNA translation after delivery into the skin or muscle is restricted to the site of infection and the draining LNs. The data also show that mRNA vaccine uptake and translation and immune activation occurs quickly after administration regardless if the I.D. or I.M. route is used.

Type I IFNs have been shown to be critical for inducing anti-tumor responses both in mice and humans in response to intravenously administered RNA aimed as cancer immunotherapy (Kranz et al., Nature, 534(7607): 396-401 (2016)). Consistent with the presently disclosed data, upregulation of co-stimulatory molecules on DCs was shown to be driven by IFNα induced exclusively by the mRNA cargo and not by the lipid carrier. In addition, earlier studies demonstrated that targeting of DCs by mRNA was efficient and necessary for the induction of antigen-specific T cells (Kranz et al., Nature, 534(7607): 396-401 (2016)). However, other studies utilizing mice devoid of the IFNα receptor showed that IFN reduces the antigen expression and consequently the induction of antigen-specific immunity (Pollard et al.,Molecular Therapy: J of ASGT, 21(1): 251-59 (2013)). The contradictory results may be related to differences in the amounts of IFNα stimulated by the different mRNA constructs and the formulation and route of administration used. The timing of transfection and initiation of mRNA vs. induction of IFNα secretion may also be critical for whether there is any reduction of antigen expression. Reduced immunogenicity due to inhibited mRNA vaccine translation by innate immune activation can be overcome by replacing uridine nucleosides with naturally occurring base modification such as pseudouridine and 5-methyl cytidine (Anderson et al., Nucleic Acids Research, 39(21): 9329-38 (2011); Kariko et al., Immunity, 23(2): 165-75 (2005); Claudio et al., EMBO Journal, 32(9): 1214-24 (2014)). mRNA constructs have been developed, which contain modified bases and donor methyl group on the capped RNA to allow for increased translation efficiency (Kuge et al., Nucleic Acids Research, 26(13): 3208-14 (1998)), while avoiding excess innate activation. These mRNA constructs have showed promise for regenerative as well as cancer therapy (Zangi et al., Nat Biotechnology, 31(10): 898-907 (2013); Warren et al., Cell Stem Cell, 7(5): 618-30 (2010); Kranz et al., Nature, 534(7607): 396-401 (2016); Wang et al., Molecular Therapy: J of the ASGT, 21(2): 358-67 (2013)).

IFNα is a multifaceted group of cytokines that influence the immune responses in several ways (Ivashkiv et al., Nat Rev Immunology, 14(1): 36-49 (2014)). IFNα promotes migratory capacity of immune cells to lymphoid tissues (Asselin-Paturel et al., J Exp Medicine, 201(7): 1157-67 (2005); Cicinnati et al., J of Interferon & Cytokine Research, 29(3): 145-60 (2009); Shiow et al., Nature, 440(7083): 540-44 (2006)), DC maturation (Pollard et al., Molecular Therapy: J of the ASGT, 21(1): 251-59 (2013); Montoya et al., Blood, 99(9): 3263-71 (2002); Asselin-Paturel et al., J Exp Medicine, 201(7): 1157-67 (2005); Cicinnati et al., J of Interferon & Cytokine Research, 29(3): 145-60 (2009)), cross-priming (Le Bon et al., Nat Immunology, 4(10): 1009-15 (2003)) and supports B cell survival, differentiation and Ig class switch (Pollard et al., Molecular Therapy: J of the ASGT, 21(1): 251-59 (2013); Gujer et al., Immunology, 132(3): 257-69 (2011); Le Bon et al., Nat Immunology, 4(10): 1009-15 (2003); Shaw et al., Blood, 115(15): 3051-57 (2010)). IFNα production induced by mRNA vaccine therefore likely polarizes the vaccine-specific responses. A much better understanding of how strong vaccine responses can be elicited, tailored and sustained over time with modified mRNA construct would have significant impact on improving the design of new vaccines.

### Materials and Methods

### Lipid nanoparticle vaccine formulation

The mRNA encoding hemagglutinin of H10N8 Influenza A virus (A/ Jiangxi-Donghu/ 346/ 2013) or the yellow fluorescent protein mCitrine were transcribed in vitro by T7 polymerase from a linear DNA template, which incorporates 5' and 3' untranslated regions (UTRs), including a poly-A tail (Warren et al., Cell Stem Cell, 7(5): 618-30 (2010)). S-adenosylmethionine was added to methylate capped RNA (cap1) for increased mRNA translation efficiency (Kuge et al., Nucleic Acids Research, 26(13): 3208-14 (1998)). Lipid nanoparticle (LNP) were formulated using a modified protocol previously described (Chen et al., J Controlled Release, 235: 236-44 (2016)). Briefly, lipids were dissolved in ethanol at a molar ratio of 50: 10: 38.5: 1.5 (ionizable lipid: DSPC: cholesterol: PEG-lipid). The lipid mixture was combined with a 50 mM citrate buffer (pH 4.0) containing mRNA at 3: 1 ratio (aqueous: ethanol) using a microfluidic mixer (Precision Nanosystems). For formulations containing GLA (Avanti Lipids), lipids were combined in a molar ratio of 50: 9.83: 38.5: 1.5: 0.17 (ionizable lipid: DSPC: cholesterol: PEG-lipid: GLA). All formulations were dialyzed against PBS, concentrated using Amicon ultra centrifugal filters (EMD Millipore) and passed through a 0.22 µm filter. Particles were 80 - 100 nm in size with > 95% RNA encapsulation.

### Immunizations

This animal study was approved by the Local Ethical Committee on Animal Experiments. Twelve Chinese rhesus macaques were housed in the Astrid Fagraeus laboratory at Karolinska Institutet according to guidelines of the Association for Assessment and Accreditation of Laboratory Animal Care and all procedures were performed abiding to the provisions and general guidelines of the Swedish Animal Welfare Agency. For assessment of immunogenicity, animals were allocated to three groups (n=4/group) receiving either intramuscular (I.M.) or intradermal (I.D.) administration of LNP/H10 mRNA (50µg) and the last group received LNP/H10 mRNA formulated with GLA adjuvant (5ug) by the I.M. route (group 3). Prime and boost immunizations were delivered at week 0 and 4 respectively, and animals receiving LNP/H10mRNA and GLA received additional boosting at week 15. For innate immune response studies, two groups of animals (n=4/group) received either two I.M. or I.D. injections of Atto-655 labeled LNP/mCitrine mRNA (50ug/site) at distal sites at 5 hour and 24 hour respectively. Internal control injections consisted of PBS (24 hour) and empty non-labeled LNP (5 hour or 24 hour). All injections were given during general anesthesia. The final volume for all I.M. injections was 0.5ml and administered on marked injection sites as described in detail (Liang et al., J Immunological Methods, 425: 69-78 (2015)). The volumes for I.D. injections were 0.1ml (immunogenicity) or 0.25ml (innate response study), where the latter was split into three separate deliveries adjacent to each other (<10 mm apart) at marked injection site to increase the area of antigen exposure.

### Analyses of vaccine-specific responses

The hemagglutination inhibition (HAI) assay was performed with 0.5% turkey red blood cells (Rockland Antibodies and Assays) diluted in PBS. Non-specific HAI was prevented by incubating serum overnight at +37°C with receptor destroying enzymes (Denka Seiken). Serial dilutions (1:2) of serum samples were performed in V-bottom 96-well plates in duplicates, starting from 1:10 dilution. Recombinant HA of H10N8 influenza A virus (4 units), A/Jiangxi-Donghu/346/2013 (Medigen Inc.) were added to diluted serum and incubated for 30 minutes at room temperature. The reciprocal of the last serum dilution that resulted non-agglutinated red blood cells represented the HAI titer. Titers <10 were assigned as 1. Stimulation of H10-specific recall CD4 T cell responses were performed on U-bottom 96-well plates where 1.5 million PBMCs/well were stimulated overnight with overlapping peptides of full length H10 protein (2µg) in presence of Brefeldin A (5µg/ml, Sigma). Production of IFNy, IL-2 and TNF by re-stimulated H10-specific CD4 T cells was evaluated after intracellular staining (Table 1) as described (Sandgren et al., J Immunology, 191(1): 60-69 (2013)). H10-specific CD4+ T cells were estimated by flow cytometry and their polyfunctionality was analyzed using SPICE software version 5.35.

### Tissue single cell suspensions

Injected muscle tissues were sampled during necropsy and stored separately in RPMI (Gibco) on ice as described (Liang et al., J Immunological Methods, 425: 69-78 (2015)). Injected skin (25 mm radius) was dissected for cell suspension. All tissues were processed separately without pooling 1 hour after harvesting. Muscle and skin were weighed, normalized to 2 grams by removal of fat, connective tissue and excess muscle or skin respectively. Muscles were processed and digested with Liberase as described in detail (Liang et al., J Immunological Methods, 425: 69-78 (2015)). Skin were digested with Liberase TH (0.26WU/ml) plus DNase (0.1mg/ml) at +37C° for 1 hour under agitation and Liberase activity was quenched with complete media (10% FCS, 1% penicillin/streptomycin/glutamine in RPMI). Skin digestions were filtered through 70 µm cell strainers (BD), washed with PBS and stained immediately for flow cytometry. Lymph nodes (LNs) were minced with scissors and mechanically disrupted in 70 µm cell strainers using a plunger, washed and stained immediately. PBMCs were obtained using standard protocols.

### Cell characterization by flow cytometry

Filtered and washed suspensions representing approximately 1 gram of tissue or 5 ×10⁶ LN cells were stained with live/dead fixable blue dead cell stain kit according to manufacturer's protocol (Molecular Probes), blocked with FcR-blocking reagent (Miltenyi Biotec) and stained with cocktail of fluorescent antibodies (Table 1). Stained samples were spiked with AccuCount beads (Spherotech) and cell subset numbers were calculated according to manufacturer's protocol. A total of 1-2 million events per sample were acquired using LSR Fortessa flow cytometer (BD) and analyzed with FlowJo software (Tree Star).

### CXCL10 (IP-10) ELISA

CXCL10 was detected by Human CXCL10 Quantikine ELISA (R&D Systems) according to manufacturer's protocol in serum collected pre-immunization, 1 week after prime and boost immunization respectively with LNP/H10 mRNA, 24 hours after LNP/mCitrine mRNA injection and in supernatants after in vitro stimulations

### In situ staining

For in situ staining, 4 mm punches biopsies from injection sites and draining LNs were collected and snap-frozen in OCT (Sakura). Cryosections of muscle and skin from injection sites and LN biopsies (6 µm) were stained with anti-MxA antibody (Professors Haller and Kochs, University of Freiburg, Germany) as described (Bond et al., J Invest Dermatology, 132(1): 114-23 (2012)). All except LN sections were also stained with wheat germ agglutinin-Alexa Fluor 594 to visualize plasma membrane. Sections were mounted with SlowFade^{®} Gold antifade regent with or without DAPI (Molecular Probes). Images were acquired using a Nikon Eclipse Ti-E, Nikon A1 confocal microscope, 20x objective.

### In vitro stimulation

Rhesus macaque PBMCs or APCs isolated with RosettSep monocyte enrichment kit (Stemcell technologies) were stimulated overnight with either LNP/H10 mRNA (5ug), LNP/mCitrine mRNA (5ug) or unlabeled empty LNP (5ug) where indicated. Detection of intracellular IFNα by flow cytometry was performed as above. Cellular activation by CD80/CD86 upregulation was assessed by flow cytometry (Table 1).

**Table 1: Antibody clones for labeling the indicated markers for flow cytometry**

| **Marker** | **Clone** | **Company** |
|---|---|---|
| CD1a | SK9 | BD Biosciences |
| CD1c (BDCA-1) | AD5-8E7 | Miltenyi Biotec |
| CD11c | 3.9 | Biolegend |
| CD123 | 7G3 | BD Biosciences |
| CD14 | M5E2 | Biolegend |
| CD16 | 3G8 | BD Biosciences |
| CD20 | L27 | BD Biosciences |
| CD209 (DC-SIGN) | DCN46 | BD Biosciences |
| CD3 | SP34-2 | BD Biosciences |
| CD4 | L200 | BD Biosciences |
| CD45 | D058-1283 | BD Biosciences |
| CD45RA | L48 | BD Biosciences |
| CD66 biotin | TET2 | Miltenyi Biotec |
| Streptavidin-BV421 | - | Biolegend |
| CD8 | RPA-T8 | BD Biosciences |
| CD8 | SK-1 | BD Biosciences |
| CD80 | L307.4 | BD Biosciences |
| CD86 | IT2.2 | BD Biosciences |
| CCR7 | G043H7 | Biolegend |
| HLA-DR | TU36 | Invitrogen |
| IFN alpha | LT27:295 | Miltenyi Biotec |
| IFN gamma | 4S.B3 | BD Biosciences |
| IL-2 | MQ1-17H12 | BD Biosciences |
| TNF | MAb11 | Biolegend |

### Microarray and data analysis

Skin, muscle, and LN tissues were stored in RNALater (Invitrogen) at -20C until use and homogenized in Trizol (Ambion) with Tissuelyser (Qiagen). Cleanup, quality control and quantity of the RNA were assessed. Cyanine-3 (Cy3) labeled cRNA was prepared from 200ng total RNA with Quick Amp Labeling Kit (Agilent) according to manufacturer's protocol, followed by RNeasy column purification (Qiagen). Cy3-cRNA was fragmented at 60°C for 30 minutes in fragmentation buffer and blocking agent (Agilent) as per manufacturer's instructions. Agilent hybridization buffer was added to the fragmentation mixture and hybridized to Agilent Rhesus Macaque Gene Expression Microarrays v2 (part number G2519F-026806) for 17 hours at 65°C in a rotating Agilent hybridization oven. Microarrays were first washed with GE Wash Buffer1 and then with +37°C GE Wash buffer 2 (Agilent). Slides were scanned by Agilent DNA Microarray Scanner (G2505C) and images were processed in Agilent Feature Extraction Software. Local background adjusted signals (gProcessedSignal) were further quantile normalized using the Bioconductor package to achieve consistency between samples. Normalized data was analyzed to identify statistically significant genes by comparing PBS control and LNP/mRNA vaccine samples (n=4) within injection sites and draining LNs. Probesets were considered significant if |log2| FC > 0.5 and p-value < 0.05 by Students t-test, corrected for False Discovery Rate. Changes in a pre-selected set of genes of interest, related to inflammation, migration and antigen uptake/presentation were also considered using descriptive plots. Probe sets corresponding to these genes were identified by manual curation of the array manufacturer's annotation. Statistical analysis and generation of descriptive was carried out using customized scripts in R and Python.

### Example 2 - mRNA Vaccines Cause Differential Accumulation of T cell Activators and Suppressors Such as Myeloid-Derived Suppressor Cells at Injection Site and Lymph Nodes after Vaccination

### Summary

Myeloid-derived suppressor cells (MDSCs) are major regulators of T cell responses in several pathological conditions. Whether MDSCs increase and influence T cell responses in temporary inflammation, such as after vaccine administration, is unknown. Using the rhesus macaque model, critical for late-state vaccine testing, it was demonstrated that monocytic (M)-MDSCs and polymorphonuclear (PMN)-MDSCs can be detected using several of the markers used in humans. However, rhesus M-MDSCs lacked expression of CD33, PMN-MDSCs were identified as CD33+ low-density neutrophils. Importantly, both M-MDSCs and PMN-MDSCs showed suppression of T cell proliferation in vitro. The frequency of circulating MDSCs rapidly and transiently increased 24 hours after vaccine administration. M-MDSCs infiltrated the vaccine injection site but not vaccine-draining lymph nodes. This was accompanied by upregulation of genes relevant to MDSCs such as arginase-1, IDO1, PDL1 and IL-10 at the injection site. MDSCs may therefore play a role in locally maintaining immune balance during vaccine-induced inflammation.

### Results

### Phenotypic similarities of MDSCs between rhesus macaques and humans

Since the identification and function of MDSCs in NHPs is to a large extent unexplored, multiple antibodies were first tested for markers used for human cells to identify MDSCs in rhesus macaques. The NHP Reagent Resource website (nhpreagents.org) was used and the cross-reactivity of the human antibody clones to rhesus macaques was confirmed. Since CD15 is not ideal for identification of granulocytes in rhesus macaques, CD66abce was used, which identifies the same cell population as reported previously (Vono et al., Blood, 2017). CD8 was used instead of CD56 to discriminate NK cells because CD56 is expressed on subsets of rhesus monocytes and most rhesus NK cells express CD8 (Sugimoto et al., J of Immunology, 195: 1774-81 (2015)). Distinct cell populations were found in the low-density cell fraction (regularly also referred to as PBMC fraction after Ficoll centrifugation) (FIG. 10A). This fraction contained CD14+ classical monocytes (HLA-DR+LinCD14+), M-MDSCs (HLA-DRLinCD14+) as well as LDNs (HLA-DR-CD66abce+). The higher granularity of LDNs vs. monocytes and M-MDSCs was confirmed by the difference in Side Scatter (SSC) parameter (FIG. 10B). The LDNs were composed of two populations based on CD33 expression; CD33- LDNs representing around 2/3 of the cells and CD33+ LDNs. The fraction of sedimented cells consisted of NDNs, which were found to be a uniform population of CD33+ cells (FIG. 10C, left panel). In contrast to rhesus LDNs and NDNs, the human counterparts all showed CD33 expression (FIG. 10C, right panel), although human LDNs are known to consist of a heterogeneous population of immature/mature and inactivated/activated neutrophils (Scapini et al., Immunological Reviews, 273: 48-60 (2016)).

To further characterize the phenotype of CD33+ and CD33- rhesus LDNs, the expression of CD10, CD49d, CD11c and CD45RA associated with neutrophil development (Elghetany, Blood Cells, Molecules, & Diseases, 28: 260-74 (2002)) and typical neutrophil activation markers CD11b and CD62L was measured. It was found that CD10, uniquely expressed on segmented mature neutrophils, was highly expressed on CD33+ LDNs, but was low or undetectable on CD33- LDNs (FIG. 10D). Vice versa, CD49d, present on immature neutrophils and absent on segmented neutrophils, was expressed on CD33- LDNs but not on CD33+ LDNs. Both subsets expressed CD11c but not CD45RA. Downregulation of CD62L was observed only on the CD33- LDNs while CD11b was expressed on both subsets. Collectively, this suggests that rhesus CD33+ LDNs display a mature phenotype and that CD33- LDNs represent immature neutrophils with activated state.

As expected, it was found that CD33 was expressed on human M-MDSCs as well as CD14+classical monocytes and CD11c+myeloid dendritic cells (MDCs) (Cravens et al., Scandinavian J of Immunology, 65: 514-24 (2007)) (FIG. 10E, top panel). In contrast, the rhesus counterparts did not show surface expression of CD33 (FIG. 10E, top panel). This was verified using two different anti-CD33 antibody clones (HIM3-4 and AC104.3E3) of which the latter bound CD33 on rhesus LDNs. Expression of CD33 is therefore not a strategy to identify M-MDSCs in rhesus macaques. Human M-MDSCs and monocytes have been reported to express both CD11b and CCR2 (Bronte et al., Nature Communications, 7: 12150 (2016); Lesokhin et al., Cancer Research, 72: 876-86 (2012)). Similarly, rhesus M-MDSCs and monocytes co-expressed CD11b and CCR2 (FIG. 10E, bottom panel). Based on these data a strategy for identifying the different populations of MDSCs in rhesus macaques was defined.

### T cell responses are suppressed and the suppression is partly mediated by arginase-1

To functionally assess if any of the rhesus neutrophil subsets represented PMN-MDSCs with suppressive effects on T cells purified CD33- LDNs, CD33+ LDNs or NDNs were co-cultured with CFSE-labeled autologous PBMCs in presence of SEB. As expected, SEB induced strong proliferation of T cells. In contrast, the addition of CD33+ LDNs induced a clear reduction in T cell proliferation (FIG. 11A). However, CD33- LDNs and NDNs were both not able to reduce T cell proliferation. In addition, CD33+ LDNs could also inhibit the production of IFN-γ and IL-17A by T cells (FIG. 11B). To further validate this effect, PBMCs depleted of CD33+ LDNs were exposed to SEB, which resulted in an increased T cell proliferation (FIG. 11C). This demonstrates that rhesus PMN-MDSCs are represented by CD33+ LDNs.

Several mechanisms have been proposed as the suppressive function of MDSCs. Arginase-1, which is stored in granules under steady state, is a primary candidate (Rotondo et al., J Leukocyte Biology, 89: 721-27 (2011)). Exocytosis of arginase-1 from MDSCs or activated neutrophils inhibits T cell responses via metabolizing L-arginine needed for T cell survival. Consequently, high mRNA levels but low intracellular protein levels of arginase-1 have been reported in MDSCs (Rodriguez et al., Cancer Research, 69: 1553-60 (2009)). It was found that NDNs and CD33- LDNs constitutively has intracellular levels of arginase-1, while CD33+ LDNs showed much lower or undetectable levels indicating release of arginase-1 (FIG. 11D). In addition, co-cultures of PBMCs and CD33+ LDNs supplemented with L-arginine partly recovered the suppressed T cell proliferation (FIG. 11E). Addition of L-arginine to PBMC cultures with or without SEB stimulation did not increase T cell proliferation. This suggests that release of arginase-1 from rhesus CD33+ LDNs is one mechanism causing T cell inhibition.

### Functional characterization of rhesus M-MDSCs

The function of M-MDSCs was evaluated using a similar strategy as above. Since M-MDSCs could not be isolated based on CD33 expression, CD14+/HLA-DR- cells were purified, representing M-MDSCs. The cells were compared to total HLA-DR+ cells for their ability to interfere with T cell proliferation. M-MDSCs were able to suppress T cell proliferation, and dampened the production of IFN-γ (FIGs. 12A-12B). In contrast, HLA-DR+ cells showed no influence on T cell response.

In vitro generation and adoptive transfer of human M-MDSCs is being explored to treat autoimmune diseases or graft-versus-host diseases (GVHD) (Cripps and Gorham, International Immunopharmacology, 11: 789-93 (2011); Le Blanc et al., Oncoimmunology, 2: e25009 (2013)). Human M-MDSCs can be differentiated in vitro from monocytes either using sorted cells or bulk PBMCs by exposure to cytokines (Lechner et al., J of Immunology, 185: 2273-84 (2010); Obermajer et al., Blood, 118: 5498-5505 (2011)). It was reported that generation of rhesus M-MDSCs from monocytes using GM-CSF, IL-4 plus PGE₂ was not successful although this cocktail was able to generate human M-MDSCs (Zahorchak et al., Am J of Transplantation, 16: 661-71 (2016)). Therefore several different cytokine combinations were tested for generating M-MDSCs from rhesus PBMCs. In cultures supplemented with GM-CSF either alone or in combination with IL-6, IL-11, IL-1β or PGE₂ for 7 days, a population of CD14+CD11b+ cells was differentiated (FIGs. 12C-12D, top panel). This population appeared to contain both monocytes and M-MDSCs based on the differential expression of HLA-DR (FIGs. 12C-12D, bottom panel). The combination of GM-CSF and IL-6 was particularly effective in differentiating HLA-DR^{low/-} cells. The generated M-MDSC-like cells were assessed for their T cell inhibitory function. Due to the small numbers of HLA-DR-CD14+ cells that could be retrieved from the cultures, it was only technically feasible to sort the total CD14+ cells and co-culture them with PBMCs plus SEB. Nevertheless, it was found that the T cell proliferation was strongly reduced in the presence of CD14+ cells isolated from GM-CSF/IL-6-cultures compared to CD14+ cells from unstimulated cultures (FIGs. 12E-12F). A population of cells reminiscent of M-MDSCs with suppressive effects on T cells can therefore be generated in vitro from rhesus PBMCs.

### An increase in suppressor cells (MDSCs) is accompanied with upregulated expression of MDSC-relevant genes after vaccine administration at the injection site, leading to regulation of injection site inflammation.

Numerous studies have been performed to understand the relationship between MDSCs and different pathological conditions while the role of MDSCs during temporary inflammatory conditions like vaccination is largely unexplored. How the levels of MDSCs were affected by vaccine administration was analyzed by monitoring rhesus macaques receiving a novel influenza vaccine (Bahl et al., Molecular Therapy: J ofASGT, 2017). This vaccine formulation induced influenza hemagglutinin-specific CD4+T cell response detected by IFN-γ production as well as neutralizing hemagglutinin antibody titers above the protective levels reported for seasonal influenza vaccination (FIG. 13A). It was found that the frequencies of M-MDSCs in the blood rapidly increased at day 1 after vaccine administration (FIG. 13B, top panel). Classical monocytes also increased following administration. CD33+ LDNs and CD33- LDNs also showed a trend towards increased frequencies (FIG. 13B, bottom panel). The levels of all subsets had returned to pre-vaccination levels after 7 days (data not shown).

It is well described that MDSCs regulate immune responses locally in inflamed tissues or tumors. It has previously been found that administration of different formulations of vaccines leads to a local inflammation at the site of injection and draining lymph nodes (dLNs) as a result of infiltration of immune cells and cell activation (Liang et al., Science Translational Medicine, 2017; Vono et al., Blood, 2017). Therefore the frequency of MDSCs in biopsies was analyzed from the site of injection (muscle) as well as dLNs collected at 1 day after vaccination. It was found that similar to blood, the frequencies of both M-MDSCs and classical monocytes were significantly elevated at the site of vaccine injection compared to the donor-matched PBS-injection site (FIG. 13C, top panel). Monocytes also accumulated in the vaccine-dLNs while this was not found for M-MDSCs (FIG. 13C, bottom panel). Infiltration of granulocytes was also found both at the vaccine injection site and dLNs. However, due to the limited knowledge about the phenotype of PMN-MDSCs in tissues, it cannot be concluded whether the infiltrating granulocyte population contained suppressive PMN-MDSCs.

Transcriptomics analyses to measure MDSC-associated genes in the biopsies was also performed. 59 genes were selected and divided into four distinct functional properties. Gene expression of inflammatory molecules such as S100A8/A9, IL-1β, COX-2 and IL-6 which are proposed to be critical in the expansion of MDSCs, were markedly upregulated at the vaccine injection sites compared to PBS injection sites (FIG. 13D, panel 1). Several genes encoding for mediators involved in the suppressive mechanisms by MDSCs such as the typical soluble molecules arginase-1, Indoleamine 2,3-dioxygenase (IDO), nitric oxide synthase (NOS) and IL-10, as well as surface molecule PD-L1 that mediated T cell suppression via cell-cell interaction were also upregulated (FIG. 13D, panel 2). Elevated gene expression of chemokines such as CCL2, CCL4 and CCL5 known to attract MDSCs to tumor and infection sites (Kumar et al., Trends in Immunology, 37: 208-20 (2016)) was also observed in vaccine injection sites (FIG. 13D, panel 3). Mediators in inflammatory pathways such as MyD88 and NLRP3 were also elevated at the vaccine injection sites (FIG. 13D, panel 4). The increase of genes was also found in vaccine-dLNs but was much lower than in the vaccine injection sites. Genes such as arginase-1, IL-10, CCL2 and CCL5 that were elevated at the vaccine injection sites showed a smaller change in the dLNs (FIGs. 13E-13F).

### Discussion

As one of the major immune regulators, MDSCs have been intensively studied with most focus on their contribution to tumor development, but also in other inflammatory conditions. Although the significance of MDSCs in immune regulation has been supported by numerous studies, critical information about their heterogeneity and phenotypic identification is still lacking. Initial studies mainly defined MDSCs based on expression of myeloid markers and absence of HLA-DR plus lymphoid-associated antigens. However, this criterion is not sufficient due to the phenotypic similarity of MDSCs to other cell subsets. The main challenge is the identification of PMN-MDSCs, which share almost all surface markers with other neutrophil subsets. Increasing evidence shows that human PMN-MDSCs represent a unique subset of LDNs and Ficoll centrifugation is therefore regarded as the only method to enrich PMN-MDSCs from blood (Bronte et al., Nature Communications, 7: 12150 (2016)). Additional functional assessment is therefore critical to accurately identify them.

It was found that CD33+ LDNs represent PMN-MDSCs in rhesus macaques and possess suppressive effects on T cells, which is partly mediated by release of arginase-1. Other mechanisms are likely also involved and remain to be investigated. Interestingly, it was found that suppressive rhesus CD33+ LDNs display a mature phenotype, which was unexpected since MDSCs are considered immature myeloid cells. However, a recent study also showed that suppressive human CD10+ LDNs display a mature phenotype (Marini et al., Blood, 129: 1343-56 (2017)). In fact, the relationship between suppressive neutrophils and PMN-MDSCs has been a growing discussion, particularly with regards to cell origin, diversity and nomenclature (Brandau et al., Seminars in Cancer Biology, 23: 171-82 (2013)).

M-MDSCs have been investigated in more depth than PMN-MDSCs because of their longer lifespan and better cell viability after cryopreservation (Kotsakis et al., J of Immunological Methods, 381: 14-22 (2012)). M-MDSCs have been reported to be more potent than PMN-MDSCs at excreting a suppressive effect (Youn and Gabrilovich, European J of Immunology, 40: 2969-75 (2010)). Human M-MDSCs are few in healthy individuals but still show a suppressive effect on T cell proliferation (Wang et al., J of Immunology, '94: 4215-21 (2015)). Consistent with this, it was found that the few rhesus M-MDSCs purified from healthy animals had a suppressive effect on T cells. In vitro generation of human M-MDSCs using different combinations of cytokines has been proposed as a promising therapeutic strategy for treatment of autoimmune diseases or GVHD by adoptive transfer of in vitro generated autologous M-MDSCs (Cripps and Gorham, International Immunopharmacology, 11: 789-93 (2011); Le Blanc et al., Oncoimmunology, 2: e25009 (2013)). It was found that rhesus M-MDSCs can be generated using similar cytokine combinations especially IL-6 and GM-CSF. NHPs may therefore be a valuable model for pre-clinical investigations of MDSC-based immunotherapy and offer a high degree of clinical translatability.

NHPs are and have been critical in the development of several vaccines against infectious diseases. The use of NHPs for validating the potential of new powerful vaccine platforms such as modified mRNA vaccines before proceeding to clinical trials has been of great importance (Bahl et al., Molecular Therapy: J of the ASGT, 2017). Therefore, MDSCs were studied after administration of an mRNA vaccine encoding for influenza hemagglutinin that induces protective levels of antibodies. It has previously been shown that administration of successful vaccine formulations induces a robust temporary inflammation and innate immune activation culminating in priming of vaccine-specific responses (Liang et al., Science Translational Medicine, 2017; Liang and Lore, Clinical & Translational Immunology, 5: e74 (2016)). Whether MDSCs are induced as a result of the inflammation and have a role in regulating the generation of adaptive responses is not known. In the current study, it was found that circulating MDSCs were induced rapidly after vaccination, accompanied by the non-suppressive counterparts of myeloid cells. However, the expansion of MDSCs did not suppress the vaccine outcome, indicated by well-detectable vaccine-specific T and B cell responses in all animals. Elevated levels of MDSCs have earlier been proposed to suppress vaccine responses at later time points (Sui et al., J Clinical Investigation, 124: 2538-49 (2014)). Future studies of whether the influence of MDSCs is a reason for poor vaccine responses found in patient groups with conditions associated with high MDSC levels would be highly relevant.

As the accumulation of M-MDSCs was observed in vaccine-injection sites and not in vaccine-dLNs, MDSCs may not mobilize to the location where most of T cell response occurs. A large number of MDSC-relevant genes were upregulated at vaccine injection sites but to a lower degree in dLNs. The rapid and transient expansion of circulating MDSCs and their differential distribution in peripheral sites suggest an immune-balancing role. Since the majority of vaccines are administrated by i.m. injection, local innate immune events play an important role in shaping adaptive immunity and finally determine vaccine outcome (Liang and Lore, Clinical & Translational Immunology, 5: e74 (2016)). An inflammation at the injection site is necessary to induce sufficient vaccine responses but it needs to resolve rapidly to avoid local or systemic side effects (Lapphra and Scheifele, Paediatrics & Child Health, 14: 245 (2009); von Elten et al., Human Vaccines & Immunotherapeutics, 10: 1767-70 (2014)). It is hypothesized that the inflammation induced by vaccine administration contributes to the expansion of circulating MDSCs. The inflammatory microenvironment formed at the local injection site recruits circulating MDSCs that in turn produce immune suppressive mediators and prevent excessive inflammation. However, compared to monocytes and DCs, much fewer numbers of MDSCs appear to migrate to the vaccine dLNs, which could explain why the generation of adaptive immune responses is not suppressed.

Overall, this study provides original data for identifying MDSCs in NHPs both phenotypically and functionally. In addition, it was demonstrated that MDSCs are induced by vaccination. Further investigations of the relationship between MDSCs and vaccine outcome is required to understand how innate immune activation dictate vaccine responses.

### Materials and Methods

### Animals and sample collection

All animal experiments were performed in accordance with the guidelines of the Association for Assessment and Accreditation of Laboratory Animal Care and the Swedish Animal Welfare Agency. The study was approved by the Local Ethical Committee. Rhesus macaques were housed at Astrid Fagraeus Laboratory in Karolinska Institutet, Sweden.

### Cells preparation

Peripheral venous blood was collected and the cells processed within 1 hour. PBMCs were isolated using Ficoll (GE Healthcare) according to the manual. CD33+ LDNs, CD33- LDNs, M-MDSCs were isolated by magnetic-activated cell sorting (MACS). NDNs were isolated using dextran sedimentation assay.

### Microarray analysis

Biopsies of muscle and lymph nodes were collected and total RNA was extracted using TRIzol (Invitrogen) plus Tissuelyser (Qiagen) according to the manufacturer's instruction. 2 µg total RNA was used for probe synthesis of Cyanini-3 (Cy3) labeled cRNA using Quick Amp Labeling Kit (Agilent) prior to purification with RNeasy column (Qiagen). Cy3-cRNA was then hybridized to Agilent Rhesus Macaque Gene Expression Microarray (G2519F; Design ID: V2-026806) and processed according to manuals. The background corrected data from output were further normalized using the Bioconductor package to achieve consistency between samples. A set of genes relevant to MDSCs was selected and gene expression in vaccine/PBS-injection muscle sites or injection site draining LN was measured and compared. Agilent gene data are deposited in Gene Expression Omnibus (accession number, GSE98211).

### Statistical analysis

Statistical analysis was conducted by Prism Version 6.0 software. All values are presented as mean ± SEM for at least four independent experiments. Differences between groups were analyzed by unpaired or paired student t test. A p-value <0.05 is considered to be statistically significant.

### Example 3 - mRNA Vaccines Cause Differential Accumulation of B cells Predictive of Antibody Avidity After Vaccination

### Summary

The immunological events leading to strong antibody responses elicited by mRNA vaccines are unknown. In this study, it is demonstrated that protective levels of antibodies to mRNA encoding an antigen were induced after, in some cases one immunization, and in other cases, two immunizations of modified non-replicating mRNA encoding antigen encapsulated in LNP in non-human primates. While both intradermal (ID) and intramuscular (IM) administration induced protective titers, ID delivery generated this response more rapidly. Circulating antigen-specific memory B cells expanded after each immunization, along with a transient appearance of plasmablasts. The memory B cell pool waned over time but remained detectable throughout the 25-week study. Following prime immunization, antigen-specific plasma cells were found in the bone marrow and persisted overtime.

Importantly, germinal centers (GC) were formed in vaccine-draining lymph nodes along with an increase in circulating antigen-specific ICOS+PD-1 +CXCR3+ T follicular helper cells, a population correlated with high avidity antibody responses after vaccination in humans. The study demonstrates that mRNA/LNP vaccines potently induce an immunological repertoire associated with the generation of high magnitude and quality antibodies, and that the production of such cell populations can serve as a quality control parameter for the development of highly effective mRNA vaccines.

### Results:

### mRNA Vaccine encoding H10 induces protective levels of antibodies

Rhesus macaques were immunized either intramuscularly (IM) or intradermally (ID) with an mRNA vaccine encoding full-length HA of H10N8 (A/Jiangxi-Donghu/346/2013) (H10) formulated in LNP (FIG. 14A). A third group received this formulation combined with the TLR4-agonist glucopyranosyl lipid adjuvant (GLA) to evaluate whether an adjuvant could further enhance the responses. All animals received a homologous prime-boost immunization at 0 and 4 weeks (FIG. 14B). In addition, the GLA group received a third immunization at 15 weeks.

All animals induced neutralizing antibody titers against HA above the accepted level of protection for seasonal influenza vaccination, as measured by hemagglutination inhibition assay (HAI) (FIG. 14C). Although some of the animals in the ID group showed titers at the protective level already after the prime immunization, all groups had titers that exceeded this level after boost and they persisted above for the remainder of the study. The titers were significantly higher in the ID group compared to the IM groups for up to two weeks after the boost, but were thereafter at similar levels. The group that received GLA did not show higher HAI titers compared to the other groups. This indicates that the mRNA/LNP formulation itself was sufficiently immunogenic. The third immunization in the GLA group resulted in a transient increase in HAI titers, which returned to similar levels as the other groups five weeks later.

HAI titers combine both antibody quantity and quality, however by combining the two measurements certain mechanistic insights into antibody development over time are lost. Therefore, total IgG titers against H10 as well as antibody avidity were also measured separately. H10-specific IgG antibody titers were induced both in the IM and ID groups already after prime immunization, were increasing at the time of the second immunization and peaked two weeks thereafter (FIG. 14D). The avidity index of H10-specific IgG antibodies determined by a chaotropic ELISA wash assay did not improve between the second immunization at week 4 and week 6 (FIG. 14D). However, at week 11 there was a clear increase in avidity, which continued to increase until the study end. There were no significant differences in IgG titers or avidity between the ID and IM group, with the exception of higher IgG titers (p<0.0001) in the ID group two weeks after boost. Collectively, this demonstrated that two immunizations with the H10 mRNA/LNP formulation were sufficient to induce protective and durable antibody titers.

### Rapid and sustained B cell responses after mRNA vaccination

To characterize the kinetics of the B cell responses at the cellular level, the frequency of H10-specific memory B cells was determined by ELISpot (FIGs. 15A-15C). Circulating H10-specific memory B cells were readily detectable two weeks after the prime immunization (FIGs. 15A-15C). The number of H10-specific memory B cells contracted slightly but expanded again with the boost immunization. This was followed by a gradual decline. The GLA group showed an additional increase two weeks after the second boost as expected (FIG. 15C). By study end at 25 weeks, the IM and ID groups showed similar levels of circulating H10-specific memory B cells (FIG. 15D), whereas the GLA group had higher levels due to the more recent boost.

Similar to the rapid induction of circulating H10-specific memory B cells, we detected H10-specific PCs in the bone marrow two weeks after the prime immunization in all groups (FIGs. 15A-15C). However, in contrast to the memory B cell pool, the number of PCs remained relatively stable throughout the study. This dichotomy in fluctuation between memory B cells and PCs is in line with previous reports on protein vaccine immunizations in rhesus macaques.

The number of H10-specific PCs declined to significantly lower levels in the IM group compared to the ID group at study end (FIG. 15E). The numbers of H10-specific plasmablasts in some of the animals one week after immunization were close to the limit of detection after the prime immunization but increased to readily detectable levels after the boost immunization (FIG. 15F).

It is shown that priming of vaccine-specific T cells occurs in the vaccine-draining LNs. To investigate whether priming of B cells also takes place at this site, vaccine-draining LNs were collected nine days after prime immunization and compared with control LNs that did not drain at the vaccine delivery site. H10-specific memory B cells and PCs were detected only in the vaccine-draining LNs (FIGs. 15G-15H). This was observed with both ID and IM administration. This demonstrated that the H10 mRNA/LNP vaccine induces robust levels of vaccine-specific memory B cells and PCs already after prime immunization and that these levels are well-sustained.

### Germinal center formation in vaccine-draining lymph nodes

Durable levels of H10-specific memory B cells and PCs and a steady increase of IgG antibody avidity induced by the H10 mRNA/LNP vaccine are likely the outcome of a strong GC reaction. Since priming of H10-specific B cells appeared to be restricted to the vaccine-draining LNs, we also analyzed the expansion of GCs at this site. Using immunofluorescence and confocal imaging of cryosections we quantified the area of GCs, the numbers of PD-1+ Tfh cells and proliferating Ki67+ cells within individual GCs (FIG. 16A). Since the HAI titers did not differ between the three study groups, the data was not stratified by group for this analysis. Instead, LNs were evaluated pre-immunization and two weeks post-boost immunization from seven of the animals. The total GC area, total numbers of GC Tfh cells or GC Ki67+ cells by were normalized by LN area. In five out of the seven animals, there was an increase in the GC area/LN area ratio post-immunization (Fig 16B). There was also an increase in the number of GC Ki67+cells/LN area ratio and the number of GC Tfh cells/LN area ratio (FIGs. 16C-16D). An advantage of analyzing tissue sections is the option to study individual GCs in intact LN architecture rather than all GC cells combined as is done with cell suspensions. The area of individual GCs was significantly increased post vaccination (FIG. 16E). This was also observed for Tfh cells and GC Ki67+ cells within individual GCs (FIGs. 16F-16G). The increase in the number of GC Ki67+ cells was higher than the increase in the number of GC Tfh cells as expected since GC B cells have exceptional proliferating capacity. To this end, it has been shown that the B cell/Tfh cell ratio is higher in top neutralizers after HIV Env vaccination. A significant increase was observed in the Ki67+ cell/Tfh cell ratio post-vaccination in individual GCs (FIG. 16H). There was a strong correlation between the number of Ki67+ cells and the area within each GC (p<0.001 R2=0.4613) (FIG. 16I). This was also found for the number of GC Tfh cells and the GC area of individual GCs (p<0.001 R2=0.5869) (FIG. 16J). Additionally, Tfh cell numbers correlated with Ki67+ cell numbers within each GC (p<0.001 R2=0.5617) (FIG. 16K).

Increase of the chemokine CXCL13 in plasma was recently proposed as a biomarker for GC activity in the LNs. There was a modest elevation in CXCL13 levels detected transiently at one week after prime vaccination (FIG. 16E). GC formation after prime vaccination was also investigated using flow cytometry by enumerating the frequency of BCL6+ Ki67+ GC B cells expressing H10-specific B cell receptors in the vaccine-draining LNs (FIG. 16M). No H10-specific GC B cells could be detected in control non-draining LNs, but both unswitched IgM+ and class-switched IgM- BCL6+Ki67+ GC H10-specific B cells were detected in vaccine-draining LNs nine days after prime immunization (FIG. 16M). Taken together, this demonstrates that the mRNA/LNP vaccine is a potent inducer of GC activity.

### Circulating H10-specific ICOS+ PD-1+CXCR3+ T follicular helper cells are induced after vaccination and correlate with antibody avidity

As mentioned above, cTfh cells can be identified in blood as CXCR5+ICOS+PD-1+ CD4+ T cells, and specifically the Th1-polarized CXCR3+ cTfh cell subset has been shown to correlate with high-avidity antibodies seven days after influenza vaccination in humans. cTfh cell subset frequencies were investigated pre-vaccination and seven days after prime and boost. It was first concluded that there was no general increase in CXCR3+ or CXCR3- total CD4+ T cells (FIG. 17A). CXCR5+ICOS+PD-1+CXCR3+/- cTfh cells within the central memory (CD28+CD95+) CD4+T cell population were then specifically analyzed (FIG. 17B). While there was no increase in CXCR3- cTfh cells (FIG. 17C), there was a significant increase in the number of CXCR3+ cTfh cells both after prime and boost (FIG. 17D).

Whether the CXCR3+ cTfh cells were expanded in the vaccine-draining LNs before entering the circulation was determined by analyzing the set of LNs collected at nine days after prime. The frequency of Tfh cells was increased in the vaccine-draining LNs compared to control non-draining LNs (FIG. 17E). In addition, the majority of the Tfh cells in vaccine-draining LNs expressed CXCR3 (FIG. 17E). The CXCR3+ Tfh cells in vaccine-draining LNs also expressed Ki67 at higher levels than the CXCR3- Tfh cells in the same LNs as well as compared to CXCR3+/- Tfh cells from the control LNs (FIG. 17F). Although difficult to prove, these findings may indicate that the cTfh1 polarized cells found in peripheral blood after mRNA vaccine administration have been generated in the vaccine-draining LNs.

The number of CXCR3+ cTfh cells after boost showed a trend of correlation with antibody avidity at week 6 and 25 and correlated significantly at week 11 (p=0.0432 R2=0.5916) (FIG. 18A). To investigate the specificity of the cTfh cells, PBMCs were stimulated with H10 overlapping peptides for an intracellular cytokine assay (FIG. 18B). There were few H10-specific cells evidenced by IFNγ production within the total CD4+ central memory T cell population one week after prime but there was a clear increase one week after boost (FIG. 18C). The peptide stimulation and culture procedure resulted in some loss in the resolution of the staining of CXCR5+ICOS+PD-1 +CXCR3+ cTfh cells. However, a significant increase in the number of IFNγ+CXCR3+cTfh cells one week after prime and boost could still be observed (FIG. 18D). Collectively, this demonstrates that the H10/LNP vaccine administration induces H10-specific cTfh cells of the CXCR3+Th1-polarized profile that correlate with the avidity of H10-specific IgG antibodies.

### Materials and methods

### Production of modified mRNA and lipid nanoparticles

Modified mRNA encoding the hemagglutinin of H10N8 Influenza A virus (A/ Jiangxi-Donghu/ 346/ 2013) were generated as previously described (43). The lipid mixture was combined with a 50 mM citrate buffer (pH 4.0) containing mRNA at 3: 1 ratio (aqueous: ethanol) using a microfluidic mixer (Precision Nanosystems). For formulations containing GLA (Avanti Lipids), lipids were combined in a molar ratio of 50: 9.83: 38.5: 1.5: 0.17 (ionizable lipid: DSPC: cholesterol: PEG-lipid: GLA). All formulations were dialyzed against PBS, concentrated using Amicon ultra centrifugal filters (EMD Millipore) and passed through a 0.22 µm filter. Particles were 80-100 nm in size with >95% RNA encapsulation.

### Immunizations and sample collection

This animal study was approved by the Local Ethical Committee on Animal Experiments. Chinese rhesus macaques were housed in the Astrid Fagraeus laboratory at Karolinska Institutet according to guidelines of the Association for Assessment and Accreditation of Laboratory Animal Care and all procedures were performed abiding to the provisions and general guidelines of the Swedish Animal Welfare Agency. Animals were divided into three groups (n=4/group) receiving H10 mRNA/LNP (50 µg) either by IM or ID delivery or H10 mRNA/LNP co-formulated with GLA adjuvant (5 µg) delivered IM. Prime and boost immunizations were delivered at week 0 and 4 respectively. Animals receiving H10 mRNA/LNP with GLA received an additional boost at week 15. The animals were sedated with ketamine 10-15 mg/kg given IM (Ketaminol 100 mg/ml, Intervet, Sweden) during the immunizations, blood and bone marrow sampling. Bone marrow was sampled before immunization and at 2, 6 and 25 weeks from the humerus as previously described (44).

An axillary LN was collected before vaccination, opposite from the planned vaccination site, and a collateral axillary LN after boost. The animals were anaesthetized by IM injection of 10-15 mg/kg of ketamine and 0.05 mg/kg of medetomidine. Carprofen (4 mg/kg) was given IM as analgesia. LNs were removed in an aseptic manner using minimal entry holes with the aim of removing a singular LNs in each procedure. The anesthesia was reversed with atipamezole, 0.25 mg/kg IM after suturing.

### Blood and bone marrow processing

Peripheral blood was drawn into EDTA tubes and PBMCs were isolated using Ficoll-Paque^{™} PLUS (GE Healthcare) and washed with PBS. Red blood cells were removed using red blood cell lysis buffer and cells were frozen in 10% DMSO (Sigma-Aldrich) diluted in heat-inactivated FBS. Bone marrow mononuclear cells were isolated and stored in a similar manner as PBMCs from blood.

### Lymph node (LN) processing

Lymph nodes were processed as previously describe (23). Briefly, LNs were cleaned of fat and cut into small pieces using surgical scissors before being minced and filtered through 70 µm cell strainers. The cells were frozen as described above.

### Hemagglutination inhibition (HAI) assay

The hemagglutination inhibition assay was performed using 0.5% turkey red blood cells (Rockland Antibodies and Assays) diluted in PBS to investigate protective antibody titers. Serum was incubated overnight at +37°C with receptor destroying enzymes (Denka Seiken) to prevent non-specific HAI. Serial dilutions (1:2) of serum samples were performed in V-bottom 96-well plates in duplicates, starting from 1:10 dilution. Recombinant HA of H10N8 influenza A virus (4 units), A/Jiangxi-Donghu/346/2013 (Medigen Inc.) were added to diluted serum and incubated for 30 min at room temperature. The reciprocal of the last serum dilution that resulted non-agglutinated red blood cells represented the HAI titer. Titers <10 were assigned as 1.

### IgG Avidity ELISA

Clear flat-bottom immune 96-well plates (Thermofisher) were coated for 3h in 37°C with 100ng of H10 protein per well. After washing, wells were blocked for 1 hour at 37°C in 2% milk powder (blocking buffer) diluted in PBS. Blocking buffer was discarded and plasma samples diluted in blocking buffer were added and incubated for 1 hour at 37°C. Plasma was discarded and wells were incubated for 10 min with PBS or sodium thyocyanate (NaSCN) at 1.5-4.5M. After washing, wells were incubated with anti-monkey IgG HRP antibody (Nordic Labs) in washing buffer (0,05% Tween20 in PBS) for 1h at 37°C. After washing 100ul of TMB solution (BioLegend) was added to each well, the reaction was stopped at 5 min with 100ul of 1M Sulfuric Acid. Wells were read at 450nm using an ELISA reader (PerkinElmer).

### B cell ELISpot

The frequency of H10-specific antibody-secreting cells (ASCs) and memory B cells was determined as previously described (22), with some modifications. In brief, MAIPSWU10 96-well plates (Millipore) were coated with 10 µg/ml of antihuman IgG (Fcγ; Jackson ImmunoResearch Laboratories). Cells were transferred in duplicate dilution series and cultured overnight at 37°C. For enumeration of ASCs, cells were plated directly without prior stimulation, whereas for memory B cells cells were pre-stimulated for 4 days at 2×10⁶ cells/ml with 5 µg/ml CpG-B (ODN 2006; Invivogen), 10 µg/ml Pokeweed mitogen (PWM; Sigma-Aldrich), and 1:10000 Protein A from Staphylococcus aureus Cowan strain (SAC; Sigma- Aldrich). Plates were washed with PBS containing 0.05% Tween-20 (PBS-T), then incubated with 0.25 µg/ml biotinylated goat antihuman IgG (Fcγ; Jackson ImmunoResearch Laboratories) for total IgG determination, 0.1 µg/mL biotinylated H10 for H10-specific determination, or 0.1 µg/mL biotinylated OVA in PBS-T. The plates were washed and then incubated with streptavidin-conjugated alkaline phosphatase (Mabtech) diluted 1:1000 in PBS-T. Spots were developed with BCIP/NBT substrate (Mabtech) and counted using an AID ELISpot Reader and version 6 of the accompanying software (Autoimmun Diagnostika). Unspecific spots were subtracted from antigen-specific wells, as defined by spots counted in samples incubated in OVA-probed wells.
Recombinant HA of H10N8 influenza A virus (H10), A/Jiangxi-Donghu/346/2013 (Medigen Inc.) and ovalbumin (OVA; Invivogen) molecules were biotinylated using the EZ-Link Sulfo-NHS-Biotinylation kit (Thermo Fisher) using a 1:1 molar ratio and unreacted biotin was removed using the included Zeba Spin Desalting Columns.

### Phenotypic analysis and H10 recall assay of Tfh cells

1.5×10⁶ cells from indicated time points were stained with LIVE/DEAD Fixable Blue Dead Cell kit according to manufacturer's protocol (Invitrogen). Samples were surfaced stained with a panel of fluorescently labeled antibodies (Supplemental Table I) to identify specific cell subsets. Additionally, 1.5×10⁶ PBMCs were rested for 3 hours and then stimulated overnight in complete media (10% FCS, 1% penicillin/ streptomycin/ glutamine in RPMI, all from Gibco, Stockholm, Sweden) in U-bottom 96-well plates with H10 peptides (15mers overlapping by 11 amino acids, 2 µg/ml) and Brefeldin A at 10 µg/ml. Cells were stained with surface-specific antibodies (Supplemental Table 1), fixed and permeabilized using fixation and permeabilization solution (BD Biosciences) before stained for intracellular cytokines (Supplemental Table 1). Samples were resuspended in 1% paraformaldehyde before acquisition using a Fortessa flow cytometer (BD Biosciences). Results were analyzed using FlowJo version 9.7.6. Background cytokine staining was subtracted, as defined by staining in samples incubated without peptide.

### Detection of germinal centers in situ

Extirpated LNs were snapfrozen using dry ice in OCT media (Tissue-Tek) and kept in -80°C until use. Tissues were thawed to -20°C and then sectioned (8µm) and fixed for 15 min in 2% formaldehyde in PBS. Tissues were permeabilized using tris-buffered saline (TBS) with 0.1% saponin and 1% hepes buffer (permwash with pH 7.4), all future reagents were diluted in permwash. LNs were blocked with 1% FCS and then stained with anti CD3 (Dako), Ki67 (BD),) and PD-1 (R&D systems). After this, biotinylated anti-mouse or anti-goat (Dako) or anti-rabbit (Vector Labs) secondary antibodies were added, which were detected by the addition of streptavidin-conjugated Alexa Fluor 405/555/647 (Invitrogen). Image tiles of entire LNs were acquired using a Nikon Eclipse Ti-E confocal microscope. GCs were defined as dense follicular structures including CD3+PD-1+cells (light zone) and Ki67+ cells (dark zone) (Fig 3 A). Image analysis was done using Cell Profiler software (Broad Institute Inc.) with in-house algorithms. Briefly, GCs were manually identified in the program to enable automatic enumeration of PD-1+ and Ki67+ cells within the individual GCs and the area of the GCs. PD-1+cells were almost exclusively CD3+ and Ki67+ cells were mostly CD3- (GC B cells).

### CXCL13 ELISA

Undiluted plasma stored in -20°C was thawed and analyzed using a Quantikine Human CXCL13/BLC/BCA-1 Elisa (R&D systems) according to manufacturer's instructions.

### Detection of antigen-specific GC B cells by flow cytometry

2×10⁶ cells from LN cell suspensions were stained with LIVE/DEAD Fixable Blue Dead Cell kit according to manufacturer's protocol (Invitrogen). Samples were then incubated with a titrated amount of H10-tetramer probe for 20 min at 4°C. H10-tetramer probes were prepared beforehand by mixing biotinylated H10 protein with streptavidin-BV421 (Biolegend) at a 4: 1 molar ratio. Samples were washed and stained with a surface antibody cocktail (Supplemental Table 2). Before intracellular staining (Supplemental Table 2), samples were fixed and permeabilized using the Transcription Factor Buffer Set (BD Biosciences). Samples were resuspended in 1% paraformaldehyde before acquisition using a Fortessa flow cytometer (BD Biosciences). Results were analyzed using FlowJo version 9.7.6.

### Statistical Analysis

Statistical analysis was conducted by Prism Version 6.0 (GraphPad) software. All of the data are presented as the mean ± standard error of the mean (SEM). Difference between groups was analyzed as described in figure legends. A p-value <0.05 was considered to be statistically significant.

### EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.
In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03. It should be appreciated that embodiments described in this document using an open-ended transitional phrase (e.g., "comprising") are also contemplated, in alternative embodiments, as "consisting of" and "consisting essentially of" the feature described by the open-ended transitional phrase. For example, if the disclosure describes "a composition comprising A and B", the disclosure also contemplates the alternative embodiments "a composition consisting of A and B" and "a composition consisting essentially of A and B".

## Claims

1. A method for evaluating whether an mRNA vaccine composition, comprising mRNA encoding an antigen, will produce immunity against the antigen, the method comprising the steps of:
identifying in a mammalian subject that has been injected with the mRNA vaccine composition, T cell suppression values in local populations of inflammatory cells in a sample that has been taken from the injection site of the mRNA vaccine composition and in a sample that has been taken from the draining lymph node of the subject, and
determining a quantitative value of the amount of differential T Cell Activation Potential (dTCAP) based on the T cell suppression values from the injection site (T-suppis) and draining lymph node (T-supp_{LN}), wherein the quantitative value of the dTCAP is indicative of the nature of the immune response generated in response to the mRNA vaccine administration;
wherein the dTCAP is calculated as a ratio of the T-suppis to the T-supp_{LN} and wherein a ratio of at least 6:1 is indicative of a positive mRNA vaccine outcome;
wherein the T-supp_{LN} is measured as an amount of Myeloid-Derived Suppressor Cells (MDSCs) present in a draining lymph node at a time following mRNA vaccine administration; and
wherein the T-suppis is measured as an amount of MDSCs present in the injection site at a time following mRNA vaccine administration.

2. The method of claim 1, wherein the amount of MDSCs present in the draining lymph node and/or injection site is calculated 1-7 days following mRNA vaccine administration.

3. The method of any one of claim 1 or claim 2, wherein the amount of MDSCs present in the draining lymph node is 0-10 MDSCs/10⁵ live cells.

4. The method of any one of claim 1 or claim 2, wherein the amount of MDSCs present in the injection site is 50-1,000 MDSCs/10⁵ live cells.

5. The method of any one of claims 1-4, wherein the quantitative value of the dTCAP is indicative of an immunostimulatory activity of the mRNA vaccine.

6. The method of claim 5, wherein the activity is antigen specific T-cell activity.

7. A method for evaluating whether an mRNA vaccine composition, comprising mRNA encoding an antigen, will produce immunity against the antigen, the method comprising the steps of:
determining a quantitative value of the amount of differential T Cell Activation Potential (dTCAP) in a sample that has been taken from the draining lymph node of a mammalian subject that has been injected with the mRNA vaccine composition, wherein the quantitative value of the dTCAP is indicative of the nature of the immune response generated in response to the mRNA vaccine administration;
wherein the dTCAP is calculated as the ratio of draining lymph node antigenicity to draining lymph node tolerability;
wherein the draining lymph node antigenicity is the amount of T cell activators present at the draining lymph node, wherein the T cell activators are T follicular helper cells (Tfh);
wherein the draining lymph node tolerability is the amount of MDSCs present at the draining lymph node; and
wherein a ratio of greater than 1000: 1 is indicative of a positive mRNA vaccine outcome.

8. The method of any one of claims 1-6, wherein the sample from the injection site or the draining lymph node is a biopsy sample.

## Patentansprüche

1. Verfahren zum Bewerten, ob eine mRNA-Impfstoffzusammensetzung, umfassend mRNA, die für ein Antigen kodiert, Immunität gegen das Antigen erzeugen wird, das Verfahren umfassend die Schritte:
Identifizierung in einem Säugetier-Subjekt, dem die mRNA-Impfstoffzusammensetzung injiziert wurde, von T-Zell-Suppressionswerten in lokalen Populationen von Entzündungszellen in einer Probe, die von der Injektionsstelle der mRNA-Impfstoffzusammensetzung entnommen wurde und in einer Probe, die aus dem drainierenden Lymphknoten des Subjekts entnommen wurde, und
Bestimmen eines quantitativen Wertes der Menge des differentiellen T-Zell-Aktivierungspotentials (T Cell Activation Potential, dTCAP)) basierend auf den T-Zell-Suppressionswerten von der Injektionsstelle (T-supp_{IS}) und dem drainierenden Lymphknoten (T-supp_{LN}), wobei der quantitative Wert des dTCAP indikativ für die Art der Immunantwort ist, die als Reaktion auf die Verabreichung des mRNA-Impfstoffs erzeugt wird;
wobei der dTCAP als ein Verhältnis von dem T-supp_{IS} zu dem T-supp_{LN} berechnet wird und wobei ein Verhältnis von mindestens 6:1 indikativ für ein positives mRNA-Impfergebnis ist;
wobei der T-supp_{LN} als eine Menge von Myeloidabgeleiteten Suppressorzellen (Myeloid-Derived Suppressor Cells (MDSCs)) gemessen wird, die in einem drainierenden Lymphknoten zu einem Zeitpunkt nach der Verabreichung des mRNA-Impfstoffs vorhanden sind; und
wobei der T-supp_{IS} als eine Menge von MDSCs gemessen wird, die zu einem Zeitpunkt nach der Verabreichung des mRNA-Impfstoffs an der Injektionsstelle vorhanden sind.

2. Verfahren gemäß Anspruch 1, wobei die Menge an MDSCs, die im drainierenden Lymphknoten und/oder an der Injektionsstelle vorhandenen sind, 1-7 Tage nach Verabreichung des mRNA-Impfstoffs berechnet wird.

3. Verfahren gemäß irgendeinem von Anspruch 1 oder Anspruch 2, wobei die Menge der MDSCs, die in dem drainierenden Lymphknoten vorhandenen sind, 0-10 MDSCs/10⁵ lebende Zellen ist.

4. Verfahren gemäß irgendeinem von Anspruch 1 oder Anspruch 2, wobei die Menge an MDSCs, die in der Injektionsstelle vorhanden ist, 50-1.000 MDSCs/10⁵ lebende Zellen ist.

5. Verfahren gemäß irgendeinem der Ansprüche 1-4, wobei der quantitative Wert von dem dTCAP indikativ für eine immunstimulierende Aktivität des mRNA-Impfstoffs ist.

6. Verfahren gemäß Anspruch 5, wobei die Aktivität eine antigenspezifische T-Zell-Aktivität ist.

7. Verfahren zum Bewerten, ob eine mRNA-Impfstoffzusammensetzung, umfassend mRNA, die für ein Antigen kodiert, Immunität gegen das Antigen erzeugen wird, das Verfahren umfassend die Schritte:
Bestimmen eines quantitativen Wertes der Menge des differentiellen T-Zell-Aktivierungspotentials (dTCAP) in einer Probe, die aus dem drainierenden Lymphknoten eines Säugetier-Subjekts entnommen wurde, dem die mRNA-Impfstoffzusammensetzung injiziert wurde, wobei der quantitative Wert des dTCAP indikativ für die Art der Immunantwort ist, die als Reaktion auf die mRNA-Impfstoffverabreichung erzeugt wird;
wobei der dTCAP als das Verhältnis von Antigenität des drainierenden Lymphknotens zu Toleranz des drainierenden Lymphknotens berechnet wird;
wobei die Antigenität des drainierenden Lymphknotens die Menge an T-Zell-Aktivatoren ist, die an dem drainierenden Lymphknoten vorhanden sind, wobei die T-Zell-Aktivatoren follikuläre T-Helferzellen (Tfh) sind;
wobei die Toleranz des drainierenden Lymphknotens die Menge der im drainierenden Lymphknoten vorhandenen MDSCs ist; und
wobei ein Verhältnis von mehr als 1000:1 indikativ für ein positives mRNA-Impfergebnis ist.

8. Verfahren gemäß irgendeinem der Ansprüche 1-6, wobei die Probe von der Injektionsstelle oder dem drainierenden Lymphknoten eine Biopsieprobe ist.

## Revendications

1. Procédé pour évaluer si une composition de vaccin à ARNm, comprenant de l'ARNm codant pour un antigène, conférera une immunité contre l'antigène, le procédé comprenant les étapes suivantes :
identification chez un sujet mammifère qui a reçu une injection de la composition de vaccin à ARNm, de valeurs de suppression de lymphocytes T dans des populations locales de cellules inflammatoires dans un échantillon qui a été prélevé à partir du site d'injection de la composition de vaccin à ARNm et dans un échantillon qui a été prélevé à partir d'un ganglion lymphatique drainant du sujet, et
détermination d'une valeur quantitative de la quantité de potentiel différentiel d'activation de lymphocytes T (dTCAP) sur la base des valeurs de suppression de lymphocytes T provenant du site d'injection (T-supp_{IS}) et du ganglion lymphatique drainant (T-supp_{LN}), dans lequel la valeur quantitative du dTCAP indique la nature de la réponse immunitaire générée en réponse à l'administration de vaccin à ARNm ;
dans lequel le dTCAP est calculé en tant que rapport de la T-supp_{IS} à la T-supp_{LN} et dans lequel un rapport d'au moins 6:1 est indicatif d'un résultat positif de vaccin à ARNm ;
dans lequel la T-supp_{LN} est mesurée en tant que quantité de cellules myéloïdes suppressives (MDSC) présentes dans un ganglion lymphatique drainant après l'administration de vaccin à ARNm ; et
dans lequel la T-supp_{IS} est mesurée en tant que quantité des MDSC présentes au site d'injection après l'administration de vaccin à ARNm.

2. Procédé selon la revendication 1, dans lequel la quantité des MDSC présentes dans le ganglion lymphatique drainant et/ou au site d'injection est calculée 1 à 7 jours suivant l'administration de vaccin à ARNm.

3. Procédé selon l'une quelconque de la revendication 1 ou la revendication 2, dans lequel la quantité des MDSC présentes dans le ganglion lymphatique drainant est de 0-10 MDSC/10⁵ cellules vivantes.

4. Procédé selon l'une quelconque de la revendication 1 ou la revendication 2, dans lequel la quantité des MDSC présentes au site d'injection est de 50-1000 MDSC/10⁵ cellules vivantes.

5. Procédé selon l'une quelconque des revendications 1 à 4, dans lequel la valeur quantitative du dTCAP indique une activité immunostimulatrice du vaccin à ARNm.

6. Procédé selon la revendication 5, dans lequel l'activité est une activité de lymphocytes T spécifiques de l'antigène.

7. Procédé pour évaluer si une composition de vaccin à ARNm, comprenant de l'ARNm codant pour un antigène, conférera une immunité contre l'antigène, le procédé comprenant les étapes suivantes :
détermination d'une valeur quantitative de la quantité de potentiel différentiel d'activation de lymphocytes T (dTCAP) dans un échantillon qui a été prélevé à partir du ganglion lymphatique drainant d'un sujet mammifère qui a reçu une injection de la composition de vaccin à ARNm, dans lequel la valeur quantitative du dTCAP est indicative de la nature de la réponse immunitaire générée en réponse à l'administration de vaccin à ARNm ;
dans lequel le dTCAP est calculé en tant que rapport de l'antigénicité de ganglion lymphatique drainant à la tolérance de ganglion lymphatique drainant ;
dans lequel l'antigénicité de ganglion lymphatique drainant est la quantité d'activateurs de lymphocytes T présents au niveau du ganglion lymphatique drainant, dans lequel les activateurs de lymphocytes T sont des lymphocytes T auxiliaires folliculeux (Tfh) ;
dans lequel la tolérance de ganglion lymphatique drainant est la quantité de MDSC présentes au niveau du ganglion lymphatique drainant ; et
dans lequel un rapport supérieur à 1000:1 indique un résultat positif de vaccin à ARNm.

8. Procédé selon l'une quelconque des revendications 1 à 6, dans lequel l'échantillon provenant du site d'injection ou du ganglion lymphatique drainant est un échantillon de biopsie.
